(19) Europäisches Patentamt
European Patent Office
Office européen des brevets

(11) Veröffentlichungsnummer: **0 401 798 B1**

# (12) EUROPÄISCHE PATENTSCHRIFT

(45) Veröffentlichungstag der Patentschrift: **11.01.95**

(21) Anmeldenummer: **90110724.3**

(22) Anmeldetag: **06.06.90**

(51) Int. Cl.[6]: **C07C 225/16**, C07C 221/00, C07C 49/327, C07C 215/30, C07C 217/48, C07D 295/112, A61K 31/135, A01N 35/02

## (54) Substituierte Aminoalkylbenzolderivate.

(30) Priorität: **08.06.89 CH 2159/89**
**19.03.90 CH 890/90**

(43) Veröffentlichungstag der Anmeldung:
**12.12.90 Patentblatt 90/50**

(45) Bekanntmachung des Hinweises auf die Patenterteilung:
**11.01.95 Patentblatt 95/02**

(84) Benannte Vertragsstaaten:
**AT BE CH DE DK ES FR GB GR IT LI LU NL SE**

(56) Entgegenhaltungen:
**GB-A- 923 727**
**US-A- 3 123 643**

(73) Patentinhaber: **F. HOFFMANN-LA ROCHE AG**
**Postfach 3255**
**CH-4002 Basel (CH)**

(72) Erfinder: **Guerry, Philippe, Dr.**
**Burgfelderstrasse 30**
**CH-4055 Basel (CH)**
Erfinder: **Jolidon, Synèse, Dr.**
**Schillerstrasse 5**
**CH-4147 Birsfelden (CH)**
Erfinder: **Zurflüh, René, Dr.**
**Dachslenbergstrasse 54**
**CH-8180 Bülach (CH)**

(74) Vertreter: **Lederer, Franz, Dr. et al**
**Lederer, Keller & Riederer**
**Patentanwälte**
**Prinzregentenstrasse 16**
**D-80538 München (DE)**

EP 0 401 798 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Verbindungen der allgemeinen Formel

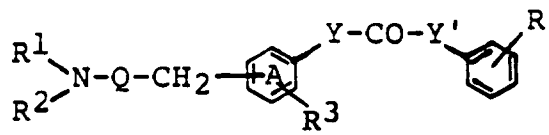

I

worin $R^1$ und $R^2$ je Wasserstoff, niederes Alkyl oder niederes Alkenyl oder zusammen geradkettiges Alkylen mit 2 bis 4 Kohlenstoffatomen, $R^3$ Wasserstoff, Halogen oder niederes Alkyl, Q Alkylen mit 2 bis 11 Kohlenstoffatomen und mindestens 2 Kohlenstoffatomen zwischen den zwei freien Valenzen oder Alkenylen mit 4 bis 11 Kohlenstoffatomen und mindestens 4 Kohlenstoffatomen zwischen den zwei freien Valenzen und Y und Y' je eine direkte Bindung oder die Gruppe $-CH_2-$, $-CH_2CH_2-$,$-CH=CH-$ oder $-C{\equiv}C-$ bedeuten, die Gruppe $R^1R^2N-Q-CH_2-$ an die 3- oder 4-Stellung des mit A bezeichneten Ringes gebunden ist, und das Symbol R bedeutet, dass der Ring unsubstituiert oder durch Halogen, Trifluormethyl, Cyano, Nitro, niederes Alkyl oder niederes Alkoxy substituiert ist,
und ihre pharmazeutisch annehmbaren Säureadditionssalze. Diese Verbindungen besitzen wertvolle pharmakologische Eigenschaften. Sie besitzen insbesondere eine ausgeprägte antifungale Wirkung und zeigen synergistische Effekte in Kombination mit bekannten, antifungal wirksamen Substanzen, welche die Sterol-Biosynthese hemmen, wie Ketoconazol und Terbinafin. Die Verbindungen der Formel I können demnach als Heilmittel verwendet werden, insbesondere zur Bekämpfung oder Verhütung von topischen oder systemischen Infektionen, welche durch pathogene Pilze, verursacht werden.

Verbindungen der Formel I, worin $R^1$ und $R^2$ je Wasserstoff oder niederes Alkyl oder zusammen geradkettiges Alkylen mit 2 bis 4 Kohlenstoffatomen, $R^3$ Wasserstoff, Q unverzweigtes Alkylen mit 2 bis 3 Kohlenstoffatomen und Y und Y' je eine direkte Bindung bedeuten, sind aus der U.S. Patentschrift Nr. 3,123,643 bekannt. Sie werden dort als Zwischenprodukte zur Herstellung von gewissen Cholesterol-Synthese-Hemmern beschrieben. Für die Zwischenprodukte selbst wird kein therapeutischer Verwendungszweck beschrieben. Es hat sich überraschenderweise gezeigt, dass auch diese bekannten Verbindungen der Formel I die obigen therapeutischen Wirkungen besitzen.

Gegenstand der vorliegenden Erfindung sind demnach: Die obigen Verbindungen der Formel I und ihre pharmazeutisch annehmbaren Säureadditionssalze zur Anwendung als therapeutische Wirkstoffe; Arzneimittel auf der Basis dieser Stoffe und deren Herstellung; die Verwendung dieser Stoffe als Heilmittel und zur Herstellung von antifungal wirksamen Arzneimitteln; sowie die neuen Verbindungen der Formel I und ihre pharmazeutisch annehmbaren Säureadditionssalze als solche, d.h. diejenigen Verbindungen der Formel I, worin Y und Y' nicht gleichzeitig eine direkte Bindung bedeuten, wenn $R^1$ und $R^2$ je Wasserstoff oder niederes Alkyl oder zusammen geradkettiges Alkylen mit 2 bis 4 Kohlenstoffatomen, $R^3$ Wasserstoff und Q geradkettiges Alkylen mit weniger als 4 Kohlenstoffatome bedeuten, die Herstellung dieser neuen Verbindungen und gewisse Zwischenprodukte zu deren Herstellung als solche.

Der Ausdruck "nieder" bezeichnet Reste und Verbindungen mit höchstens sieben, vorzugsweise höchstens vier Kohlenstoffatomen. Der Ausdruck "Alkyl" bezeichnet geradkettige oder verzweigte, gesättigte Kohlenwasserstoffreste, wie Methyl, Aethyl, Propyl, Isopropyl und t-Butyl. Der Ausdruck "Alkenyl" bezeichnet geradkettige oder verzweigte Kohlenwasserstoffreste mit einer olefinischen Doppelbindung, wie Allyl und 2-Butenyl. Der Ausdruck "Alkoxy" bezeichnet über ein Sauerstoffatom gebundene Alkylgruppen, wie Methoxy und Aethoxy. Der Ausdruck "Alkylen" bezeichnet geradkettige oder verzweigte, gesättigte Kohlenwasserstoffreste mit zwei freien Valenzen, wie Dimethylen, Trimethylen, Tetramethylen, Pentamethylen oder Hexamethylen. Der Ausdruck "Alkenylen" bezeichnet geradkettige oder verzweigte Kohlenwasserstoffreste mit zwei freien Valenzen und einer olefinischen Doppelbindung, wie 2-Buten-1,4-diyl. Der Ausdruck "Halogen" bezeichnet die vier Formen Fluor, Chlor, Brom und Jod.

Der weiter unten verwendete Ausdruck "Abgangsgruppe" bezeichnet Halogenatome, insbesondere Chlor, Brom und Jod, und niedere Alkyl- und Arylsulfonyloxygruppen, wie Methylsulfonyloxy, Benzolsulfonyloxy, p-Toluolsulfonyloxy und p-Chlorsulfonyloxy.

In einer speziellen Ausführungsform betrifft die vorliegende Erfindung Verbindungen der obigen Formel I, worin Q Alkylen mit 4 bis 11 Kohlenstoffatomen und mindestens 2 Kohlenstoffatomen zwischen den zwei freien Valenzen bedeutet, und $R^1$, $R^2$, $R^3$, R, Y und Y' obige Bedeutung besitzen.

Q enthält dabei vorzugsweise 4 bis 7 Kohlenstoffatome und ist vorzugsweise unverzweigt. $R^1$ und $R^2$ bedeuten vorzugsweise je $C_{1-4}$-Alkyl oder $C_{3-4}$-Alkenyl oder zusammen $C_{3-4}$-Alkylen. $R^3$ bedeutet

vorzugsweise Wasserstoff. Die Gruppe $R^1R^2N$-Q-$CH_2$- ist vorzugsweise an die 4-Stellung des mit A bezeichneten Ringes gebunden. Y bedeutet vorzugsweise eine direkte Bindung oder die Gruppe -$CH_2$-, insbesondere eine direkte Bindung. Y' bedeutet vorzugsweise eine direkte Bindung oder die Gruppe -$CH_2$-, -$CH_2CH_2$- oder -CH=CH-, insbesondere eine direkte Bindung oder die Gruppe -$CH_2$-. Das Symbol R bedeutet vorzugsweise, dass der Ring unsubstituiert oder durch Halogen, insbesondere Chlor, Brom oder Fluor, Trifluormethyl, Nitro oder niederes Alkyl substituiert, vorzugsweise mono- oder disubstituiert ist.

Im Rahmen dieses Aspektes besonders bevorzugte Verbindungen der Formel I sind:
4-[7-(Dimethylamino)heptyl]benzophenon,
4'-[7-(Dimethylamino)heptyl]-2-phenylacetophenon,
4'-Fluor-4-[7-(dimethylamino)heptyl]benzophenon,
4-[7-(Dimethylamino)heptyl]-4'-(trifluormethyl)benzophenon,
2,4-Difluor-4'-[7-(dimethylamino)heptyl]benzophenon,
4-[7-(Allylmethylamino)heptyl]-4'-brombenzophenon,
4-[6-(Allylmethylamino)hexyl]-4'-brombenzophenon und
4-[7-(Allylmethylamino)heptyl]benzophenon.

In einer weiteren speziellen Ausführungsform betrifft die vorliegende Erfindung Verbindungen der obigen Formel I, worin Q Alkylen mit 2 oder 3 Kohlenstoffatomen und mindestens 2 Kohlenstoffatomen zwischen den zwei freien Valenzen und Y und Y' je eine direkte Bindung oder die Gruppe -$CH_2$-, -$CH_2CH_2$-, -CH=CH- oder -C≡C-, jedoch nicht beide gleichzeitig eine direkte Bindung bedeuten, und $R^1$, $R^2$, $R^3$ und R obige Bedeutung besitzen.

Der Kohlenwasserstoffrest Q ist dabei vorzugsweise unverzweigt. $R^1$ und $R^2$ bedeuten vorzugsweise je $C_{1-4}$--Alkyl oder $C_{3-4}$-Alkenyl oder zusammen $C_{3-4}$-Alkylen. $R^3$ bedeutet vorzugsweise Wasserstoff. Die Gruppe $R^1R^2N$-Q-$CH_2$- ist vorzugsweise an die 4-Stellung des mit A bezeichneten Ringes gebunden. Vorzugsweise bedeuten Y eine direkte Bindung und Y' die Gruppe -$CH_2$-, -$CH_2CH_2$- oder -CH=CH-. Das Symbol R bedeutet vorzugsweise, dass der Ring unsubstituiert oder durch Halogen, insbesondere Chlor, Brom oder Fluor, Trifluormethyl, Nitro oder niederes Alkyl substltuiert, vorzugsweise mono- oder disubstituiert ist.

Im Rahmen dieses Aspektes besonders bevorzugte Verbindungen der Formel I sind:
3-(4-Chlorphenyl)-4'-[3-(dimethylamino)propyl]propiophenon,
3-(2-Methylphenyl)-4'-[3-(dimethylamino)propyl]propiophenon und
(E)-3-Phenyl-4'-[3-(1-pyrrolidinyl)propyl]acrylophenon.

Die neuen Verbindungen der Formel I und ihre pharmazeutisch annehmbaren Säureadditionssalze können erfindungsgemäss hergestellt werden, indem man

a) eine Verbindung der allgemeinen Formel

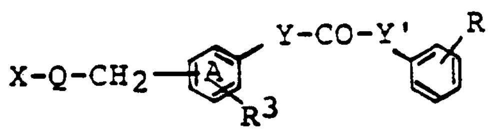

II

worin X eine Abgangsgruppe bedeutet, und A, $R^3$, Q, Y, Y' und R obige Bedeutung besitzen,

mit einem Amin der allgemeinen Formel $HNR^1R^2$, worin $R^1$ und $R^2$ obige Bedeutung besitzen, umsetzt, oder

b) eine Verbindung der allgemeinen Formel

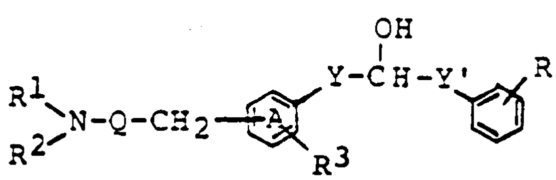

III

worin A, $R^1$, $R^2$, $R^3$, Q, Y, Y' und R obige Bedeutung besitzen,

oxidiert, oder

c) eine Verbindung der allgemeinen Formel

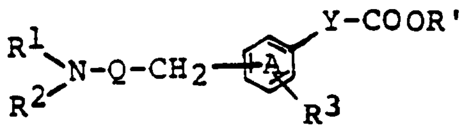

IV

worin R' niederes Alkyl bedeutet, und A, $R^1$, $R^2$, $R^3$, Q und Y obige Bedeutung besitzen, mit einer Verbindung der allgemeinen Formel

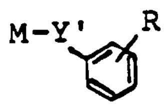

V

worin M -MgCl, -MgBr, -MgJ oder -Li bedeutet, und Y' und R obige Bedeutung besitzen, umsetzt, oder

d) eine Verbindung der allgemeinen Formel

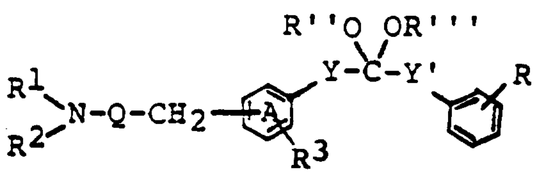

VI

worin R'' und R''' je niederes Alkyl oder zusammen Dimethylen oder Trimethylen bedeuten, und A, $R^1$, $R^2$, $R^3$, Q, Y, Y' und R obige Bedeutung besitzen, mit einer wässrigen Säure behandelt, oder

e) eine Verbindung der allgemeinen Formel

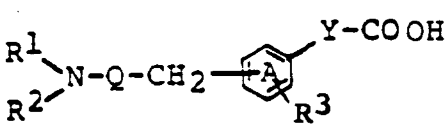

oder

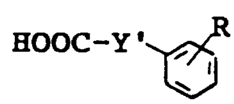

VIIb

VIIa

in Form eines reaktiven Derivates in Gegenwart einer Lewis-Säure mit einer Verbindung der allgemeinen Formel

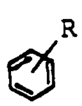

VIIIa bzw.

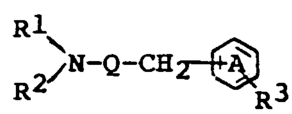

VIIIb

worin A, $R^1$, $R^2$, $R^3$, Q, Y, Y' und R obige Bedeutung besitzen, umsetzt, oder

f) eine Verbindung der allgemeinen Formel

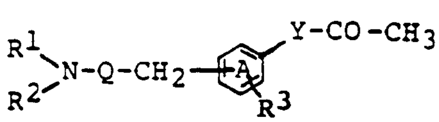

IX

worin A, $R^1$, $R^2$, $R^3$, Q und Y obige Bedeutung besitzen,

in Gegenwart einer Base mit einer Verbindung der allgemeinen Formel

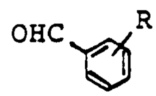

X

worin R obige Bedeutung besitzt,
umsetzt, oder

g) eine Verbindung der allgemeinen Formel

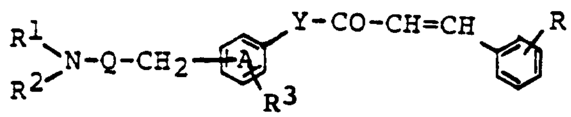

oder

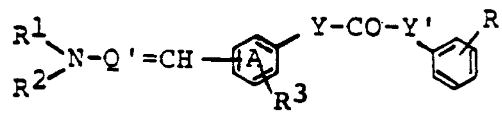

Ia XI

worin Q' die um ein Wasserstoffatom verminderte Gruppe Q bedeutet, und A, $R^1$,$R^2$, $R^3$, Q, Y, Y' und R obige Bedeutung besitzen,
hydriert, und

h) eine erhaltene Verbindung der Formel I erwünschtenfalls in ein pharmazeutisch annehmbares Säureadditionssalz überführt.

Die Umsetzung einer Verbindung der Formel II mit einem Amin der Formel $HNR^1R^2$ gemäss Verfahrensvariante a) kann nach an sich bekannten und jedem Fachmann geläufigen Methoden durchgeführt werden. Die Reaktion wird vorzugsweise in einem polaren Lösungsmittel und in Gegenwart einer Base als säurebindendes Mittel in einem Temperaturbereich von etwa 0 °C bis etwa 150 °C durchgeführt. Geeignete Lösungsmittel sind beispielsweise niedere Alkohole, wie Methanol und Aethanol, und niedere Dialkylketone, wie Aceton. Geeignete Basen sind beispielsweise überschüssiges Amin der Formel $HNR^1R^2$, tertiäre Amine, wie Triäthylamin, und anorganische Basen, wie Alkalimetallcarbonate, -hydroxide und -alkoholate.

Die Oxidation einer Verbindung der Formel III gemäss Verfahrensvariante b) kann nach an sich bekannten und jedem Fachmann geläufigen Methoden durchgeführt werden. Die Reaktion wird vorzugsweise in einem inerten Lösungsmittel und in Gegenwart eines Oxidationsmittels in einem Temperaturbereich von etwa -80 °C bis etwa Raumtemperatur durchgeführt. Geeignete Lösungsmittel sind beispielsweise chlorierte, niedere Kohlenwasserstoffe, wie Methylenchlorid und Chloroform. Geeignete Oxidationsmittel sind beispielsweise Mangandioxid oder Mischungen von Dimethylsulfoxid mit Oxalylchlorid, Dicyclohexylcarbodiimid oder Acetanhydrid und einem tertiären Amin, wie Triäthylamin.

Die Umsetzung einer Verbindung der Formel IV mit einer Verbindung der Formel V gemäss Verfahrensvariante c) kann nach an sich bekannten und jedem Fachmann geläufigen Methoden durchgeführt werden. Die Reaktion wird vorzugsweise in einem inerten Lösungsmittel und in einem Temperaturbereich von etwa -80 °C bis etwa Raumtemperatur durchgeführt. Geeignete Lösungsmittel sind beispielsweise offenkettige und cyclische Aether, wie Diäthyläther, Methyl-t-butyläther und Tetrahydrofuran, und Mischungen davon.

Für die Behandlung einer Verbindung der Formel VI mit einer wässrigen Säure gemäss Verfahrensvariante d) verwendet man vorzugsweise eine verdünnte, wässrige Mineralsäure, z.B. verdünnte Salzsäure, und arbeitet in einem Temperaturbereich von etwa 0 °C bis etwa Raumtemperatur.

Die Umsetzung eines reaktiven Derivates einer Verbindung der Formel VIIa mit einer Verbindung der Formel VIIIa bzw. die Umsetzung eines reaktiven Derivates einer Verbindung der Formel VIIb mit einer Verbindung der Formel VIIIb gemäss Verfahrensvariante e) kann nach an sich bekannten und jedem Fachmann geläufigen Methoden durchgeführt werden. Die Reaktion wird vorzugsweise in einem inerten Lösungsmittel und in Gegenwart einer Lewis-Säure in einem Temperaturbereich von etwa 0 °C bis etwa 100 °C durchgeführt. Geeignete Lösungsmittel sind beispielsweise halogenierte niedere Kohlenwasserstoffe, wie Methylenchlorid, Chloroform und Aethylenchlorid, Nitrobenzol, Schwefelkohlenstoff und überschüssige Verbindung der Formel VIIIa. Als Lewis-Säure verwendet man vorzugsweise Aluminiumchlorid. Geeignete reaktive Derivate von Verbindungen der Formel VIIa oder VIIb sind beispielsweise die entsprechenden Carbonsäurechloride.

Die Umsetzung einer Verbindung der Formel IX mit einer Verbindung der Formel X in Gegenwart einer Base gemäss Verfahrensvariante f) kann nach an sich bekannten und jedem Fachmann geläufigen Methoden durchgeführt werden. Die Reaktion wird vorzugsweise in einem polaren Lösungsmittel und in

einem Temperaturbereich von etwa 0 °C bis etwa 60 °C durchgeführt. Geeignete Lösungsmittel sind beispielsweise niedere Alkohole, wie Methanol und Aethanol, und Mischungen davon mit Wasser. Als Basen verwendet man vorzugsweise Alkalimetallcarbonate und -hydroxide, wie Kaliumcarbonat und Natriumhydroxid.

Die Hydrierung einer Verbindung der Formel Ia oder XI gemäss Verfahrensvariante g) kann nach an sich bekannten und jedem Fachmann geläufigen Methoden durchgeführt werden. Die Reaktion wird vorzugsweise in einem polaren Lösungsmittel mit elementarem Wasserstoff in Gegenwart eines geeigneten Hydrierungskatalysators und in einem Temperaturbereich von etwa 0 °C bis etwa Raumtemperatur durchgeführt. Geeignete Lösungsmittel sind beispielsweise niedere Alkohole, wie Methanol und Aethanol. Geeignete Katalysatoren sind beispielsweise Palladium oder Platin auf Kohle, Platinoxid oder Raney-Nickel.

Die Herstellung von pharmazeutisch annehmbaren Säureadditionssalzen von Verbindungen der Formel I gemäss Verfahrensvariante h) kann nach an sich bekannten und jedem Fachmann geläufigen Methoden durchgeführt werden. Es kommen dabei Salze mit pharmazeutisch annehmbaren anorganischen und organischen Säuren in Betracht. Bevorzugte Säureadditionssalze sind die Hydrochloride, Hydrobromide, Sulfate, Nitrate, Citrate, Acetate, Succinate, Fumarate, Methansulfonate und die p-Toluolsulfonate.

Die bekannten Verbindungen der Formel I und ihre pharmazeutisch annehmbaren Säureadditionssalze können ebenfalls nach den obigen Verfahren a)-h) hergestellt werden. Die entsprechenden Ausgangsstoffe können, wie nachstehend für die Ausgangsstoffe für die neuen Verbindungen der Formel I beschrieben, hergestellt werden.

Die verschiedenen als Ausgangsstoffe verwendeten Verbindungen können beispielsweise gemäss den nachfolgenden Reaktionsschemata I-VIII und den nachfolgenden Beschreibungen der verschiedenen Reaktionen hergestellt werden. In diesen Reak- tionsschemata haben $R^1$, $R^2$, $R^3$, R, R', R'', R''', A, M, Q, Q', X, Y und Y' die obige Bedeutung, M' bedeutet -MgCl, -MgBr oder -MgJ, und Ø bedeutet Phenyl.

Reaktionsschema I

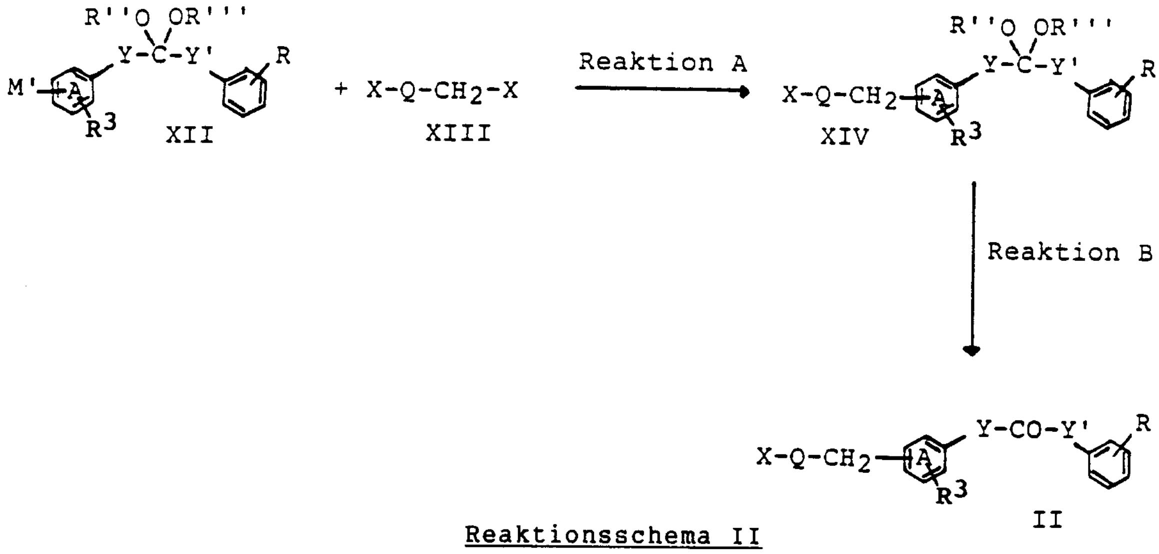


XII, XIII, XIV, II

Reaktionsschema II

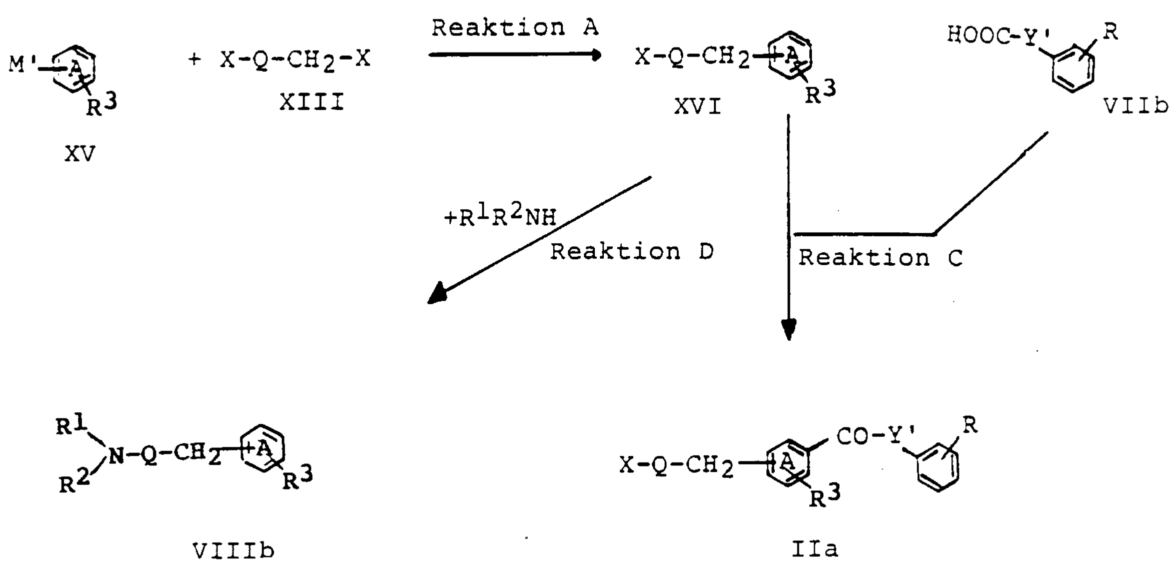


XV, XIII, XVI, VIIb, VIIIb, IIa

Reaktionsschema III

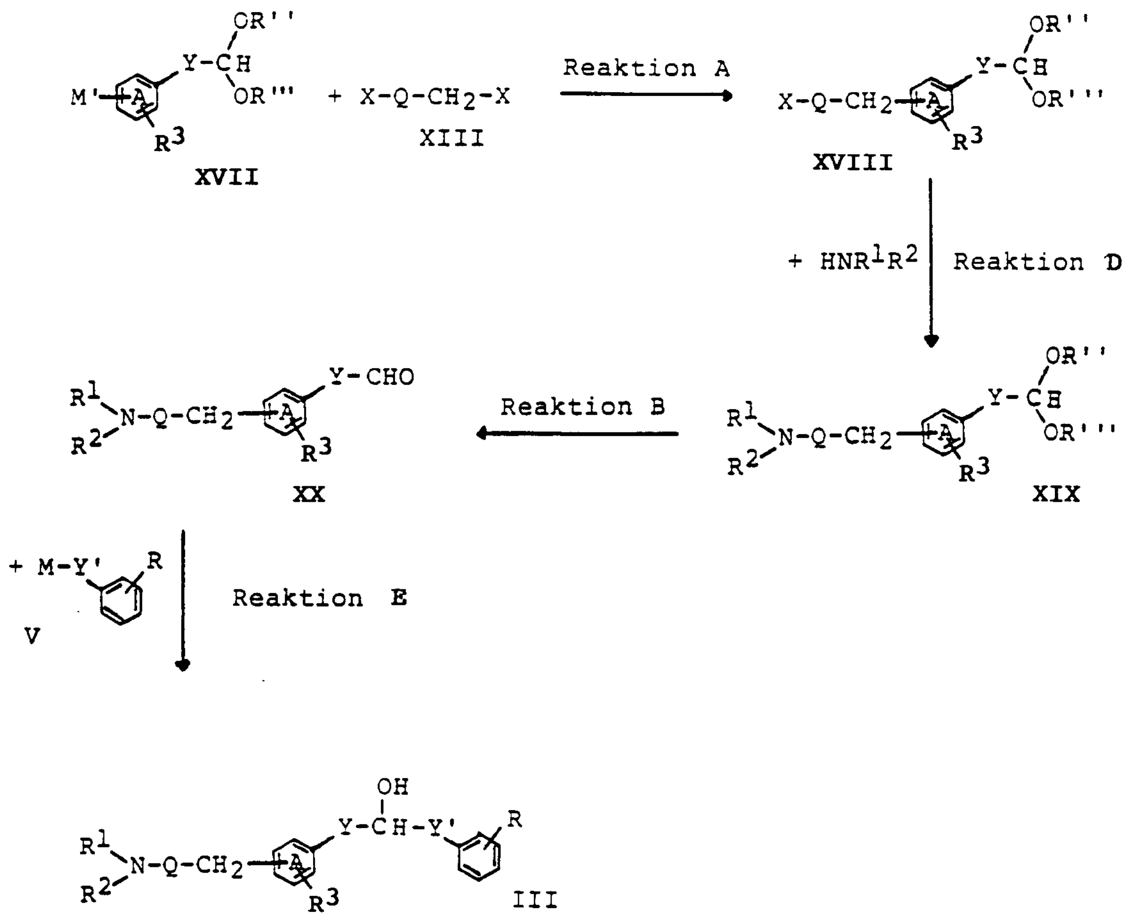


Reaktionsschema IV

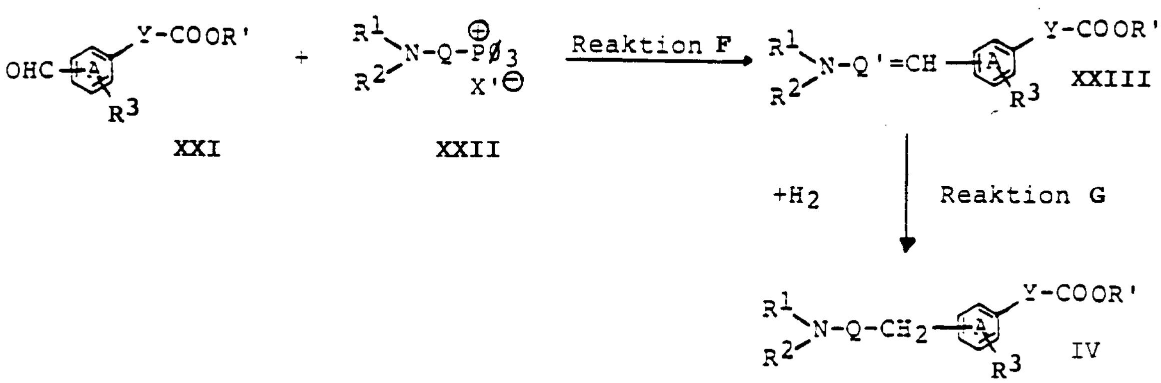

Reaktionsschema V

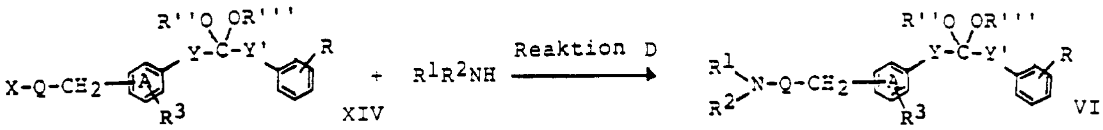


Reaktionsschema VI

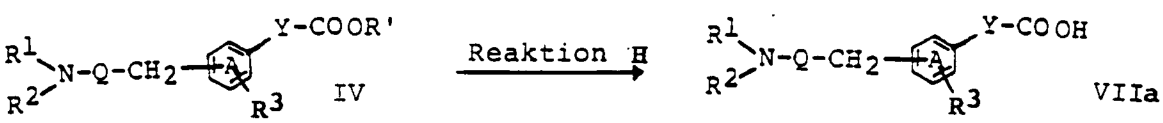

Reaktionsschema VII

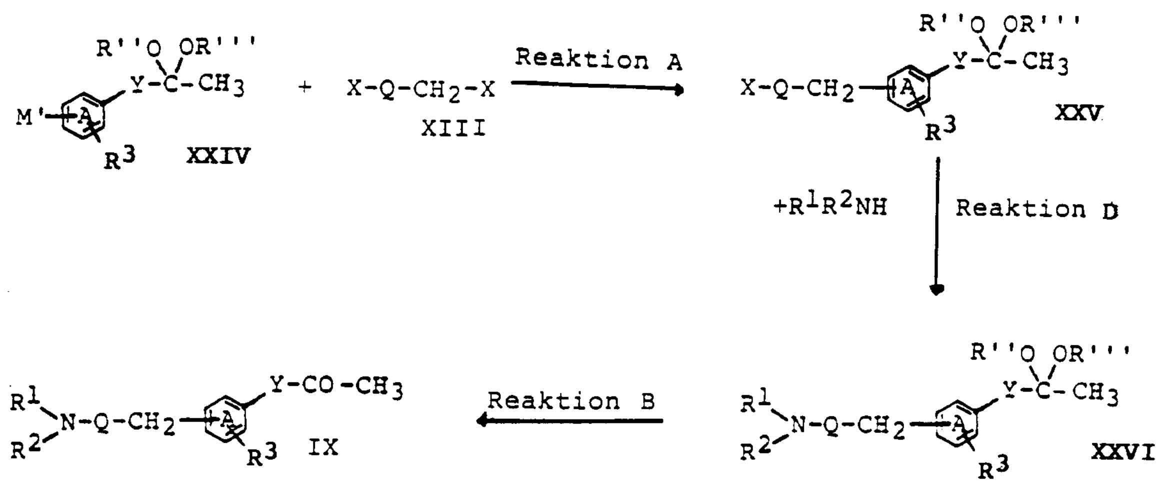


XXIV XIII XXV XXVI IX

Reaktionsschema VIII

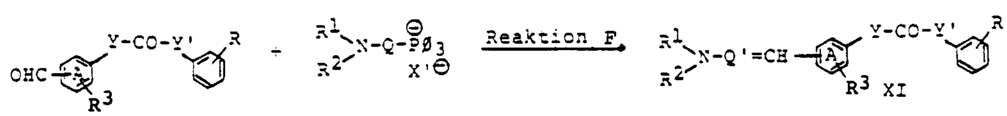


XXVII XXII XI

Reaktion A

Diese Reaktion kann nach an sich bekannten und jedem Fachmann geläufigen Methoden durchgeführt werden und wird vorzugsweise in einem inerten organischen Lösungsmittel in Gegenwart eines geeigneten Kopplungsreagenzes, wie Dilithiumtetrachlorocuprat und in einem Temperaturbereich von etwa 0 °C bis etwa Raumtemperatur durchgeführt. Geeignete Lösungsmittel sind beispielsweise offenkettige und cyclische Aether, wie Diäthyläther, Dimethoxyäthan und Tetrahydrofuran.

Reaktion B

Diese Reaktion kann nach an sich bekannten und jedem Fachmann geläufigen Methoden durchgeführt werden und wird vorzugsweise durch Behandeln mit einer wässerigen Säure durchgeführt. Man verwendet vorzugsweise eine verdünnte, wässrige Mineralsäure, z.B. verdünnte Salzsäure, und arbeitet in einem Temperaturbereich von etwa 0 °C bis etwa Raumtemperatur.

Reaktion C

Diese Reaktion kann nach an sich bekannten und jedem Fachmann geläufigen Methoden durchgeführt werden. Die Verbindung der Formel VIIb wird in Form eines reaktiven Derivates, beispielsweise in Form des entsprechenden Carbonsäurechlorides eingesetzt. Die Reaktion wird vorzugsweise in einem inerten Lösungsmittel und in Gegenwart einer Lewis-Säure in einem Temperaturbereich von etwa 0 °C bis etwa 100 °C durchgeführt. Geeignete Lösungsmittel sind beispielsweise halogenierte niedere Kohlenwasserstoffe, wie Methylenchlorid, Chloroform und Aethylenchlorid, Nitrobenzol und Schwefelkohlenstoff. Als Lewis-Säure verwendet man vorzugsweise Aluminiumchlorid.

Reaktion D

Die Umsetzung mit einem Amin der Formel $HNR^1R^2$ kann nach an sich bekannten und jedem Fachmann geläufigen Methoden durchgeführt werden. Die Reaktion wird vorzugsweise in einem polaren Lösungsmittel und in Gegenwart einer Base als säurebindendes Mittel in einem Temperaturbereich von etwa 0 °C bis etwa 150 °C durchgeführt. Geeignete Lösungsmittel sind beispielsweise niedere Alkohole, wie Methanol und Aethanol, und niedere Dialkylketone, wie Aceton. Geeignete Basen sind beispielsweise überschüssiges Amin der Formel $HNR^1R^2$, tertiäre Amine, wie Triäthylamin, und anorganische Basen, wie Alkalimetallcarbonate, -hydroxide und -alkoholate.

Reaktion E

Die Umsetzung mit einer Verbindung der Formel V kann nach an sich bekannten und jedem Fachmann geläufigen Methoden durchgeführt werden. Die Reaktion wird vorzugsweise in einem inerten Lösungsmittel und in einem Temperaturbereich von etwa -80 °C bis etwa Raumtemperatur durchgeführt. Geeignete Lösungsmittel sind beispielsweise offenkettige und cyclische Aether, wie Diäthyläther, Methyl-t-butyläther und Tetrahydrofuran, und Mischungen davon.

Reaktion F

Diese Reaktion kann nach an sich bekannten und jedem Fachmann geläufigen Methoden durchgeführt werden und wird vorzugsweise in einem inerten organischen Lösungsmittel in Gegenwart einer starken Base und in einem Temperaturbereich von etwa -80 °C bis etwa Raumtemperatur durchgeführt. Geeignete Lösungsmittel sind beispielsweise offenkettige und cyclische Aether, wie Diäthyläther, t-Butylmethyläther und Tetrahydrofuran. Geeignete starke Basen sind beispielsweise niedere Alkalimetallalkoholate, wie Kalium-t-butylat, Natriumhydrid und niederes Alkyllithium, wie n-Butyllithium.

Reaktion G

Diese Hydrierung kann nach an sich bekannten und jedem Fachmann geläufigen Methoden durchgeführt werden und wird vorzugsweise in einem polaren Lösungsmittel mit molekularem Wasserstoff in Gegenwart eines geeigneten Hydrierungskatalysators und in einem Temperaturbereich von etwa 0 °C bis etwa Raumtemperatur durchgeführt. Geeignete Lösungsmittel sind beispielsweise niedere Alkohole, wie Methanol und Aethanol. Geeignete Katalysatoren sind beispielsweise Palladium oder Platin auf Kohle, Platinoxid oder Raney-Nickel.

Reaktion H

Bei dieser Reaktion handelt es sich um eine Hydrolyse. Diese kann nach an sich bekannten und jedem Fachmann geläufigen Methoden durchgeführt werden und wird vorzugsweise durch Behandeln mit einem Alkalimetallhydroxid, wie Natrium- und Kaliumhydroxid, oder mit einer Mineralsäure, wie Salzsäure und Bromwasserstoffsäure, in einem polaren Lösungsmittel und in einem Temperaturbereich von etwa 0 °C bis etwa 100 °C durchgeführt. Geeignete Lösungsmittel sind beispielsweise Mischungen von niederen Alkoholen, wie Methanol und Aethanol, und mit Wasser mischbaren offenkettigen und cyclischen Aethern, wie Tetrahydrofuran, mit Wasser.

Die Ausgangsstoffe der Formeln II, III und VI, worin $R^1$, $R^2$, $R^3$, R, Q, Y und Y' die für die neuen Verbindungen der Formel I angegebenen Bedeutungen besitzen, sind ebenfalls neu und Gegenstand der vorliegenden Erfindung.

Wie bereits erwähnt besitzen die Verbindungen der Formel I und ihre pharmazeutisch annehmbaren Säureadditionssalze wertvolle antifungale Eigenschaften. Sie sind wirksam gegen eine Vielzahl von pathogenen Pilzen, die topische und systemische Infektionen hervorrufen, wie Candida albicans und Histoplasma capsulatum. Die 2,3-Epoxysgualen-Lanosterol-Cyclase, ein Enzym involviert in der Sterolbiosynthese der eucaryotischen Zelle, ist ein essentielles Enzym für die Pilze. So ist z.B. ein S.cerevisiae Stamm, in welchem dieses Enzym fehlt nicht lebensfähig [F. Karst & F. Lacroute, Molec. Gen. Genet. 154, 269 (1977)-]. Die hemmende Wirkung der Verbindungen der Formel I auf obengenanntes Enzym aus C.albicans wurde als Mass für die antifungale Wirkung genommen. Die Hemmung kann, beispielsweise, mittels der nachfolgend beschriebenen Methode gemessen werden.

Bestimmung des IC50-Wertes für die Hemmung der 2,3-Epoxysqualen-Lanosterol-Cyclase von Candida albicans

Die Zellen einer Kultur von Candida albicans am Ende der logarithmischen Wachstumsphase werden gesammelt und mit 100mM Phosphatpuffer (pH = 6.9), Digestionspuffer und 50mM Phosphatpuffer (pH = 7.4), welcher 1M Mannitol und 5mM DTT enthält, gewaschen.

1,0 g dieser Zellen werden in 5 ml Digestionspuffer aufgeschlämmt, mit 1 mg Zymolase 100T (Seikagaku Kogyo, Japan) und 12,5 µl β-Mercaptoäthanol versetzt und während 30 Minuten bei 30 °C inkubiert. Die entstehenden Protoplasten werden durch Zentrifugation (10 Minuten bei 2500 g) isoliert und anschliessend durch Zugabe von 2 ml 100mM Phosphatpuffer (pH = 6,9) zum Platzen gebracht. Durch erneute Zentrifugation (10 Minuten bei 10000 g) erhält man einen zellfreien Extrakt (CFE) als Ueberstand. Dieser wird auf 10 mg Protein pro ml verdünnt, und der pH wird auf 6,9 gebracht.

Die Aktivität der 2,3-Epoxysqualen-Lanosterol-Cyclase im CFE wird durch die Umsetzung von $^{14}C$-Squalen-Epoxid in Gegenwart von n-Decylpentaoxyäthylen als Detergens gemessen. Titration mit angemessenen Mengen der Testsubstanz erlauben die Bestimmung des $IC_{50}$-Wertes (Konzentration der Testsubstanz, welche die Enzymaktivität um die Hälfte verringert).

Der Versuch wird wie folgt durchgeführt:

Durch Ultraschallbehandlung bereitet man eine 250µM Lösung von $^{14}C$-Squalen-Epoxid in 100mM Phosphatpuffer (pH = 6,9) unter Zusatz von 1% n-Decylpentaoxyäthylen vor. 100 µl dieser Lösung wird mit 20 µl einer Lösuang der Testsubstanz in Dimethylsulfoxid (bzw. 20 µl reinem Dimethylsulfoxid als Kontrolle) versetzt. Nach Zugabe von 880 µl CFE wird die gut gemischte Lösuang unter Schütteln während 1 Stunde bei 30° inkubiert. Anschliessend wird durch Zugabe von 500 µl 15-proz. Kaliumhydroxid in 90-proz. Aethanol die Reaktion gestoppt.

Das Gemisch wird zweimal mit 1 ml n-Hexan extrahiert, das Hexan wird abgedampft und der Lipid-Rückstand wird in 200 µl Diäthyläther aufgenommen. Nach Dünnschichtchromatographie an Kieselgel mit Methylenchlorid als Laufmittel werden die Platten mit einem Radioaktivitäts-Dünnschicht-Scanner untersucht.

Unter den verwendeten Bedingungen wird ausschliesslich Lanosterol als radioaktives Produkt gefunden. Dessen Menge wird mit der Menge radioaktivem Lanosterol in der Kontrolle verglichen.

Die $IC_{50}$-Werte werden graphisch ermittelt und in µg Testsubstanz pro ml angegeben. Die nachfolgende Tabelle I enthält für repräsentative Vertreter der durch die Formel I definierten Verbindungsklasse die in obigem Versuch ermittelten $IC_{50}$-Werte, sowie Angaben über die akute Toxizität bei subkutaner Verabreichung an Mäusen ($DL_{50}$ in mg/kg).

## Tabelle I

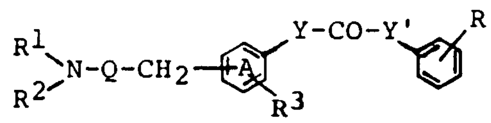

| Nr. | $R^1$ | $R^2$ | $R^3$ | Q (Pos.) | Y | Y' | R | $IC_{50}$ in µg/ml | $DL_{50}$ in mg/kg s.c. |
|---|---|---|---|---|---|---|---|---|---|
| 1 | $-CH_3$ | $-CH_3$ | H | $-(CH_2)_6-(4)$ | - | - | H | 0,17 | 312-625 |
| 2 | $-CH_3$ | $-CH_3$ | H | $-(CH_2)_6-(4)$ | - | $-CH_2-$ | H | 0,48 | |
| 3 | $-CH_3$ | $-CH_3$ | H | $-(CH_2)_6-(4)$ | - | - | 4-Fluor | 0,42 | |
| 4 | $-CH_3$ | $-CH_3$ | H | $-(CH_2)_6-(4)$ | - | - | 4-Trifluormethyl | 1,35 | |
| 5 | $-CH_3$ | $-CH_3$ | H | $-(CH_2)_6-(4)$ | - | - | 2,4-Difluor | 0.55 | |
| 6 | $-CH_3$ | $-CH_3$ | H | $-(CH_2)_2-(4)$ | - | $-CH=CH-$ | 4-Chlor | 1,20 | |
| 7 | $-CH_3$ | $-CH_3$ | H | $-(CH_2)_2-(4)$ | - | $CH_2CH_2-$ | 4-Chlor | 0,40 | |
| 8 | $-CH_3$ | $-CH_3$ | H | $-(CH_2)_2-(4)$ | - | $CH_2CH_2-$ | 4-Fluor | 0,55 | |
| 9 | $-CH_3$ | $-CH_3$ | H | $-(CH_2)_2-(4)$ | - | $CH_2CH_2-$ | 2-Methyl | 0,11 | |
| 10 | $-CH_3$ | $-CH_3$ | H | $-(CH_2)_2-(4)$ | - | $CH_2CH_2-$ | 3-Chlor | 0,44 | |
| 11 | $-CH_3$ | $-CH_3$ | H | $-(CH_2)_2-(4)$ | - | $CH_2CH_2-$ | 4-Nitro | 0,28 | |
| 12 | $-(CH_2)_4-$ | | H | $-(CH_2)_2-(4)$ | - | $-CH=CH-$ | H | 0,25 | |

| Nr. | $R^1$ | $R^2$ | $R^3$ | Q (Pos.) | Y | Y' | R | $IC_{50}$ in µg/ml | $DL_{50}$ in mg/kg s.c. |
|---|---|---|---|---|---|---|---|---|---|
| 13 | $-(CH_2)_4-$ | | H | $-(CH_2)_2-(4)$ | - | $-CH_2CH_2-$ | H | 0,40 | |
| 14 | $-CH_3$ | $-CH_3$ | H | $-(CH_2)_2-(4)$ | - | - | H | 5 | |
| 15 | $-CH_3$ | $-CH_3$ | H | $-(CH_2)_7-(4)$ | - | - | H | 0,41 | |
| 16 | $-CH_3$ | $-CH_3$ | H | $-(CH_2)_7-(4)$ | - | - | H | 0,14 | |
| 17 | $-CH_3$ | $-CH_3$ | H | $-(CH_2)_6-(4)$ | - | - | 4-Chlor | 0,49 | |
| 18 | $-CH_3$ | $-CH_3$ | H | $-(CH_2)_6-(4)$ | - | - | 4-Brom | 0,86 | |
| 19 | $-CH_3$ | $-CH_3$ | H | $-(CH_2)_6-(4)$ | - | - | 2,4-Dichlor | 0,75 | |
| 20 | $-CH_3$ | $-CH_3$ | H | $-(CH_2)_6-(4)$ | - | - | 4-Cyano | 0,26 | |
| 21 | $-CH_2-CH=CH_2$ | $-CH_3$ | H | $-(CH_2)_6-(4)$ | - | - | H | 0,05 | |
| 22 | $-CH_2-CH=CH_2$ | $-CH_3$ | H | $-(CH_2)_6-(4)$ | - | - | 4-Brom | 0,19 | |
| 23 | $-CH_2-CH=CH_2$ | $-CH_3$ | H | $-(CH_2)_6-(4)$ | - | - | 4-Cyano | 0,06 | |
| 24 | $-CH_2-CH=CH_2$ | $-CH_3$ | H | $-(CH_2)_5-$ " | - | - | 4-Brom | 0,095 | 312-625 |
| 25 | $-CH_3$ | $-CH_3$ | H | $-(CH_2)_5-$ " | - | - | 4-Brom | 0,92 | |
| 26 | $-CH_3$ | $-CH_3$ | H | $-(CH_2)_5-$ " | - | - | 3-Brom | 0,42 | |
| 27 | $-CH_3$ | $-CH_3$ | H | $-(CH_2)_5-$ " | - | - | 2-Methyl | 0,16 | |
| 28 | $-CH_3$ | $-CH_3$ | H | $-(CH_2)_5-$ " | - | - | 4-Nitro | 0,21 | |
| 29 | $-CH_3$ | $-CH_3$ | H | $-(CH_2)_5-$ " | - | - | 2-Chlor | 0,11 | |

Die bereits erwähnte synergistische Wirkung der Verbindungen der Formel I und ihrer pharmazeutisch annehmbaren Säureadditionssalze in Kombination mit Sterol-Biosynthese-Hemmern, wie Ketoconazol, kann beispielsweise mittels der Agar-Verdünnungs-Methode gezeigt werden. Man verwendet hierfür Casitonagar und Inokula (10 Zellen/ml) von Kulturen von Candida albicans, welche 48 Stunden alt sind. Die Testsubstanzen (TS, Verbindungen der Formel I) werden in Konzentrationen von 80-1,25 µg/ml und die Sterol-Biosynthese-Hemmer (SBH) in Konzentrationen von 20-0,001 µg/ml appliziert, wobei die Verdünnungsschritte jeweils 1:2 betragen. Die Kulturen werden jeweils während 2 Tagen bei 37°C inkubiert. Man ermittelt dann die minimalen Hemmkonzentrationen (MIC) der verschiedenen Wirkstoffe bei der alleinigen und bei der kombinierten Applikation und berechnet aus den ermittelten MIC-Werten die fraktionierte Hemmkonzentration (FIC) nach folgender Formel:

$$FIC = \frac{\text{MIC (TS allein)}}{\text{MIC (TS in Kombination)}} + \frac{\text{MIC (SBH allein)}}{\text{MIC (SBH in Kombination)}}$$

Eine synergistische Wirkung liegt vor, wenn die FIG <0,5 ist. Die in der nachfolgenden Tabelle II enthaltenen Daten für die Verbindung 1 gemäss Tabelle I, einem repräsentativen Vertreter der durch die Formel I definierten Verbindungsklasse, in Kombination mit Ketoconazol, einem repräsentativen Sterol-Biosynthese-Hemmer, belegen die synergistische Wirkung.

Tabelle II

| C.albicans | MIC in µg/ml | | | | |
|---|---|---|---|---|---|
| | Verbindung 1 | Ketoconazol | Verbindung 1 | Ketoconazol | FIC |
| | allein | | in Kombination | | |
| $H_{12}$ | 20 | 5 | 2,5 | 0,3 | 0,19 |
| $H_{29}$ | 10 | 1,29 | 1,2 | 0,075 | 0,19 |
| $H_{42}$ | 20 | 5 | 1,2 | 0,15 | 0,005 |
| $B_5$ | 20 | 0,15 | 1,2 | 0,075 | 0,53 |
| $B_4$ | 20 | 2,5 | 1,2 | 0,15 | 0,06 |

Die Verbindungen der Formel I und ihre pharmazeutisch annehmbaren Säureadditionssalze können als Heilmittel, z.B. in Form pharmazeutischer Präparate zur enteralen, parenteralen oder topischen Applikation, Verwendung finden. Sie können beispielsweise peroral, z.B. in Form von Tabletten, Lacktabletten, Dragées, Hart- und Weichgelatinekapseln, Lösungen, Emulsionen oder Suspensionen, rektal, z.B. in Form von Suppositorien, parenteral, z.B. in Form von Injektionslösungen oder Infusionslösungen, oder topisch, z.B. in Form von Salben, Crèmen oder Oelen, verabreicht werden.

Die Herstellung der pharmazeutischen Präparate kann in jedem Fachmann geläufiger Weise dadurch erfolgen, dass man die beschriebenen Verbindungen der Formel I und ihre pharmazeutisch annehmbaren Säureadditionssalze, gegebenenfalls in Kombination mit anderen therapeutisch wertvollen Stoffen, beispielsweise den erwähnten Sterol-Biosynthese-Hemmern, zusammen mit geeigneten, nicht-toxischen, inerten, therapeutisch verträglichen festen oder flüssigen Trägermaterialien und gegebenenfalls den üblichen pharmazeutischen Hilfsstoffen in eine galenische Darreichungsform bringt.

Für die Kombination mit Verbindungen der Formel I geeignete Sterol-Biosynthese-Hemmer sind beispielsweise die systemisch, antifungal wirksamen Azole vom Typ des Miconazols, z.B. Ketoconazol, Itraconazol und Fluconazol, und die systemisch, antifungal wirksamen Allylamine vom Typ des Naftifins, z.B. Naftifin und Terbinafin.

Als Trägermaterialien eignen sich sowohl anorganische als auch organische Trägermaterialien. So kann man für Tabletten, Lacktabletten, Dragées und Hartgelatinekapseln beispielsweise Lactose, Maisstärke oder Derivate davon, Talk, Stearinsäure oder deren Salze als Trägermaterialien verwenden. Für Weichgelatinekapseln eignen sich als Trägermaterialien beispielsweise pflanzliche Oele, Wachse, Fette und halbfeste und flüssige Polyole (je nach Beschaffenheit des Wirkstoffes sind jedoch bei Weichgelatinekapseln keine Träger erforderlich). Zur Herstellung von Lösungen und Sirupen eignen sich als Trägermaterialien beispielsweise Wasser, Polyole, Saccharose, Invertzucker und Glucose. Für Injektionslösungen eignen sich als Trägermaterialien beispielsweise Wasser, Alkohole, Polyole, Glycerin und pflanzliche Oele. Für Suppositorien eignen sich als Trägermaterialien beispielsweise natürliche oder gehärtete Oele, Wachse, Fette und halbflüssige oder flüssige Polyole. Für topische Präparate eignen sich als Trägermaterialien Glyceride, halbsynthetische und synthetische Glyceride, hydrierte Oele, flüssige Wachse, flüssige Paraffine, flüssige Fettalkohole, Sterole, Polyäthylenglycole und Cellulosederivate.

Als pharmazeutische Hilfsstoffe kommen die üblichen Stabilisierungs-, Konservierungs-, Netz- und Emulgiermittel, Mittel zur Verbesserung der Konsistenz, Mittel zur geschmacklichen Verbesserung, Salze zur Veränderung des osmotischen Druckes, Puffersubstanzen, Lösungsvermittler, Färbe- und Ueberzugsmittel und Antioxidantien in Frage.

Die Dosierung der Verbindungen der Formel I kann, abhängig von den zu bekämpfenden pathogenen Pilzen, dem Alter und dem individuellen Zustand des Patienten und von der Applikationsweise, innerhalb weiter Grenzen variieren und ist natürlich in jedem einzelnen Fall den individuellen Gegebenheiten anzupassen. Zur Verhütung und Bekämpfung von topischen und systemischen Infektionen durch pathogene Pilze kommt für den erwachsenen Patienten im Falle der Monotherapie eine tägliche Dosis von etwa 0,01 g bis etwa 4 g, insbesondere etwa 0,05 g bis etwa 2 g in Betracht. Je nach Dosierung ist es dabei zweckmässig, die Tagesdosis in mehreren Dosierungseinheiten zu verabreichen. Im Falle der Kombinationstherapie kommt eine tägliche Dosis von etwa 0,01 g bis etwa 2 g, insbesondere etwa 0,02 bis etwa 1 g einer Verbindung der Formel I und von etwa 0,02 g bis etwa 0,2 g eines Sterol-Biosynthese-Hemmers in Betracht.

Die pharmazeutischen Monopräparate enthalten zweckmässigerweise etwa 10-1000 mg, vorzugsweise 50-500 mg einer Verbindung der Formel I. Die Kombinationspräparate enthalten zweckmässigerweise etwa 10-500 mg, vorzugsweise 20-250 mg einer Verbindung der Formel I und etwa 50-100 mg eines Sterol-Biosynthese-Hemmers.

Die nachfolgenden Beispiele dienen der näheren Erläuterung der vorliegenden Erfindung. Sie sollen deren Umfang jedoch in keiner Weise beschränken. Sämtliche Temperaturen sind in Celsiusgraden angegeben.

Beispiel 1

a) 9,3 g 1,6-Dibromhexan und 5 g Triphenylphosphin werden unter Argon bei 100° während 1 Stunde gerührt. Anschliessend wird das Reaktionsgemisch abgekühlt und an 90 g Kieselgel mit Methylenchlorid/Methanol 95:5 chromatographiert. Das erhaltene 6-Bromhexyl-phosphoniumbromid (6,37 g, 65%) wird mit 35 ml einer 33-proz. Lösung von Dimethylamin in Aethanol versetzt und während 24 Stunden bei Raumtemperatur unter Argon gerührt. Die Lösung wird eingedampft, und der Rückstand wird im Hochvakuum sorgfältig getrocknet. Das erhaltene 6-(Dimethylamino)hexyl-triphenylphosphoniumbromid-hydrobromid wird in 50 ml Tetrahydrofuran suspendiert, worauf man portionenweise bei 0° mit 2,09 g Kalium-t-butylat versetzt. Das Gemisch wird für 15 Minuten bei 0° gerührt. Anschliessend gibt man bei 0° eine Lösung von 1,86 g 4-Benzoyl-benzaldehyd [Tetrahed. Lett. 24, 4287 (1983)] in 10 ml Tetrahydrofuran dazu und rührt das Gemisch 18 Stunden bei Raumtemperatur. Das Reaktionsgemisch wird eingedampft. Der Rückstand wird in 100 ml 1N Salzsäure aufgenommen und dreimal mit 100 ml Aether extrahiert. Die wässrige Phase wird unter Eiskühlung mit 2N Natronlauge basisch gestellt und dreimal mit Methlyenchlorid extrahiert. Die organischen Phasen werden über Magnesiumsulfat getrocknet und eingedampft. Das erhaltene Rohprodukt wird an 100 g Aluminiumoxid neutral (Aktivitätsstufe III) mit Methylenchlorid/Essigester 95:5 chromatographiert. Nach Eindampfen erhält man 1,98 g (31%) 4-[(Z)-7-(Dimethylamino)-1-heptenyl]benzophenon als farbloses Oel.
$^1$H-NMR ($CDCl_3$): 1,3-1,8 (m;6H); 2,22 (s;6H); 2,29 (t;J = 8Hz;2H); 2,35 (m;2H), 5,80 (dxt;J = 12Hz;J = 7Hz;1H); 6,48 (dxt;J = 12Hz;J = 2Hz;1H); 7,39 (d;J = 8Hz;2H); 7,4-7,8 (m;7H) ppm.

b) Man löst 1,12 g 4-[(Z)-7-(Dimethylamino)-1-heptenyl]benzophenon in 30 ml Methanol, gibt 20 mg 5-proz. Palladium auf Kohle dazu und rührt die Suspension unter Wasserstoff bei Normaldruck und Raumtemperatur während 2,5 Stunden. Man filtriert das Reaktionsgemisch durch Kieselgel und dampft das Filtrat ein. Man erhält 1,10 g (98%) 4-[7-(Dimethylamino)heptyl]benzophenon als farbloses Oel.
$^1$H-NMR ($CDCl_3$): 1,3-1,6 (m;8H); 1,67 (q;J = 7,5Hz;2H); 2,23 (s;6H); 2,29 (t;J = 8Hz;2H); 2,69 (t;J = 7,5Hz;2H); 7,3-7,9 (m;9H) ppm.

Beispiel 2

a) Zu einer Suspension von 5,06 g 6-(Dimethylamino)hexyl-triphenylphosphoniumbromid-Hydrobromid (vgl. Beispiel 1a) in 50 ml Tetrahydrofuran bei 0° unter Argon, gibt man 2,47 g Kalium-t-butylat. Zu dieser Suspension tropft man eine Lösung von 1,64 g p-Formylbenzoesäuremethylester in 20 ml Tetrahydrofuran innerhalb von 15 Minuten dazu. Das Reaktionsgemisch wird bei Raumtemperatur während 18 Stunden gerührt und dann eingedampft. Der Rückstand wird in 100 ml 1N Salzsäure gelöst, und die wässrige Phase wird dreimal mit 100 ml Diäthyläther gewaschen. Die wässrige Phase wird mit festem Kaliumcarbonat basisch gestellt und dreimal mit 100 ml Aether extrahiert. Die ätherischen Phasen

werden über Magnesiumsulfat getrocknet und eingedampft. Der Rückstand wird an 100 g Kieselgel mit Methylenchlorid/Methanol/Ammoniumhydroxid 100:10:1 chromatographiert. Man erhält 880 mg (32%) 4-[-(Z)-7-(Dimethylamino)-1-heptenyl]benzoesäuremethylester als farbloses Oel.

b) Man löst 0,88 g 4-[(Z)-7-(Dimethylamino)-1-heptenyl]benzoesäuremethylester in 20 ml Aethanol, gibt 20 mg 5-proz. Palladium auf Kohle dazu und rührt die Suspension unter Wasserstoff bei Normaldruck und Raumtemperatur während 2 Stunden. Man filtriert das Reaktionsgemisch durch Kieselgur und dampft das Filtrat ein. Man erhält 0,85 g (95%) 4-[7-(Dimethylamino)heptyl]benzoesäuremethylester als farbloses Oel.

c) Zu einer Lösung von 0,85 g 4-[7-(Dimethylamino)heptyl]benzoesäuremethylester in 20 ml Tetrahydrofuran bei -78° unter Argon tropft man eine Lösung von Benzylmagnesiumbromid in 20 ml Aether (aus 74 mg Magnesium und 524 mg Benzylbromid hergestellt) innerhalb von 30 Minuten dazu. Man rührt das Reaktionsgemisch bei -78° während 2 Stunden, giesst es dann auf 50 ml einer gesättigten Ammoniumchloridlösung und extrahiert dreimal mit 50 ml Essigester. Man wäscht die vereinigten organischen Phasen mit 100 ml einer gesättigten Natriumchloridlösung, trocknet über Magnesiumsulfat und dampft ein. Nach Chromatographieren des Rückstandes an 100 g Kieselgel mit Methylenchlorid/Methanol/Ammoniumhydroxid 100:10:1 erhält man 340 mg (35%) 4'-[7-(Dimethylamino)heptyl]-2-phenylacetophenon als gelbliches Oel.

$^1$H-NMR ($CDCl_3$): 1,3-1,7 (m,10H); 2,40 (s,6H); 2,52 (t,J = 8Hz,2H); 2,64 (t,J = 7Hz,2H); 4,26 (s,2H); 7,3-7,4 (m,7H); 7,93 (d,J = 8Hz,2H) ppm.

Beispiel 3

In Analogie zu Beispiel 2c) erhält man aus 4-[7-(Dimethylamino)heptyl]benzoesäuremethylester und

a) Phenäthylmagnesiumbromid das 4'-[7-(Dimethylamino)heptyl]-3-phenylpropiophenon als farbloses Oel (Ausbeute 35%).

$^1$H-NMR ($CDCl_3$): 1,3-1,7 (m,10H); 2,20 (s,6H); 2,1-2,3 (m,2H); 2,64 (t,J = 8Hz,2H); 3,0-3,4 (m,4H); 7,2-7,4 (m,7H); 7,88 (d,J = 8Hz,2H) ppm.

b) p-(Trifluormethyl)phenylmagnesiumbromid das 4-[7-(Dimethylamino)heptyl]-4'-(trifluormethyl)-benzophenon als farbloses Oel (Ausbeute 40%).

$^1$H-NMR ($CDCl_3$): 1,3-1,7 (m,10H); 2,24 (s,6H); 2,28 (t,J = 8Hz,2H); 2,69 (t,J = 8Hz,2H); 7,29 (d,J = 9Hz,2H); 7,7-7,9 (m,6H) ppm.

MS: 391 (1,5%, $M^+$); 372 (1%); 235 (2,5%); 173 (3,8%); 145 (4,5%); 58 (100%).

Beispiel 4

a) 1,2 g 4-[7-(Dimethylamino)heptyl]benzoesäuremethylester werden mit 25 ml Methanol und 10 ml 20-proz. Natronlauge in Wasser versetzt, worauf man während 2 Stunden unter Rückfluss erhitzt. Nach Abkühlen auf 0° stellt man das pH der Lösung mit Essigsäure auf 5 ein. Das ausgefallene Material wird abgenutscht und im Hochvakuum bei ca. 6,7 Pa über Nacht getrocknet. Man erhält 0,97 g (85%) 4-[7-(Dimethylamino)heptylbenzoesäure.

b) Man suspendiert dieses Material in 20 ml Methylenchlorid, versetzt mit 1,2 ml Oxalylchlorid, rührt das Reaktionsgemisch bei Raumtemperatur während 2 Stunden und dampft dann ein. Das rohe Säurechlorid wird im Hochvakuum getrocknet, in 3 ml Fluorbenzol gelöst, und die Lösung wird auf 0° abgekühlt. Zu dieser Lösung gibt man 760 mg Aluminiumchlorid dazu und rührt 30 Minuten bei 0° und dann während 2 Stunden bei Raumtemperatur. Man verdünnt das Reaktionsgemisch mit 50 ml Methylenchlorid und wäscht es zweimal mit 50 ml 2N Natronlauge. Man trocknet die Methylenchloridphase über Magnesiumsulfat und dampft ein. Das Rohprodukt wird an 100 g Kieselgel mit Methylenchlorid/Methanol/Ammoniumhydroxid 90:10:1 gereinigt. Man erhält 0,5 g (40%) 4-[7-(Dimethylamino)heptyl]-4'-fluorbenzophenon als farbloses Oel.

$^1$H-NMR ($CDCl_3$): 1,3-1,7 (m,10H); 2,23 (s,6H); 2,26 (t,J = 7,5Hz,2H); 2,68 (t,J = 7,5Hz,2H); 7,1-7,2 (m,2H); 7,28 (d,J = 8Hz,2H); 7,70 (d,J = 8Hz,2H), 7,7-7,9 (m,2H) ppm.

Beispiel 5

In Analogie zu Beispiel 4b) erhält man aus 4-[7-(Dimethylamino)heptyl]benzoesäure und 1,3-Difluorbenzol das 2,4-Difluor-4'-[7-(dimethylamino)heptyl]benzophenon als farbloses Oel (Ausbeute 32%).

$^1$H-NMR ($CDCl_3$): 1,3-1,7 (m,10H); 2,25 (s,6H); 2,28 (t,J = 7Hz,2H); 2,67 (t,J = 8Hz,2H); 6,8-7,8 (m,7H) ppm.

Beispiel 6

Zu einer Suspension von 486 mg Magnesium-Spänen in 10 ml Tetrahydrofuran tropft man unter Argon innerhalb von 30 Minuten eine Lösung von 6,1 g 2-(p-Bromphenyl)-2-phenyl-1,3-dioxolan (Deutsche Offenlegungsschrift Nr. 2509474) in 50 ml Tetrahydrofuran. Die erhaltene braune Lösung wird bei Raumtemperatur während 1 Stunde gerührt, dann auf 0° abgekühlt und zu einer Lösung von 5,12 g Dibromhexan und 0,1 mmol Dilithiumtetrachlorocuprat (Synthesis, 1971, 303) in 10 ml Tetrahydrofuran innerhalb von einer Stunde bei 0° zugetropft. Man rührt das Gemisch 18 Stunden bei Raumtemperatur und dampft ein. Der Rückstand wird mit 100 ml einer gesättigten Ammoniumchloridlösung versetzt und dreimal mit 100 ml Aether extrahiert. Die organischen Phasen werden über Magnesiumsulfat getrocknet und eingedampft. Der Rückstand enthaltend 2-[4-(6-Bromhexyl)phenyl]-2-phenyl-1,3-dioxolan, wird mit 25 ml einer 33-proz. Lösung von Dimethylamin in Aethanol versetzt und während 24 Stunden bei Raumtemperatur gerührt. Die Lösung wird eingeengt; der Rückstand, enthaltend 2-[4-[6-(Dimethylamino)hexyl]phenyl]-2-phenyl-1,3-dioxolan, wird in 100 ml 1N Salzsäure aufgenommen und dreimal mit 100 ml Aether gewaschen. Die wässrige Phase wird unter Eiskühlung mit 2N Natronlauge basisch gestellt und dreimal mit 100 ml Aether extrahiert. Die organischen Phasen werden über Magnesiumsulfat getrocknet und eingedampft. Das rohe Produkt wird durch Destillation im Kugelrohrofen bei 180-190° und ca. 6,7 Pa gereinigt. Man erhält 2,55 g (41%) 4-[6-(Dimethylamino)hexyl]benzophenon als leicht gelbes Oel.

Dieses Oel wird in 5 ml Aethanol gelöst und zu einer heissen Lösung von 0,956 g Fumarsäure in 20 ml Aethanol gegeben. Nach Zugabe von 50 ml Aether und Abkühlen auf 0° werden die farblosen Kristalle abgenutscht, mit Aether gewaschen und getrocknet. Man erhält 3 g (85%) 4-[6-(Dimethylamino)hexyl]-benzophenon-fumarat 1:1 mit einem Smp. von 87-89°.

Beispiel 7

a) 15 g 4-(3-Brompropyl)acetophenon und 10,8 ml Pyrrolidin werden in 60 ml Aethanol gelöst und während 24 Stunden auf 40° erwärmt. Dann wird das Reaktionsgemisch eingedampft, und der Rückstand wird mit 250 ml Essigester und 150 ml halbgesättigter Kochsalzlösung versetzt, worauf man ausschüttelt. Die wässrige Phase wird nochmals mit 250 ml Essigester extrahiert, und die organischen Phasen werden nochmals mit 150 ml halbgesättigter Kochsalzlösung gewaschen. Die vereinigten organischen Extrakte werden über Magnesiumsulfat getrocknet und eingedampft. Man erhält 13,8 g (96%) 4'-[3-(1-Pyrrolidinyl)propyl]acetophenon als bräunliche Flüssigkeit; Massenspektrum m/e: $M^+$ 431 (6,4), 84 (100), 42 (10,9).

b) 4 g 4'-[3-(1-Pyrrolidinyl)propyl]acetophenon und 2 g Benzaldehyd werden in 80 ml Methanol gelöst und unter Eisbadkühlung mit einer Lösung von 9,55 g Kaliumcarbonat in 38 ml Wasser innert 15 Minuten versetzt. Dann lässt man 18 Stunden bei Raumtemperatur und weitere 18 Stunden bei ca. 40° reagieren. Das Reaktionsgemisch wird in 200 ml Essigester aufgenommen und mit 100 ml Wasser ausgeschüttelt. Die wässrige Phase wird nochmals mit 200 ml Essigester, und die organischen Phasen werden nochmals mit 100 ml halbgesättigter Kochsalzlösung ausgezogen. Die vereinigten organischen Phasen werden über Magnesiumsulfat getrocknet und eingedampft. Der Rückstand wird an Kieselgel mit Aceton chromatographiert. Man erhält 4,5 g (81%) (E)-3-Phenyl-4'-[3-(1-pyrrolidinyl)propyl]acrylophenon als gelbes Oel; Massenspektrum m/e: $M^+$ 319 (7.1), 131 (1,4), 84 (100), 42 (7,5). Zur Bildung des Hydrochlorids wird das freie Amin in wenig Aethanol gelöst und mit 10M äthanolischer Salzsäurelösung versetzt. Nach Umkristallisieren des erhaltenen Materials aus Isopropanol erhält man das reine Hydrochlorid; Smp. 211-213°.

Beispiel 8

4,1 g 4-[3-(Dimethylamino)propyl]acetophenon und 3,1 g 4-Chlorbenzaldehyd werden in 80 ml Methanol gelöst und bei Raumtemperatur innert 15 Minuten mit einer Lösung von 6,4 g Natriumhydroxid in 32 ml Wasser versetzt. Dann lässt man 48 Stunden bei Raumtemperatur rühren. Zur Aufarbeitung wird auf 150 ml Eiswasser gegossen. Die ausgefallenen Kristalle werden abgenutscht, dreimal mit je 30 ml Wasser gewaschen und bei 40° unter reduziertem Druck getrocknet. Man erhält 6,36 g (97%) (E)-3-(4-Chlorphenyl) -4'-[3-(dimethylamino)propyl]acrylophenon; Smp. 83-86°.

Das 4-[3-(Dimethylamino)propyl]acetophenon kann in Analogie zu Beispiel 7a) hergestellt werden.

Beispiel 9

3 g (E)-3-(4-Chlorphenyl)-4'-[3-(dimethylamino)propyl]acrylophenon werden in 100 ml Aethanol gelöst und nach Zugabe von 300 mg 5-proz. Palladium auf Kohle hydriert. Nach Aufnahme der theoretischen Wasserstoffmenge wird der Katalysator abfiltriert, und das Filtrat wird eingedampft. Der Rückstand wird in Aethanol gelöst und mittels 10M äthanolischer Salzsäurelösung in das Hydrochlorid übergeführt, welches durch Zugabe von Hexan ausgefällt wird. Nach Umkristallisieren aus Essigester/Aethanol 5:1 erhält man 1,44 g (43%) 3-(4-Chlorphenyl)-4'-[3-(dimethylamino)propyl]propiophenon-Hydrochlorid; Smp. 161-164°.

Beispiel 10

In Analogie zu den Beispielen 7b) und 8 können die nachfolgend aufgeführten Verbindungen hergestellt werden:

a) (E)-3-(4-Methylphenyl-4'-[3-(dimethylamino]propyl]acrylophenon; Smp. 76,5-77,5°;

b) (E)-3-(4-Isopropylphenyl)-4'-[3-(dimethylamino]propyl]acrylophenon; Smp. 44-45°;

c) (E)-3-(4-Methoxyphenyl)-4'-[3-(dimethylamino]propyl]acrylophenon als Oel; $^1$H-NMR ($CDCl_3$, 400 MHz): 2,24 (s,6H); 3,86 (s,3H) ppm;

d) (E)-3-[4-(Trifluormethyl)phenyl]-4'-[3-(dimethylamino]propyl]acrylophenon; Smp. 71,5-73,5°;

e) (E)-3-(3-Methylphenyl)-4'-[3-(dimethylamino]propyl]acrylophenon als Oel; $^1$H-NMR ($CDCl_3$, 400 MHz): 2,25 (s,6H); 2,40 (s,3H) ppm;

f) (E)-3-(4-Nitrophenyl)-4'-[3-(dimethylamino)propyl]acrylophenon; Smp. 97-99°;

g) (E)-3-(3-Chlorphenyl)-4'-[3-(dimethylamino]propyl]acrylophenon als Wachs; Massenspektrum m/e: $M^+$ 327 (1,1), 205 (0,8), 143 (0,9), 58 (100);

h) (E)-3-Phenyl-4'-[3-(äthylmethylamino)propyl]acrylophenon als Oel; Massenspektrum m/e: $M^+$ 307 (4), 72 (100) (das als Ausgangsstoff verwendete 4-[3-(Aethylmethylamino)propyl]acetophenon kann in Analogie zu Beispiel 7a) hergestellt werden);

i) (E)-3-(4-t.Butylphenyl)-4'-[3-(dimethylamino)propyl]acrylophenon-hydrochlorid; Smp. 217-219° (aus Aethanol);

j) (E)-3-(3,5-Dichlorphenyl)-4'-[3-(dimethylamino)propyl]acrylophenon-hydrochlorid; Smp. >230° (Zersetzung);

k) (E)-3-(4-Fluorphenyl)-4'-[3-(dimethylamino)propyl]acrylophenon-hydrochlorid; Smp. 219-220,5° (aus Aethanol);

l) (E)-3-(2-Methylphenyl)-4'-[3-(dimethylamino)propyl]acrylophenon-hydrochlorid; Smp. 150,5-152° (aus Aethanol/Toluol).

Beispiel 11

In Analogie zu Beispiel 9 können die nachfolgend aufgeführten Verbindungen hergestellt werden:

a) 3-(4-Methylphenyl-4'-[3-(dimethylamino)propyl]propiophenon als Oel; $^1$H-NMR ($CDCl_3$, 400 MHz): 2,22 (s,6H); 2,32 (s,3H) ppm;

b) 3-(4-Isopropylphenyl)-4'-[3-(dimethylamino)propyl]propiophenon als Oel; Massenspektrum m/e: $M^+$ 337 (5), 133 (4), 58 (100);

c) 3-(4-Methoxyphenyl)-4'-[3-(dimethylamino)propyl]propiophenon als Sirup; Massenspektrum m/e: $M^+$ 325 (4), 121 (9), 58 (100);

d) 3-[4-(Trifluormethyl)phenyl]-4'-[3-(dimethylamino)propyl]propiophenon als Oel; Massenspektrum m/e: $M^+$ 363 (2), 159 (3), 58 (100);

e) 3-(3-Methylphenyl)-4'-[3-(dimethylamino)propyl]propiophenon als Oel; Massenspektrum m/e: $M^+$ 309 (10,4), 145 (3,8), 105 (7,0), 58 (100);

f) 3-(3-Chlorphenyl)-4'-[3-(dimethylamino)propyl]propiophenon als Oel; Massenspektrum m/e: $M^+$ 329 (1,0), 125 (2,5), 58 (100);

g) 3-Phenyl-4'-[3-(äthylmethylamino)propyl]propiophenon als Sirup; Massenspektrum m/e: $M^+$ 309 (6), 294 (1), 91 (6), 72 (100);

h) 3-(4-t-Butylphenyl)-4'-[3-(dimethylamino)propyl]propiophenon als Oel; Massenspektrum m/e: $M^+$ 351 (34,7), 131 (32,8), 91 (34,5), 58 (100);

i) 3-(4-Fluorphenyl)-4'-[3-(dimethylamino)propyl]propiophenon als Sirup; Massenspektrum m/e: $M^+$ 313 (3), 109 (11), 58 (100);

j) (3-(2-Methylphenyl)-4'-[3-(dimethylamino)propyl]propiophenon; Smp. 145-147° (aus Tetrahydrofuran);

k) 3-Phenyl-4'-[3-(1-pyrrolidinyl)propyl]propiophenon als Sirup; Massenspektrum m/e: $M^+$ 321 (7), 91 (6), 84 (100).

Beispiel 12

In Analogie zu Beispiel 1 können die nachfolgend aufgeführten Verbindungen hergestellt werden:

a) 4-[5-(Dimethylamino)pentyl]benzophenon als farbloses Oel (Ausbeute 96%).
Massenspektrum m/e: $M^+$ 295 (2%, $M^+$), 100 (3%), 58 (100%).

b) 4-[8-(Dimethylamino)octyl]benzophenon als farbloses Oel (Ausbeute 88%).
Massenspektrum m/e: $M^+$ 337 (2%, $M^+$), 149 (2,8%), 105 (3,3%), 58 (100%).

Beispiel 13

a) Zu einer Lösung von 10 g 7-Chlorheptanol (Rec. Trav. Chim. Pays-Bas, 99, 87 (1980)) und 18,7 ml Triäthylamin in 100 ml Methylenchlorid unter Argon bei 0 °C tropft man eine Lösung von 13,3 g p-Toluolsulfonylchlorid in 100 ml Methylenchlorid innerhalb von 1 Stunde. Das Reaktionsgemisch wird bei Raumtemparatur während 6 Stunden gerührt, dann zweimal mit je 200 ml 2N Salzsäure, zweimal mit je 200 ml gesättigter Natriumbicarbonatlösung und einmal mit 200 ml gesättigter Natriumchloridlösung gewaschen. Die organische Phase wird über Magnesiumsulfat getrocknet und eingeengt. Das rohe 7-Chlor-heptyl-p-toluolsulfonat (19,5 g, 96%) wird in 40 ml Tetrahydrofuran mit 0,6 mMol Dilithiumtetrachlorcuprat gelöst. Diese Lösung wird unter Argon auf 0 °C abgekühlt. Man tropft dann eine Lösung von Phenylmagnesiumbromid (hergestellt aus 3,1 g Magnesiumspänen und 20 g Brombenzol in 80 ml Tetrahydrofuran) innerhalb von zwei Stunden dazu. Man rührt das Reaktionsgemisch während 18 Stunden bei Raumtemperatur und dampft es ein.

Der Rückstand wird mit 200 ml einer gesättigten Ammoniumchloridlösung versetzt und dreimal mit je 200 ml Aether extrahiert. Die organischen Phasen werden über Magnesiumsulfat getrocknet und eingedampft. Der Rückstand wird im Hochvakuum bei 100-110 °C und etwa 13,4 Pa destilliert. Man erhält 13 g (86%) 7-Chlorheptylbenzol als farblose Flüssigkeit.

b) 1,07 g 7-Chlorheptylbenzol und 0,89 g 4-Chlorbenzoylchlorid werden in 10 ml Nitrobenzol gelöst und unter Argon und unter Eiskühlung mit 0,82 g Aluminiumchlorid versetzt. Dann rührt man während 18 Stunden bei Raumtemperatur, nimmt in 100 ml eiskalter 2N Salzsäurelösung auf und extrahiert dreimal mit je 50 ml Aether. Die organischen Phasen werden mit 50 ml einen 10-proz. Natriumbicarbonatlösung und 50 ml einer gesättigten Natriumchloridlösung gewaschen, über Magnesiumsulfat getrocknet und eingeengt. Man entfernt das Nitrobenzol durch Destillation bei 80-100 °C und 20 Pa. Durch Destillation bei 240-250 °C und etwa 13,4 Pa erhält man 1,32 g (74%) 4-Chlor-4'-(7-chlorheptyl)benzophenon als leicht gelbes Oel.
$^1$H-NMR ($CDCl_3$): 1,2-1,9 (m, 9H); 1,72 (t,J = 7,5 Hz, 2H); 3,53 (t,J = 7,2 Hz, 2H); 7,2-7,9 (m, 8H) ppm.

c) 1,32 g 4-Chlor-4'-(7-chlorheptyl)benzophenon und 0,85 g Natriumjodid werden unter Rückfluss in 20 ml Aethylmethylketon erwärmt. Nach 24 Stunden wird das Reaktionsgemisch eingedampft. Der Rückstand wird in 50 ml Wasser suspendiert, worauf man dreimal mit je 50 ml Essigester extrahiert, die vereinigten organischen Phasen mit 50 ml einer gesättigten Natriumchloridlösung wäscht, über Magnesiumsulfat trocknet und eindampft. Man erhält 1,5 g (90%) rohes 4-Chlor-4'-(7-jodheptyl)benzophenon als gelbliches Oel, das man ohne weitere Reinigung in die nächste Stufe einsetzt.

d) Man versetzt dieses rohe Oel mit 10 ml einer 33-proz. Lösung von Dimethylamin in Aethanol, rührt während 24 Stunden bei Raumtemperatur, dampft das Reaktionsgemisch ein, nimmt den Rückstand in 50 ml 1N Salzsäure auf und wäscht dreimal mit je 50 ml Aether. Die wässrige Phase wird unter Eiskühlung mit 2N Natronlauge basisch gestellt und dreimal mit je 50 ml Aether extrahiert. Die organischen Phasen werden über Magnesiumsulfat getrocknet und eingedampft. Das rohe Produkt wird an 100 g Kieselgel mit Methylenchlorid/Methanol/Ammoniumchlorid 90:10:1 chromatographiert. Man erhält 0,6 g (51%) 4-Chlor-4'-[7-(dimethylamino)heptyl]benzophenon als farbloses Oel.
Massenspektrum m/e: 357 (0,6%, $M^+$), 128 (4%), 58 (100%).

Beispiel 14

In Analogie zu Beispiel 13 können die nachfolgend aufgeführten Verbindungen hergestellt werden:

a) 4-Brom-4'-[7-(dimethylamin)heptyl]benzophenon als farbloses Oel (Ausbeute 38%). Durch Zugabe mit ätherischer Salzsäure wird das entsprechende Hydrochlorid mit einem Schmelzpunkt von 122-123 °C erhalten (Ausbeute 85%).

b) 2,4-Dichlor-4'-[7-(dimethylamino)heptyl]benzophenon als farbloses Oel (Ausbeute 73%). Massenspektrum m/e: 391 (0,3%, $M^+$), 173 (1,3%), 128 (4,2%), 58 (100%).
c) 4-[4-(Dimethylamino)heptyl]benzoyl]benzonitril als farbloses Oel (Ausbeute 68%). Massenspektrum m/e: 348 (0,6%, $M^+$), 128 (2,7%), 58 (100%).
d) 4-[7-(Allylmethylamino)heptyl]benzophenon (Ausbeute 66%) als farbloses Oel. Massenspektrum m/e: 349 (1,7%, $M^+$), 167 (4,3%), 84 (100%).
e) 4-[7-(Allylmethylamino)heptyl]-4'-brombenzophenon als leicht gelbes Oel (Ausbeute 62%). Massenspektrum m/e: 427 (0,7%, $M^+$), 400 (3,3%), 84 (100%).
f) 4-[4'-[7-(Allylmethylamino)heptyl]benzoyl]benzonitril als gelbes Oel (Ausbeute 46%). Massenspektrum m/e: 374 (2%, $M^+$), 345 (8%), 84 (100%).
g) 4-Brom-4'-[6-(dimethylamino)hexyl]benzophenon-hydrochlorid (Ausbeute 72%) als farblose Kristalle mit einem Schmelzpunkt von 115-117 °C.
h) 4-[6-(Allylmethylamino)hexyl]-4'-brombenzophenon (Ausbeute 77%) als farbloses Oel. Massenspektrum m/e: 415 (0,8%, $M^+$), 413 (0,8%), 386 (2%), 84 (100%).
i) 3-Brom-4'-[6-(dimethylamino)hexyl]benzophenon (Ausbeute 48%) als leicht gelbes Oel. Massenspektrum m/e: 387 (0,3%, $M^+$), 114 (7%), 58 (100%).
j) 4-[6-Dimethylamino)hexyl]-2'-methylbenzophenon (Ausbeute 36%) als leicht gelbes Oel. Massenspektrum m/e: 325 (0,9%, $M^+$), 114 (8%), 58 (100%).
k) 4-[6-(Dimethylamino)hexyl]-4'-nitrobenzophenon (Ausbeute 80%) als gelbes Oel. Massenspektrum m/e: 354 (0,4%, $M^+$), 114 (6%), 58 (100%).
l) 2-Chlor-4'-[6-(dimethylamino)hexyl]benzophenon (Ausbeute 59%). Durch Behandeln mit ätherischer Salzsäure erhält man das entsprechende Hydrochlorid mit einem Schmelzpunkt von 176-178 °C (Ausbeute 86%).
m) 4-[7-(Dimethylamino)heptyl]-2-methylbenzophenon als farbloses Oel. Massenspektrum m/e: 337 (1,5%, $M^+$), 128 (6%), 105 (4%), 77 (3%), 58 (100%).

Beispiel A

Die Verbindung 4-[7-(Dimethylamino)heptyl]benzophenon kann wie folgt als Wirkstoff zur Herstellung von Tabletten verwendet werden:

| Bestandteile | mg/Tablette |
|---|---|
| Wirkstoff | 200 |
| Milchzucker pulv. | 100 |
| Povidone K 30 | 15 |
| Na-Carboxymethylstärke | 10 |
| Talk | 3 |
| Magnesiumstearat | 2 |
| Tablettengewicht | 330 |

Der Wirkstoff und der Milchzucker pulv. werden intensiv gemischt. Die erhaltene Mischung wird dann mit einer wässrigen Lösung von Povidone K 30 befeuchtet und geknetet, worauf man die erhaltene Masse granuliert, trocknet und siebt. Man mischt das Granulat mit den übrigen Bestandteilen und verpresst dann zu Tabletten geeigneter Grösse.

**Patentansprüche**
**Patentansprüche für folgende Vertragsstaaten : AT, BE, CH, DE, DK, FR, GB, IT, LI, LU, NL, SE**

**1.** Verbindungen der allgemeinen Formel

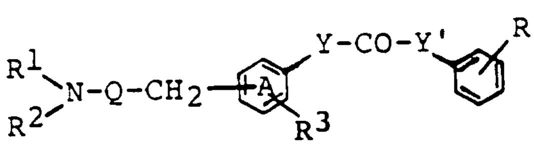

I

worin $R^1$ und $R^2$ je Wasserstoff, niederes Alkyl oder niederes Alkenyl oder zusammen geradkettiges Alkylen mit 2 bis 4 Kohlenstoffatomen, $R^3$ Wasserstoff, Halogen oder niederes Alkyl, Q Alkylen mit 2 bis 11 Kohlenstoffatomen und mindestens 2 Kohlenstoffatomen zwischen den zwei freien Valenzen oder Alkenylen mit 4 bis 11 Kohlenstoffatomen und mindestens 4 Kohlenstoffatomen zwischen den zwei freien Valenzen und Y und Y' je eine direkte Bindung oder die Gruppe $-CH_2-$, $-CH_2CH_2-$,$-CH=CH-$ oder $-C\equiv C-$ bedeuten, die Gruppe $R^1R^2N-Q-CH_2-$ an die 3- oder 4-Stellung des mit A bezeichneten Ringes gebunden ist, und das Symbol R bedeutet, dass der Ring unsubstituiert oder durch Halogen, Trifluormethyl, Cyano, Nitro, niederes Alkyl oder niederes Alkoxy substituiert ist,
und ihre pharmazeutisch annehmbaren Säureadditionssalze zur Anwendung als therapeutische Wirkstoffe.

**2.** Verbindungen nach Anpruch 1, worin $R^1$ und $R^2$ je Wasserstoff oder niederes Alkyl oder zusammen geradkettiges Alkylen mit 2 bis 4 Kohlenstoffatomen, $R^3$ Wasserstoff, Q Alkylen mit 2 bis 11 Kohlenstoffatomen und mindestens 2 Kohlenstoffatomen zwischen den zwei freien Valenzen und Y und Y' je eine direkte Bindung oder die Gruppe $-CH_2-$, $-CH_2CH_2-$, $-CH=CH-$ oder $-C\equiv C-$ bedeuten, die Gruppe $R^1R^2N-O-CH_2-$ an die 3- oder 4-Stellung des mit A bezeichneten Ringes gebunden ist, und das Symbol R bedeutet, dass der Ring unsubstituiert oder durch Halogen, Trifluormethyl, Nitro, niederes Alkyl oder niederes Alkoxy substituiert ist, und ihre pharmazeutisch annehmbaren Säureadditionssalze zur Anwendung als therapeutische Wirkstoffe.

**3.** Verbindungen der allgemeinen Formel

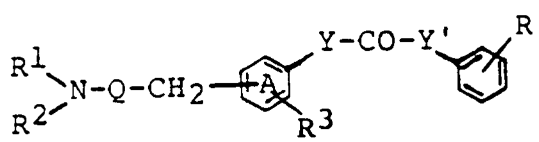

I

worin $R^1$ und $R^2$ je Wasserstoff, niederes Alkyl oder niederes Alkenyl oder zusammen geradkettiges Alkylen mit 2 bis 4 Kohlenstoffatomen, $R^3$ Wasserstoff, Halogen oder niederes Alkyl, Q Alkylen mit 2 bis 11 Kohlenstoffatomen und mindestens 2 Kohlenstoffatomen zwischen den zwei freien Valenzen oder Alkenylen mit 4 bis 11 Kohlenstoffatomen und mindestens 4 Kohlenstoffatomen zwischen den zwei freien Valenzen und Y und Y' je eine direkte Bindung oder die Gruppe $-CH_2-$, $-CH_2CH_2-$,$-CH=CH-$ oder $-C\equiv C-$ bedeuten, die Gruppe $R^1R^2N-O-CH_2-$ an die 3- oder 4-Stellung des mit A bezeichneten Ringes gebunden ist, und das Symbol R bedeutet, dass der Ring unsubstituiert oder durch Halogen, Trifluormethyl, Cyano, Nitro, niederes Alkyl oder niederes Alkoxy substituiert ist, wobei Y und Y' nicht gleichzeitig eine direkte Bindung bedeuten, wenn $R^1$ und $R^2$ je Wasserstoff oder niederes Alkyl oder zuammen geradkettiges Alkylen mit 2 bis 4 Kohlenstoffatomen, $R^3$ Wasserstoff und Q geradkettiges Alkylen mit weniger als 4 Kohlenstoffatomen bedeuten,
und ihre pharmazeutisch annehmbaren Säureadditionssalze.

**4.** Verbindungen nach Anspruch 3, worin $R^1$ und $R^2$ je Wasserstoff oder niederes Alkyl oder zusammen geradkettiges Alkylen mit 2 bis 4 Kohlenstoffatomen, $R^3$ Wasserstoff, Q Alkylen mit 2 bis 11 Kohlenstoffatomen und mindestens 2 Kohlenstoffatomen zwischen den zwei freien Valenzen und Y und Y' je eine direkte Bindung oder die Gruppe $-CH_2-$. $-CH_2CH_2-$, $-CH=CH-$ oder $-C\equiv C-$ bedeuten, die Gruppe $R^1R^2N-Q-CH_2-$ an die 3- oder 4-Stellung des mit A bezeichneten Ringes gebunden ist, und das Symbol R bedeutet, dass der Ring unsubstituiert oder durch Halogen, Trifluormethyl, Nitro, niederes Alkyl oder niederes Alkoxy substituiert ist, wobei Y und Y' nicht gleichzeitig eine direkte Bindung bedeuten, wenn $R^3$ Wasserstoff und Q geradkettiges Alkylen mit weniger als 4 Kohlenstoffatomen bedeuten.

**5.** Verbindungen nach Anspruch 3, worin Q geradkettiges Alkylen mit 4 bis 7 Kohlenstoffatomen bedeutet.

**6.** Verbindungen nach Anspruch 5, worin $R^1$ und $R^2$ je $C_{1-4}$-Alkyl oder $C_{3-4}$-Alkenyl oder zusammen $C_{3-4}$-Alkylen bedeuten.

**7.** Verbindungen nach Anspruch 5 oder 6, worin $R^3$ Wasserstoff bedeutet.

**8.** Verbindungen nach einem der Ansprüche 5 bis 7, worin die Gruppe $R^1R^2N$-Q-$CH_2$- an die 4-Stellung des mit A bezeichneten Ringes gebunden ist.

**9.** Verbindungen nach einem der Ansprüche 5 bis 8, worin Y eine direkte Bindung oder die Gruppe -$CH_2$- bedeutet.

**10.** Verbindungen nach einem der Ansprüche 5 bis 9, worin Y' eine direkte Bindung oder die Gruppe -$CH_2$-, -$CH_2CH_2$- oder -CH=CH- bedeutet.

**11.** Verbindungen nach einem der Ansprüche 5 bis 10, worin das Symbol R bedeutet, dass der Ring unsubstituiert oder durch Halogen, Trifluormethyl, Nitro oder niederes Alkyl substituiert ist.

**12.** 4-[7-(Dimethylamino)heptyl]benzophenon.

**13.** 4'-[7-(Dimethylamino)heptyl]-2-phenylacetophenon.

**14.** 4'-Fluor-4-[7-(dimethylamino)heptyl]benzophenon.

**15.** 4-[7-(Dimethylamino)heptyl]-4'-(trifluormetehyl]benzophenon.

**16.** 2,4-Difluor-4'-[7-(dimethylamino)heptyl]benzophenon.

**17.** Verbindungen nach Anspruch 3, worin Q geradkettiges Alkylen mit 2 oder 3 Kohlenstoffatomen bedeutet.

**18.** Verbindungen nach Anspruch 17, worin $R^1$ und $R^2$ je $C_{1-4}$-Alkyl oder $C_{3-4}$-Alkenyl oder zusammen $C_{3-4}$-Alkylen bedeuten.

**19.** Verbindungen nach Anspruch 17 oder 18, worin $R^3$ Wasserstoff bedeutet.

**20.** Verbindungen nach einem der Ansprüche 17 bis 19, worin die Gruppe $R^1R^2N$-Q-$CH_2$- an die 4-Stellung des mit A bezeichneten Ringes gebunden ist.

**21.** Verbindungen nach einem der Ansprüche 17 bis 20, worin Y eine direkte Bindung und Y' die Gruppe -$CH_2$-, -$CH_2CH_2$- oder -CH=CH- bedeuten.

**22.** Verbindungen nach einem der Ansprüche 17 bis 21, worin das Symbol R bedeutet, dass der Ring unsubstituiert oder durch Halogen, Trifluormethyl, Nitro oder niederes Alkyl substituiert ist.

**23.** 3-(4-Chlorphenyl)-4'-[3-(dimethylamino)propyl]propiophenon.

**24.** 3-(2-Methylphenyl)-4'-[3-(dimethylamino)propyl]propiophenon.

**25.** (E)-3-Phenyl-4'-[3-(1-pyrrolidinyl)propyl]acrylophenon.

**26.** Verbindungen nach einem der Ansprüche 3 bis 25 zur Anwendung als therapeutische Wirkstoffe.

**27.** Verbindungen nach einem der Ansprüche 1 bis 25 zur Anwendung als antifungal wirksame Stoffe.

**28.** Verbindungen der allgemeinen Formel

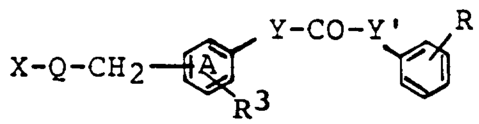

II

worin X eine Abgangsgruppe, $R^3$ Wasserstoff, Halogen oder niederes Alkyl, Q Alkylen mit 2 bis 11

Kohlenstoffatomen und mindestens 2 Kohlenstoffatomen zwischen den zwei freien Valenzen oder Alkenylen mit 4 bis 11 Kohlenstoffatomen und mindestens 4 Kohlenstoffatomen zwischen den zwei freien Valenzen und Y und Y' je eine direkte Bindung oder die Gruppe $-CH_2-$, $-CH_2CH_2-$, -CH=CH- oder -C≡C- bedeuten, die Gruppe $X-Q-CH_2-$ an die 3- oder 4-Stellung des mit A bezeichneten Ringes gebunden ist, und das Symbol R bedeutet, dass der Ring unsubstituiert oder durch Halogen, Trifluormethyl, Cyano, Nitro, niederes Alkyl oder niederes Alkoxy substituiert ist, wobei Y und Y' nicht gleichzeitig eine direkte Bindung bedeuten, wenn $R^3$ Wasserstoff und Q geradkettiges Alkylen mit weniger als 4 Kohlenstoffatomen bedeuten.

**29.** Verbindungen der allgemeinen Formel

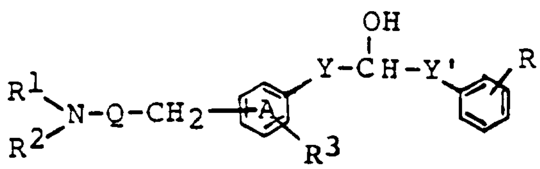

III

worin $R^1$ und $R^2$ je Wasserstoff, niederes Alkyl oder niederes Alkenyl oder zusammen geradkettiges Alkylen mit 2 bis 4 Kohlenstoffatomen, $R^3$ Wasserstoff, Halogen oder niederes Alkyl, Q Alkylen mit 2 bis 11 Kohlenstoffatomen und mindestens 2 Kohlenstoffatomen zwischen den zwei freien Valenzen oder Alkenylen mit 4 bis 11 Kohlenstoffatomen und mindestens 4 Kohlenstoffatomen zwischen den zwei freien Valenzen und Y und Y' je eine direkte Bindung oder die Gruppe $-CH_2-$, $-CH_2CH_2-$, -CH=CH- oder -C≡C- bedeuten, die Gruppe $R^1R^2N-Q^-CH_2-$ an die 3- oder 4-Stellung des mit A bezeichneten Ringes gebunden ist, und das Symbol R bedeutet, dass der Ring unsubstituiert oder durch Halogen, Trifluormethyl, Cyano, Nitro, niederes Alkyl oder niederes Alkoxy substituiert ist, wobei Y und Y' nicht gleichzeitig eine direkte Bindung bedeuten, wenn $R^1$ und $R^2$ je Wasserstoff oder niederes Alkyl oder zusammen geradkettiges Alkylen mit 2 bis 4 Kohlenstoffatomen, $R^3$ Wasserstoff und Q geradkettiges Alkylen mit weniger als 4 Kohlenstoffatomen bedeuten.

**30.** Verbindungen der allgemeinen Formel

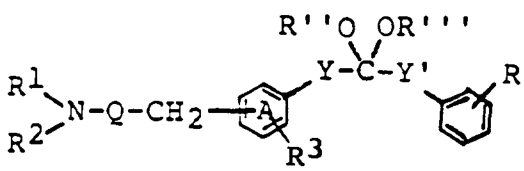

VI

worin R'' und R''' je niederes Alkyl oder zusammen Dimethylen oder Trimethylen, $R^1$ und $R^2$ je Wasserstoff, niederes Alkyl oder niederes Alkenyl oder zusammen geradkettiges Alkylen mit 2 bis 4 Kohlenstoffatomen, $R^3$ Wasserstoff, Halogen oder niederes Alkyl, Q Alkylen mit 2 bis 11 Kohlenstoffatomen und mindestens 2 Kohlenstoffatomen zwischen den zwei freien Valenzen oder Alkenylen mit 4 bis 11 Kohlenstoffatomen und mindestens 4 Kohlenstoffatomen zwischen den zwei freien Valenzen und Y und Y' je eine direkte Bindung oder die Gruppe $-CH_2-$, $-CH_2CH_2-$,-CH=CH- oder -C≡C- bedeuten, die Gruppe $R^1R^2N-Q-CH_2-$ an die 3- oder 4-Stellung des mit A bezeichneten Ringes gebunden ist, und das Symbol R bedeutet, dass der Ring unsubstituiert oder durch Halogen, Trifluormethyl, Cyano, Nitro, niederes Alkyl oder niederes Alkoxy substituiert ist, wobei Y und Y' nicht gleichzeitig eine direkte Bindung bedeuten, wenn $R^1$ und $R^2$ je Wasserstoff oder niederes Alkyl oder zusammen geradkettiges Alkylen mit 2 bis 4 Kohlenstoffatomen, $R^3$ Wasserstoff und Q geradkettiges Alkylen mit weniger als 4 Kohlenstoffatomen bedeuten.

**31.** Verfahren zur Herstellung von Verbindungen nach einem der Ansprüche 3 bis 25 und von pharmazeutisch annehmbaren Säureadditionssalzen davon, dadurch gekennzeichnet, dass man

a) eine Verbindung der allgemeinen Formel

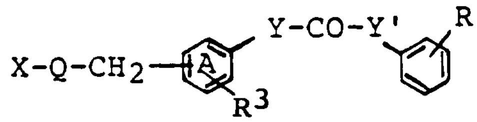

II

worin X eine Abgangsgruppe bedeutet, und A, $R^3$, Q, Y, Y' und R die in Anspruch 3 angegebene Bedeutung besitzen,
mit einem Amin der allgemeinen Formel $HNR^1R^2$, worin $R^1$ und $R^2$ die in Anspruch 3 angegebene Bedeutung besitzen, umsetzt, oder
b) eine Verbindung der allgemeinen Formel

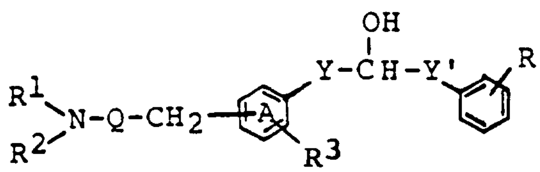

III

worin A, $R^1$, $R^2$, $R^3$, Q, Y, Y' und R die in Anspruch 3 angegebene Bedeutung besitzen,
oxidiert, oder
c) eine Verbindung der allgemeinen Formel

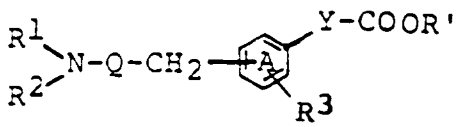

IV

worin $R^1$ niederes Alkyl bedeutet, und A, $R^1$, $R^2$, $R^3$, Q und Y die in Anspruch 3 angegebene Bedeutung besitzen,
mit einer Verbindung der allgemeinen Formel

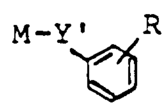

V

worin M -MgCl, -MgBr, -MgJ oder -Li bedeutet, und Y' und R die in Anspruch 3 angegebene Bedeutung besitzen,
umsetzt, oder
d) eine Verbindung der allgemeinen Formel

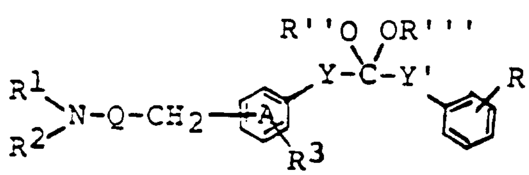

VI

worin R'' und R''' je niederes Alkyl oder zusammen Dimethylen oder Trimethylen bedeuten, und A, $R^1$, $R^2$, $R^3$, Q, Y, Y' und R die in Anspruch 3 angegebene Bedeutung besitzen,
mit einer wässrigen Säure behandelt, oder

e) eine Verbindung der allgemeinen Formel

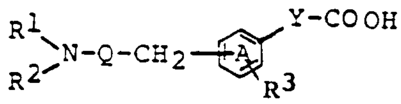

VIIa

oder

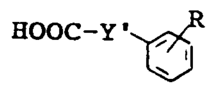

VIIb

in Form eines reaktiven Derivates in Gegenwart einer Lewis-Säure mit einer Verbindung der allgemeinen Formel

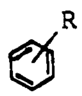

VIIIa

bzw.

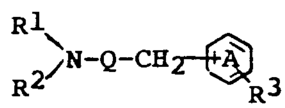

VIIIb

worin A, $R^1$, $R^2$, $R^3$, Q, Y, Y' und R die in Anspruch 3 angegebene Bedeutung besitzen, umsetzt, oder

f) eine Verbindung der allgemeinen Formel

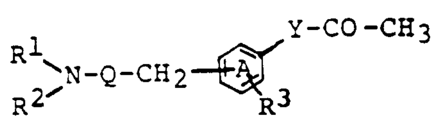

IX

worin A, $R^1$, $R^2$, $R^3$, Q und Y die in Anspruch 3 angegebene Bedeutung besitzen, in Gegenwart einer Base mit einer Verbindung der allgemeinen Formel

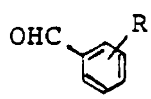

X

worin R die in Anspruch 3 angegebene Bedeutung besitzt, umsetzt, oder

g) eine Verbindung der allgemeinen Formel

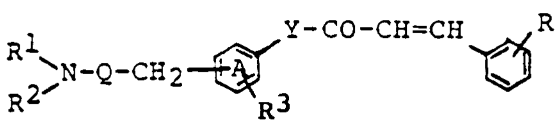

Ia

oder

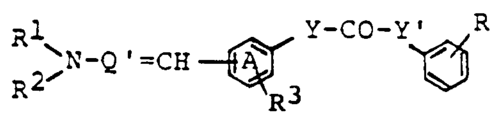

XI

worin Q' die um ein Wasserstoffatom verminderte Gruppe Q bedeutet, und A, $R^1$,$R^2$, $R^3$, Q, Y, Y' und R die in Anspruch 3 angegebene Bedeutung besitzen, hydriert, und

h) eine erhaltene Verbindung der Formel I erwünschtenfalls in ein pharmazeutisch annehmbares Säureadditionssalz überführt.

**32.** Arzneimittel, enthaltend eine Verbindung gemäss einem der Ansprüche 1 bis 25 und einen therapeutisch inerten Träger.

**33.** Antifungal wirksame Mittel, enthaltend eine Verbindung gemäss einem der Ansprüche 1 bis 25 und einen therapeutisch inerten Träger.

**34.** Arzneimittel nach Anspruch 32 oder 33, dadurch gekennzeichnet, dass sie als zusätzlichen Wirkstoff eine bekannte antifungal wirksame Substanz, welche die Sterol-Biosynthese hemmt, enthalten.

**35.** Verwendung von Verbindungen gemäss einem der Ansprüche 1 bis 25 gegebenenfalls in Kombination mit bekannten antifungal wirksamen Substanzen, welche die Sterol-Biosynthese hemmen, für die Herstellung von antifungal wirksamen Mitteln.

**Patentansprüche für folgenden Vertragsstaat : ES**

**1.** Verfahren zur Herstellung von Verbindungen der allgemeinen Formel

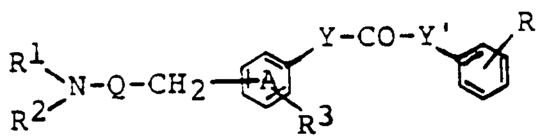

I

worin $R^1$ und $R^2$ je Wasserstoff, niederes Alkyl oder niederes Alkenyl oder zusammen geradkettiges Alkylen mit 2 bis 4 Kohlenstoffatomen, $R^3$ Wasserstoff, Halogen oder niederes Alkyl, Q Alkylen mit 2 bis 11 Kohlenstoffatomen und mindestens 2 Kohlenstoffatomen zwischen den zwei freien Valenzen oder Alkenylen mit 4 bis 11 Kohlenstoffatomen und mindestens 4 Kohlenstoffatomen zwischen den zwei freien Valenzen und Y und Y' je eine direkte Bindung oder die Gruppe $-CH_2-$, $-CH_2CH_2-$,$-CH=CH-$ oder $-C \equiv C-$ bedeuten, die Gruppe $R^1R^2N-Q-CH_2-$ an die 3- oder 4-Stellung des mit A bezeichneten Ringes gebunden ist, und das Symbol R bedeutet, dass der Ring unsubstituiert oder durch Halogen, Trifluormethyl, Cyano, Nitro, niederes Alkyl oder niederes Alkoxy substituiert ist, wobei Y und Y' nicht gleichzeitig eine direkte Bindung bedeuten, wenn $R^1$ und $R^2$ je Wasserstoff oder niederes Alkyl oder zuammen geradkettiges Alkylen mit 2 bis 4 Kohlenstoffatomen, $R^3$ Wasserstoff und Q geradkettiges Alkylen mit weniger als 4 Kohlenstoffatomen bedeuten,
und ihren pharmazeutisch annehmbaren Säureadditionssalzen, dadurch gekennzeichnet, dass man

a) eine Verbindung der allgemeinen Formel

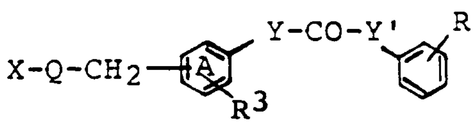

II

worin X eine Abgangsgruppe bedeutet, und A, $R^3$, Q, Y, Y' und R obige Bedeutung besitzen,
mit einem Amin der allgemeinen Formel $HNR^1R^2$, worin $R^1$ und $R^2$ obige Bedeutung besitzen, umsetzt, oder

b) eine Verbindung der allgemeinen Formel

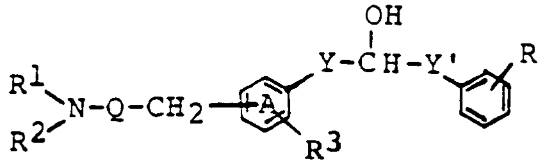

III

worin A, $R^1$, $R^2$, $R^3$, Q, Y, Y' und R obige Bedeutung besitzen,
oxidiert, oder

c) eine Verbindung der allgemeinen Formel

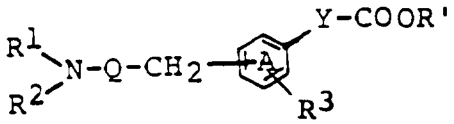

IV

worin R' niederes Alkyl bedeutet, und A, $R^1$, $R^2$, $R^3$, Q und Y obige Bedeutung besitzen, mit einer Verbindung der allgemeinen Formel

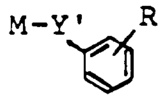

V

worin M -MgCl, -MgBr, -MgJ oder -Li bedeutet, und Y' und R obige Bedeutung besitzen, umsetzt, oder

d) eine Verbindung der allgemeinen Formel

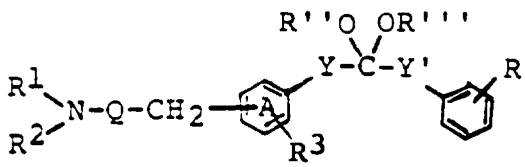

VI

worin R'' und R''' je niederes Alkyl oder zusammen Dimethylen oder Trimethylen bedeuten, und A, $R^1$, $R^2$, $R^3$, Q, Y, Y' und R obige Bedeutung besitzen, mit einer wässrigen Säure behandelt, oder

e) eine Verbindung der allgemeinen Formel

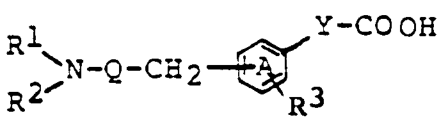

oder

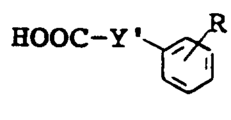

VIIb

VIIa

in Form eines reaktiven Derivates in Gegenwart einer Lewis-Säure mit einer Verbindung der allgemeinen Formel

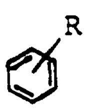

VIIIa

bzw.

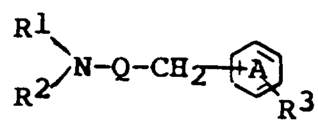

VIIIb

worin A, $R^1$, $R^2$, $R^3$, Q, Y, Y' und R obige Bedeutung besitzen, umsetzt, oder

f) eine Verbindung der allgemeinen Formel

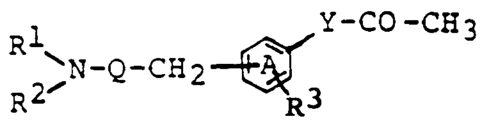

IX

worin A, $R^1$, $R^2$, $R^3$, Q und Y obige Bedeutung besitzen, in Gegenwart einer Base mit einer Verbindung der allgemeinen Formel

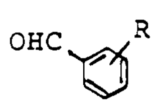

X

worin R obige Bedeutung besitzt,

umsetzt, oder

g) eine Verbindung der allgemeinen Formel

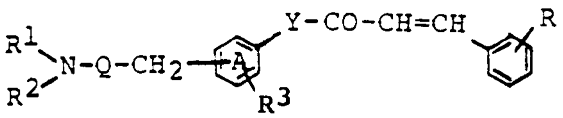

oder

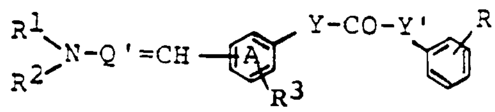

worin Q' die um ein Wasserstoffatom verminderte Gruppe Q bedeutet, und A, $R^1$,$R^2$, $R^3$, Q, Y, Y' und R obige Bedeutung besitzen,

hydriert, und

h) eine erhaltene Verbindung der Formel I erwünschtenfalls in ein pharmazeutisch annehmbares Säureadditionssalz überführt.

**2.** Verfahren nach Anspruch 1, worin $R^1$ und $R^2$ je Wasserstoff oder niederes Alkyl oder zusammen geradkettiges Alkylen mit 2 bis 4 Kohlenstoffatomen, $R^3$ Wasserstoff, Q Alkylen mit 2 bis 11 Kohlenstoffatomen und mindestens 2 Kohlenstoffatomen zwischen den zwei freien Valenzen und Y und Y' je eine direkte Bindung oder die Gruppe $-CH_2-$, $-CH_2CH_2-$, -CH = CH- oder -C≡C- bedeuten, die Gruppe $R^1R^2N-Q-CH_2-$ an die 3- oder 4-Stellung des mit A bezeichneten Ringes gebunden ist, und das Symbol R bedeutet, dass der Ring unsubstituiert oder durch Halogen, Trifluormethyl, Nitro, niederes Alkyl oder niederes Alkoxy substituiert ist, wobei Y und Y' nicht gleichzeitig eine direkte Bindung bedeuten, wenn $R^3$ Wasserstoff und Q geradkettiges Alkylen mit weniger als 4 Kohlenstoffatomen bedeuten.

**3.** Verfahren nach Anspruch 1, worin Q geradkettiges Alkylen mit 4 bis 7 Kohlenstoffatomen bedeutet.

**4.** Verfahren nach Anspruch 3, worin $R^1$ und $R^2$ je $C_{1-4}$-Alkyl oder $C_{3-4}$-Alkenyl oder zusammen $C_{3-4}$-Alkylen bedeuten.

**5.** Verfahren nach Anspruch 3 oder 4, worin $R^3$ Wasserstoff bedeutet.

**6.** Verfahren nach einem der Ansprüche 3 bis 5, worin die Gruppe $R^1R^2N-Q-CH_2-$ an die 4-Stellung des mit A bezeichneten Ringes gebunden ist.

**7.** Verfahren nach einem der Ansprüche 3 bis 6, worin Y eine direkte Bindung oder die Gruppe $-CH_2-$ bedeutet.

**8.** Verfahren nach einem der Ansprüche 3 bis 7, worin Y' eine direkte Bindung oder die Gruppe $-CH_2-$, $-CH_2CH_2-$ oder -CH = CH- bedeutet.

**9.** Verfahren nach einem der Ansprüche 3 bis 8, worin das Symbol R bedeutet, dass der Ring unsubstituiert oder durch Halogen, Trifluormethyl, Nitro oder niederes Alkyl substituiert ist.

**10.** Verfahren nach Anspruch 1, dadurch gekennzeichnet, dass man 4-[7-(Dimethylamino)heptyl]-benzophenon herstellt.

**11.** Verfahren nach Anspruch 1, dadurch gekennzeichnet, dass man 4'-[7-(Dimethylamino)heptyl]-2-phenylacetophenon herstellt.

**12.** Verfahren nach Anspruch 1, dadurch gekennzeichnet, dass man 4'-Fluor-4-[7-(dimethylamino)heptyl]-benzophenon herstellt.

**13.** Verfahren nach Anspruch 1, dadurch gekennzeichnet, dass man 4-[7-(Dimethylamino)heptyl]-4'-(trifluormetehyl]benzophenon herstellt.

**14.** Verfahren nach Anspruch 1, dadurch gekennzeichnet, dass man 2,4-Difluor-4'-[7-(dimethylamino)-heptyl]benzophenon herstellt.

**15.** Verfahren nach Anspruch 1, worin Q geradkettiges Alkylen mit 2 oder 3 Kohlenstoffatomen bedeutet.

**16.** Verfahren nach Anspruch 15, worin $R^1$ und $R^2$ je $C_{1-4}$-Alkyl oder $C_{3-4}$-Alkenyl oder zusammen $C_{3-4}$-Alkylen bedeuten.

**17.** Verfahren nach Anspruch 15 oder 16, worin $R^3$ Wasserstoff bedeutet.

**18.** Verfahren nach einem der Ansprüche 15 bis 17, worin die Gruppe $R^1R^2N$-Q-$CH_2$- an die 4-Stellung des mit A bezeichneten Ringes gebunden ist.

**19.** Verfahren nach einem der Ansprüche 15 bis 18, worin Y eine direkte Bindung und Y' die Gruppe -$CH_2$-, -$CH_2CH_2$- oder -CH = CH- bedeuten.

**20.** Verfahren nach einem der Ansprüche 15 bis 19, worin das Symbol R bedeutet, dass der Ring unsubstituiert oder durch Halogen, Trifluormethyl, Nitro oder niederes Alkyl substituiert ist.

**21.** Verfahren nach Anspruch 1, dadurch gekennzeichnet, dass man 3-(4-Chlorphenyl)-4'-[3-(dimethylamino)propyl]propiophenon herstellt.

**22.** Verfahren nach Anspruch 1, dadurch gekennzeichnet, dass man 3-(2-Methylphenyl)-4'-[3-(dimethylamino)propyl]propiophenon herstellt.

**23.** Verfahren nach Anspruch 1, dadurch gekennzeichnet, dass man (E)-3-Phenyl-4'-[3-(1-pyrrolidinyl)-propyl]acrylophenon herstellt.

**24.** Verfahren zur Herstellung von Arzneimitteln, insbesondere von antifungal wirksamen Mitteln, dadurch gekennzeichnet, dass man eine Verbindung der allgemeinen Formel

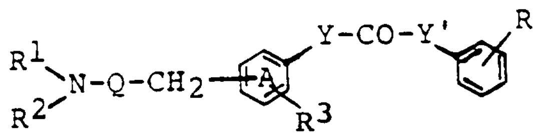

I

worin $R^1$ und $R^2$ je Wasserstoff, niederes Alkyl oder niederes Alkenyl oder zusammen geradkettiges Alkylen mit 2 bis 4 Kohlenstoffatomen, $R^3$ Wasserstoff, Halogen oder niederes Alkyl, Q Alkylen mit 2 bis 11 Kohlenstoffatomen und mindestens 2 Kohlenstoffatomen zwischen den zwei freien Valenzen oder Alkenylen mit 4 bis 11 Kohlenstoffatomen und mindestens 4 Kohlenstoffatomen zwischen den zwei freien Valenzen und Y und Y' je eine direkte Bindung oder die Gruppe -$CH_2$-, -$CH_2CH_2$-,-CH = CH- oder -C≡C- bedeuten, die Gruppe $R^1R^2N$-Q-$CH_2$- an die 3- oder 4-Stellung des mit A bezeichneten Ringes gebunden ist, und das Symbol R bedeutet, dass der Ring unsubstituiert oder durch Halogen, Trifluormethyl, Cyano, Nitro, niederes Alkyl oder niederes Alkoxy substituiert ist,
oder ein pharmazeutisch annehmbares Säureadditionssalz davon zusammen mit einem therapeutisch inerten Träger in eine galenische Darreichungsform bringt.

**25.** Verfahren zur Herstellung von antifungal wirksamen Mitteln, dadurch gekennzeichnet, dass man eine Verbindung der in Anspruch 24 definierten Formel I oder ein pharmazeutisch annehmbares Säureadditionssalz davon zusammen mit einer bekannten antifungal wirksamen Substanz, welche die Sterol-Biosynthese hemmt, und einen therapeutisch inerten Träger in eine galenische Darreichungsform bringt.

**26.** Verwendung von Verbindungen der in Anspruch 24 definierten Formel I und ihrer pharmazeutisch annehmbaren Säureadditionssalze gegebenenfalls in Kombination mit bekannten antifungal wirksamen Substanzen, welche die Sterol-Biosynthese hemmen, für die Herstellung von antifungal wirksamen Mitteln.

**Patentansprüche für folgenden Vertragsstaat : GR**

**1.** Verfahren zur Herstellung von Verbindungen der allgemeinen Formel

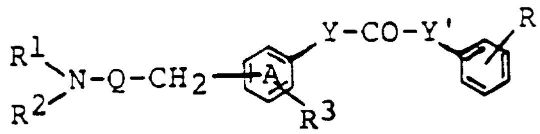

I

worin $R^1$ und $R^2$ je Wasserstoff, niederes Alkyl oder niederes Alkenyl oder zusammen geradkettiges Alkylen mit 2 bis 4 Kohlenstoffatomen, $R^3$ Wasserstoff, Halogen oder niederes Alkyl, Q Alkylen mit 2 bis 11 Kohlenstoffatomen und mindestens 2 Kohlenstoffatomen zwischen den zwei freien Valenzen oder Alkenylen mit 4 bis 11 Kohlenstoffatomen und mindestens 4 Kohlenstoffatomen zwischen den zwei freien Valenzen und Y und Y' je eine direkte Bindung oder die Gruppe $-CH_2-$, $-CH_2CH_2-$,$-CH=CH-$ oder $-C{\equiv}C-$ bedeuten, die Gruppe $R^1R^2N-Q-CH_2-$ an die 3- oder 4-Stellung des mit A bezeichneten Ringes gebunden ist, und das Symbol R bedeutet, dass der Ring unsubstituiert oder durch Halogen, Trifluormethyl, Cyano, Nitro, niederes Alkyl oder niederes Alkoxy substituiert ist, wobei Y und Y' nicht gleichzeitig eine direkte Bindung bedeuten, wenn $R^1$ und $R^2$ je Wasserstoff oder niederes Alkyl oder zuammen geradkettiges Alkylen mit 2 bis 4 Kohlenstoffatomen, $R^3$ Wasserstoff und Q geradkettiges Alkylen mit weniger als 4 Kohlenstoffatomen bedeuten,
und ihren pharmazeutisch annehmbaren Säureadditionssalzen, dadurch gekennzeichnet, dass man

a) eine Verbindung der allgemeinen Formel

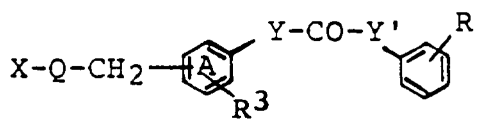

II

worin X eine Abgangsgruppe bedeutet, und A, $R^3$, Q, Y, Y' und R obige Bedeutung besitzen,
mit einem Amin der allgemeinen Formel $HNR^1R^2$, worin $R^1$ und $R^2$ obige Bedeutung besitzen, umsetzt, oder

b) eine Verbindung der allgemeinen Formel

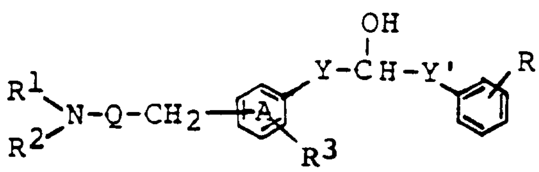

III

worin A, $R^1$, $R^2$, $R^3$, Q, Y, Y' und R obige Bedeutung besitzen,
oxidiert, oder

c) eine Verbindung der allgemeinen Formel

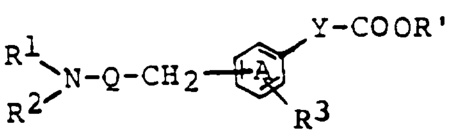

IV

worin R' niederes Alkyl bedeutet, und A, R', $R^2$, $R^3$, Q und Y obige Bedeutung besitzen,
mit einer Verbindung der allgemeinen Formel

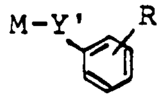

V

worin M -MgCl, -MgBr, -MgJ oder -Li bedeutet, und Y' und R obige Bedeutung besitzen, umsetzt, oder

d) eine Verbindung der allgemeinen Formel

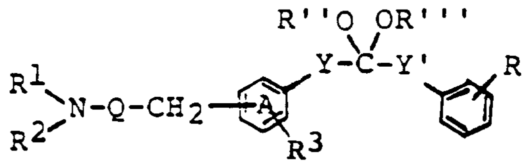

VI

worin R'' und R''' je niederes Alkyl oder zusammen Dimethylen oder Trimethylen bedeuten, und A, $R^1$, $R^2$, $R^3$, Q, Y, Y' und R obige Bedeutung besitzen, mit einer wässrigen Säure behandelt, oder

e) eine Verbindung der allgemeinen Formel

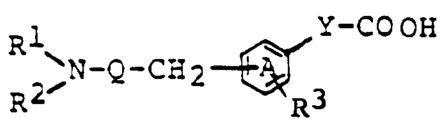

oder

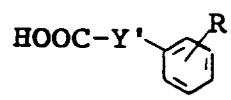

VIIb

VIIa

in Form eines reaktiven Derivates in Gegenwart einer Lewis-Säure mit einer Verbindung der allgemeinen Formel

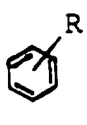

VIIIa

bzw.

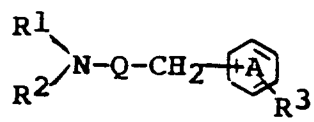

VIIIb

worin A, $R^1$, $R^2$, $R^3$, Q, Y, Y' und R obige Bedeutung besitzen, umsetzt, oder

f) eine Verbindung der allgemeinen Formel

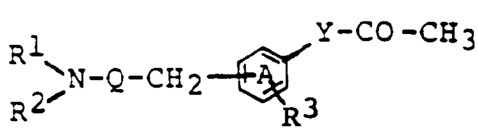

IX

worin A, $R^1$, $R^2$, $R^3$, Q und Y obige Bedeutung besitzen, in Gegenwart einer Base mit einer Verbindung der allgemeinen Formel

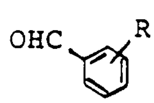

X

worin R obige Bedeutung besitzt, umsetzt, oder

g) eine Verbindung der allgemeinen Formel

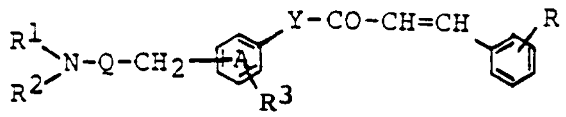

oder

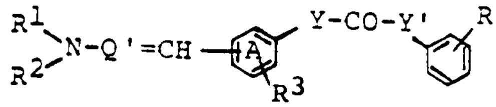

worin Q' die um ein Wasserstoffatom verminderte Gruppe Q bedeutet, und A, $R^1$,$R^2$ $R^3$, Q, Y, Y' und R obige Bedeutung besitzen,
hydriert, und
h) eine erhaltene Verbindung der Formel I erwünschtenfalls in ein pharmazeutisch annehmbares Säureadditionssalz überführt.

**2.** Verfahren nach Anspruch 1, worin $R^1$ und $R^2$ je Wasserstoff oder niederes Alkyl oder zusammen geradkettiges Alkylen mit 2 bis 4 Kohlenstoffatomen, $R^3$ Wasserstoff, Q Alkylen mit 2 bis 11 Kohlenstoffatomen und mindestens 2 Kohlenstoffatomen zwischen den zwei freien Valenzen und Y und Y' je eine direkte Bindung oder die Gruppe $-CH_2-$, $-CH_2CH_2-$, -CH = CH- oder -C≡C- bedeuten, die Gruppe $R^1R^2N-Q-CH_2-$ an die 3- oder 4-Stellung des mit A bezeichneten Ringes gebunden ist, und das Symbol R bedeutet, dass der Ring unsubstituiert oder durch Halogen, Trifluormethyl, Nitro, niederes Alkyl oder niederes Alkoxy substituiert ist, wobei Y und Y' nicht gleichzeitig eine direkte Bindung bedeuten, wenn $R^3$ Wasserstoff und Q geradkettiges Alkylen mit weniger als 4 Kohlenstoffatomen bedeuten.

**3.** Verfahren nach Anspruch 1, worin Q geradkettiges Alkylen mit 4 bis 7 Kohlenstoffatomen bedeutet.

**4.** Verfahren nach Anspruch 3, worin $R^1$ und $R^2$ je $C_{1-4}$-Alkyl oder $C_{3-4}$-Alkenyl oder zusammen $C_{3-4}$-Alkylen bedeuten.

**5.** Verfahren nach Anspruch 3 oder 4, worin $R^3$ Wasserstoff bedeutet.

**6.** Verfahren nach einem der Ansprüche 3 bis 5, worin die Gruppe $R^1R^2N-Q-CH_2-$ an die 4-Stellung des mit A bezeichneten Ringes gebunden ist.

**7.** Verfahren nach einem der Ansprüche 3 bis 6, worin Y eine direkte Bindung oder die Gruppe $-CH_2-$ bedeutet.

**8.** Verfahren nach einem der Ansprüche 3 bis 7, worin Y' eine direkte Bindung oder die Gruppe $-CH_2-$, $-CH_2CH_2-$ oder -CH = CH- bedeutet.

**9.** Verfahren nach einem der Ansprüche 3 bis 8, worin das Symbol R bedeutet, dass der Ring unsubstituiert oder durch Halogen, Trifluormethyl, Nitro oder niederes Alkyl substituiert ist.

**10.** Verfahren nach Anspruch 1, dadurch gekennzeichnet, dass man 4-[7-(Dimethylamino)heptyl]-benzophenon herstellt.

**11.** Verfahren nach Anspruch 1, dadurch gekennzeichnet, dass man 4'-[7-(Dimethylamino)heptyl]-2-phenylacetophenon herstellt.

**12.** Verfahren nach Anspruch 1, dadurch gekennzeichnet, dass man 4'-Fluor-4-[7-(dimethylamino)heptyl]-benzophenon herstellt.

**13.** Verfahren nach Anspruch 1, dadurch gekennzeichnet, dass man 4-[7-(Dimethylamino)heptyl]-4'-(trifluormetehyl]benzophenon herstellt.

**14.** Verfahren nach Anspruch 1, dadurch gekennzeichnet, dass man 2,4-Difluor-4'-[7-(dimethylamino)-heptyl]benzophenon herstellt.

**15.** Verfahren nach Anspruch 1, worin Q geradkettiges Alkylen mit 2 oder 3 Kohlenstoffatomen bedeutet.

**16.** Verfahren nach Anspruch 15, worin $R^1$ und $R^2$ je $C_{1-4}$-Alkyl oder $C_{3-4}$-Alkenyl oder zusammen $C_{3-4}$-Alkylen bedeuten.

**17.** Verfahren nach Anspruch 15 oder 16, worin $R^3$ Wasserstoff bedeutet.

**18.** Verfahren nach einem der Ansprüche 15 bis 17, worin die Gruppe $R^1R^2N$-Q-$CH_2$- an die 4-Stellung des mit A bezeichneten Ringes gebunden ist.

**19.** Verfahren nach einem der Ansprüche 15 bis 18, worin Y eine direkte Bindung und Y' die Gruppe -$CH_2$-, -$CH_2CH_2$- oder -CH=CH- bedeuten.

**20.** Verfahren nach einem der Ansprüche 15 bis 19, worin das Symbol R bedeutet, dass der Ring unsubstituiert oder durch Halogen, Trifluormethyl, Nitro oder niederes Alkyl substituiert ist.

**21.** Verfahren nach Anspruch 1, dadurch gekennzeichnet, dass man 3-(4-Chlorphenyl)-4'-[3-(dimethylamino)propyl]propiophenon herstellt.

**22.** Verfahren nach Anspruch 1, dadurch gekennzeichnet, dass man 3-(2-Methylphenyl)-4'-[3-(dimethylamino)propyl]propiophenon herstellt.

**23.** Verfahren nach Anspruch 1, dadurch gekennzeichnet, dass man (E)-3-Phenyl-4'-[3-(1-pyrrolidinyl)-propyl]acrylophenon herstellt.

**24.** Verfahren zur Herstellung von Arzneimitteln, insbesondere von antifungal wirksamen Mitteln, dadurch gekennzeichnet, dass man eine Verbindung der allgemeinen Formel

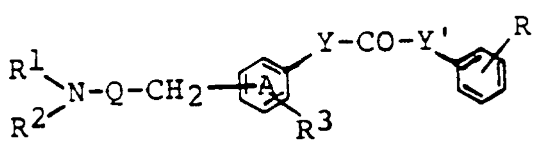

I

worin $R^1$ und $R^2$ je Wasserstoff, niederes Alkyl oder niederes Alkenyl oder zusammen geradkettiges Alkylen mit 2 bis 4 Kohlenstoffatomen, $R^3$ Wasserstoff, Halogen oder niederes Alkyl, Q Alkylen mit 2 bis 11 Kohlenstoffatomen und mindestens 2 Kohlenstoffatomen zwischen den zwei freien Valenzen oder Alkenylen mit 4 bis 11 Kohlenstoffatomen und mindestens 4 Kohlenstoffatomen zwischen den zwei freien Valenzen und Y und Y' je eine direkte Bindung oder die Gruppe -$CH_2$-, -$CH_2CH_2$-,-CH=CH- oder -C≡C- bedeuten, die Gruppe $R^1R^2N$-Q-$CH_2$- an die 3- oder 4-Stellung des mit A bezeichneten Ringes gebunden ist, und das Symbol R bedeutet, dass der Ring unsubstituiert oder durch Halogen, Trifluormethyl, Cyano, Nitro, niederes Alkyl oder niederes Alkoxy substituiert ist,
oder ein pharmazeutisch annehmbares Säureadditionssalz davon zusammen mit einem therapeutisch inerten Träger in eine galenische Darreichungsform bringt.

**25.** Verfahren zur Herstellung von antifungal wirksamen Mitteln, dadurch gekennzeichnet, dass man eine Verbindung der in Anspruch 24 definierten Formel I oder ein pharmazeutisch annehmbares Säureadditionssalz davon zusammen mit einer bekannten antifungal wirksamen Substanz, welche die Sterol-Biosynthese hemmt, und einen therapeutisch inerten Träger in eine galenische Darreichungsform bringt.

**26.** Verwendung von Verbindungen der in Anspruch 24 definierten Formel I und ihrer pharmazeutisch annehmbaren Säureadditionssalze gegebenenfalls in Kombination mit bekannten antifungal wirksamen Substanzen, welche die Sterol-Biosynthese hemmen, für die Herstellung von antifungal wirksamen Mitteln.

**27.** Verbindungen der allgemeinen Formel

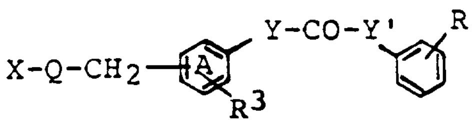

II

worin X eine Abgangsgruppe, $R^3$ Wasserstoff, Halogen oder niederes Alkyl, Q Alkylen mit 2 bis 11 Kohlenstoffatomen und mindestens 2 Kohlenstoffatomen zwischen den zwei freien Valenzen oder Alkenylen mit 4 bis 11 Kohlenstoffatomen und mindestens 4 Kohlenstoffatomen zwischen den zwei freien Valenzen und Y und Y' je eine direkte Bindung oder die Gruppe $-CH_2-$, $-CH_2CH_2-$, -CH = CH- oder -C≡C- bedeuten,
die Gruppe X-Q-$CH_2$- an die 3- oder 4-Stellung des mit A bezeichneten Ringes gebunden ist, und das Symbol R bedeutet, dass der Ring unsubstituiert oder durch Halogen, Trifluormethyl, Cyano, Nitro, niederes Alkyl oder niederes Alkoxy substituiert ist, wobei Y und Y' nicht gleichzeitig eine direkte Bindung bedeuten, wenn $R^3$ Wasserstoff und Q geradkettiges Alkylen mit weniger als 4 Kohlenstoffatomen bedeuten.

**28.** Verbindungen der allgemeinen Formel

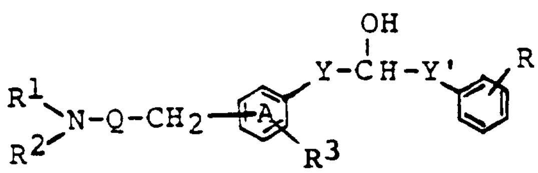

III

worin $R^1$ und $R^2$ je Wasserstoff, niederes Alkyl oder niederes Alkenyl oder zusammen geradkettiges Alkylen mit 2 bis 4 Kohlenstoffatomen, $R^3$ Wasserstoff, Halogen oder niederes Alkyl, Q Alkylen mit 2 bis 11 Kohlenstoffatomen und mindestens 2 Kohlenstoffatomen zwischen den zwei freien Valenzen oder Alkenylen mit 4 bis 11 Kohlenstoffatomen und mindestens 4 Kohlenstoffatomen zwischen den zwei freien Valenzen und Y und Y' je eine direkte Bindung oder die Gruppe $-CH_2-$, $-CH_2CH_2-$, -CH = CH- oder -C≡C- bedeuten,
die Gruppe $R^1R^2$N-Q-$CH_2$- an die 3- oder 4-Stellung des mit A bezeichneten Ringes gebunden ist, und das Symbol R bedeutet, dass der Ring unsubstituiert oder durch Halogen, Trifluormethyl, Cyano, Nitro, niederes Alkyl oder niederes Alkoxy substituiert ist, wobei Y und Y' nicht gleichzeitig eine direkte Bindung bedeuten, wenn $R^1$ und $R^2$ je Wasserstoff oder niederes Alkyl oder zusammen geradkettiges Alkylen mit 2 bis 4 Kohlenstoffatomen, $R^3$ Wasserstoff und Q geradkettiges Alkylen mit weniger als 4 Kohlenstoffatomen bedeuten.

**29.** Verbindungen der allgemeinen Formel

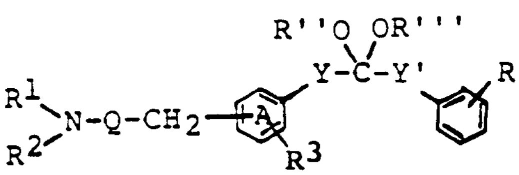

VI

worin R'' und R''' je niederes Alkyl oder zusammen Dimethylen oder Trimethylen, $R^1$ und $R^2$ je Wasserstoff, niederes Alkyl oder niederes Alkenyl oder zusammen geradkettiges Alkylen mit 2 bis 4 Kohlenstoffatomen, $R^3$ Wasserstoff, Halogen oder niederes Alkyl, Q Alkylen mit 2 bis 11 Kohlenstoffatomen und mindestens 2 Kohlenstoffatomen zwischen den zwei freien Valenzen oder Alkenylen mit 4 bis 11 Kohlenstoffatomen und mindestens 4 Kohlenstoffatomen zwischen den zwei freien Valenzen und Y und Y' je eine direkte Bindung oder die Gruppe $-CH_2-$, $-CH_2CH_2-$,-CH = CH- oder -C≡C- bedeuten, die Gruppe $R^1R^2$N-Q-$CH_2$- an die 3- oder 4-Stellung des mit A bezeichneten Ringes gebunden ist, und das

Symbol R bedeutet, dass der Ring unsubstituiert oder durch Halogen, Trifluormethyl, Cyano, Nitro, niederes Alkyl oder niederes Alkoxy substituiert ist, wobei Y und Y' nicht gleichzeitig eine direkte Bindung bedeuten, wenn $R^1$ und $R^2$ je Wasserstoff oder niederes Alkyl oder zusammen geradkettiges Alkylen mit 2 bis 4 Kohlenstoffatomen, $R^3$ Wasserstoff und Q geradkettiges Alkylen mit weniger als 4 Kohlenstoffatomen bedeuten.

## Claims

**Claims for the following Contracting States : AT, BE, CH, DE, DK, FR, GB, IT, LI, LU, NL, SE**

**1.** Compounds of the general formula

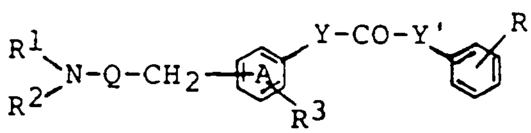

I

wherein $R^1$ and $R^2$ each signify hydrogen, lower alkyl or lower alkenyl or together signify straight-chain alkylene with 2 to 4 carbon atoms, $R^3$ signifies hydrogen, halogen or lower alkyl, Q signifies alkylene with 2 to 11 carbon atoms and at least 2 carbon atoms between the two free valencies or alkenylene with 4-11 carbon atoms and at least 4 carbon atoms between the two free valencies and Y and Y' each signify a direct bond or the group $-CH_2-$, $-CH_2CH_2-$,$-CH=CH-$ or $-C\equiv C-$, the group $R^1R^2N-Q-CH_2-$ is attached to the 3- or 4-position of the ring denoted by A and the symbol R signifies that the ring is unsubstituted or is substituted by halogen, trifluoromethyl, cyano, nitro, lower alkyl or lower alkoxy,

and their pharmaceutically acceptable acid addition salts for use as therapeutically active substances.

**2.** Compounds according to claim 1, wherein $R^1$ and $R^2$ each signify hydrogen or lower alkyl or together signify straight-chain alkylene with 2 to 4 carbon atoms, $R^3$ signifies hydrogen, Q signifies alkylene with 2 to 11 carbon atoms and at least 2 carbon atoms between the two free valencies and Y and Y' each signify a direct bond or the group $-CH_2-$, $-CH_2CH_2-$, $-CH=CH-$ or $-C\equiv C-$, the group $R^1R^2N-Q-CH_2-$ is attached to the 3- or 4-position of the ring denoted by A and the symbol R signifies that the ring is unsubstituted or is substituted by halogen, trifluoromethyl, nitro, lower alkyl or lower alkoxy, and their pharmaceutically acceptable acid addition salts for use as therapeutically active substances.

**3.** Compounds of the general formula

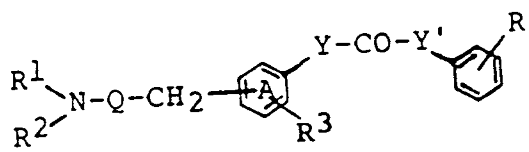

I

wherein $R^1$ and $R^2$ each signify hydrogen, lower alkyl or lower alkenyl or together signify straight-chain alkylene with 2 to 4 carbon atoms, $R^3$ signifies hydrogen, halogen or lower alkyl, Q signifies alkylene with 2 to 11 carbon atoms and at least 2 carbon atoms between the two free valencies or alkenylene with 4 to 11 carbon atoms and at least 4 carbon atoms between the two free valencies and Y and Y' each signify a direct bond or the group $-CH_2-$, $-CH_2CH_2-$, $-CH=CH-$ or $-C\equiv C-$, the group $R^1R^2N-Q-CH_2-$ is attached to the 3- or 4-position of the ring denoted by A and the symbol R signifies that the ring is unsubstituted or is substituted by halogen, trifluoromethyl, cyano, nitro, lower alkyl or lower alkoxy, with the proviso that Y and Y' do not simultaneously signify a direct bond when $R^1$ and $R^2$ each signify hydrogen or lower alkyl or together signify straight-chain alkylene with 2 to 4 carbon atoms, $R^3$ signifies hydrogen and Q signifies straight-chain alkylene with less than 4 carbon atoms,

and their pharmaceutically acceptable acid addition salts.

4. Compounds according to claim 3, wherein $R^1$ and $R^2$ each signify hydrogen or lower alkyl or together signify straight-chain alkylene with 2 to 4 carbon atoms, $R^3$ signifies hydrogen, Q signifies alkylene with 2 to 11 carbon atoms and at least 2 carbon atoms between the two free valencies and Y and Y' each signify a direct bond or the group $-CH_2-$, $-CH_2CH_2-$, -CH = CH- or -C≡C-, the group $R^1R^2N-Q-CH_2-$ is attached to the 3- or 4-position of the ring denoted by A and the symbol R signifies that the ring is unsubstituted or is substituted by halogen, trifluoromethyl, nitro, lower alkyl or lower alkoxy, with the proviso that Y and Y' do not simultaneously signify a direct bond when $R^3$ signifies hydrogen and Q signifies straight-chain alkylene with less than 4 carbon atoms.

5. Compounds according to claim 3, wherein Q signifies straight-chain alkylene with 4 to 7 carbon atoms.

6. Compounds according to claim 5, wherein $R^1$ and $R^2$ each signify $C_{1-4}$-alkyl or $C_{3-4}$-alkenyl or together signify $C_{3-4}$-alkylene.

7. Compounds according to claim 5 or 6, wherein $R^3$ signifies hydrogen.

8. Compounds according to any one of claims 5 to 7, wherein the group $R^1R^2N-Q-CH_2-$ is attached to the 4-position of the ring denoted by A.

9. Compounds according to any one of claims 5 to 8, wherein Y signifies a direct bond or the group $-CH_2-$.

10. Compounds according to any one of claims 5 to 9, wherein Y' signifies a direct bond or the group $-CH_2-$, $-CH_2CH_2-$ or -CH = CH-.

11. Compounds according to any one of claims 5 to 10, wherein the symbol R signifies that the ring is unsubstituted or is substituted by halogen, trifluoromethyl, nitro or lower alkyl.

12. 4-[7-(Dimethylamino)heptyl]benzophenone.

13. 4'-[7-(Dimethylamino)heptyl]-2-phenylacetophenone.

14. 4'-Fluoro-4-[7-(dimethylamino)heptyl]benzophenone.

15. 4-[7-(Dimethylamino)heptyl]-4'-(trifluorometehyl]benzophenone.

16. 2,4-Difluoro-4'-[7-(dimethylamino)heptyl]benzophenone.

17. Compounds according to claim 3, wherein Q signifies straight-chain alkylene with 2 or 3 carbon atoms.

18. Compounds according to claim 17, wherein $R^1$ and $R^2$ each signify $C_{1-4}$-alkyl or $C_{3-4}$-alkenyl or together signify $C_{3-4}$-alkylene.

19. Compounds according to claim 17 or 18, wherein $R^3$ signifies hydrogen.

20. Compounds according to any one of claims 17 to 19, wherein the group $R^1R^2N-Q-CH_2-$ is attached to the 4-position of the ring denoted by A.

21. Compounds according to any one of claims 17 to 20, wherein Y signifies a direct bond and Y' signifies the group $-CH_2-$, $-CH_2CH_2-$ or -CH = CH-.

22. Compounds according to any one of claims 17 to 21, wherein the symbol R signifies that the ring is unsubstituted or is substituted by halogen, trifluoromethyl, nitro or lower alkyl.

23. 3-(4-Chlorophenyl)-4'-[3-(dimethylamino)propyl]propiophenone.

24. 3-(2-Methylphenyl)-4'-[3-(dimethylamino)propyl]propiophenone.

**25.** (E)-3-Phenyl-4'-[3-(1-pyrrolidinyl)propyl]acrylophenone.

**26.** Compounds according to any one of claims 3 to 25 for use as therapeutically active substances.

**27.** Compounds according to any one of claims 1 to 25 for use as antifungally-active substances.

**28.** Compounds of the general formula

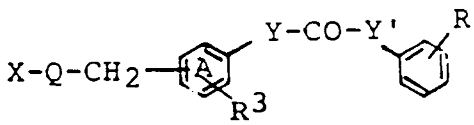

II

wherein X signifies a leaving group, $R^3$ signifies hydrogen, halogen or lower alkyl, Q signifies alkylene with 2 to 11 carbon atoms and at least 2 carbon atoms between the two free valencies or alkenylene with 4 to 11 carbon atoms and at least 4 carbon atoms between the two free valencies and Y and Y' each signify a direct bond or the group $-CH_2-$, $-CH_2CH_2-$, -CH=CH- or -C≡C-, the group X-Q-$CH_2$- is attached to the 3- or 4-position of the ring denoted by A and the symbol R signifies that the ring is unsubstituted or is substituted by halogen trifluoromethyl cyano, nitro, lower alkyl or lower alkoxy, with the proviso that Y and Y' do not simultaneously signify a direct bond when $R^3$ signifies hydrogen and Q signifies straight-chain alkylene with less than 4 carbon atoms.

**29.** Compounds of the general formula

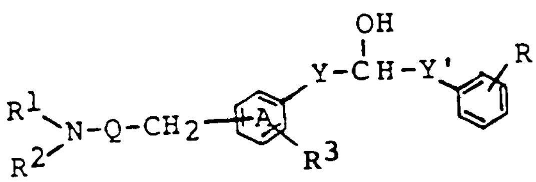

III

wherein $R^1$ and $R^2$ each signify hydrogen, lower alkyl or lower alkenyl or together signify straight--chain alkylene with 2 to 4 carbon atoms, $R^3$ signifies hydrogen, halogen or lower alkyl, Q signifies alkylene with 2 to 11 carbon atoms and at least 2 carbon atoms between the two free valencies or alkenylene with 4 to 11 carbon atoms and at least 4 carbon atoms between the two free valencies and Y and Y' each signify a direct bond or the group $-CH_2-$, $-CH_2CH_2-$, -CH=CH- or -C≡C-, the group $R^1R^2N$-Q-$CH_2$- is attached to the 3- or 4-position of the ring denoted by A and the symbol R signifies that the ring is unsubstituted or is substituted by halogen, trifluoromethyl cyano, nitro, lower alkyl or lower alkoxy, with the proviso that Y and Y' do not simultaneously signify a direct bond when $R^1$ and $R^2$ each signify hydrogen or lower alkyl or together signify straight-chain alkylene with 2 to 4 carbon atoms, $R^3$ signifies hydrogen and Q signifies straight-chain alkylene with less than 4 carbon atoms.

**30.** Compounds of the general formula

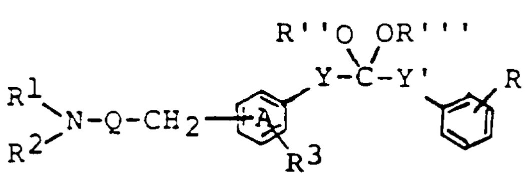

VI

wherein R'' and R''' each signify lower alkyl or together signify dimethylene or trimethylene, $R^1$ and $R^2$ each signify hydrogen, lower alkyl or lower alkenyl or together signify straight-chain alkylene with 2 to 4 carbon atoms, $R^3$ signifies hydrogen, halogen or lower alkyl, Q signifies alkylene with 2 to 11 carbon atoms and at least 2 carbon atoms between the two free valencies or alkenylene with 4 to 11 carbon atoms and at least 4 carbon atoms between the two free valencies and Y and Y' each signify a

direct bond or the group $-CH_2-$, $-CH_2CH_2-$, $-CH=CH-$ or $-C\equiv C-$, the group $R^1R^2N-Q-CH_2-$ is attached to the 3- or 4-position of the ring denoted by A and the symbol R signifies that the ring is unsubstituted or is substituted by halogen, trifluoromethyl, cyano, nitro, lower alkyl or lower alkoxy, with the proviso that Y and Y' do not simultaneously signify a direct bond when $R^1$ and $R^2$ each signify hydrogen or lower alkyl or together signify straight-chain alkylene with 2 to 4 carbon atoms, $R^3$ signifies hydrogen and Q signifies straight-chain alkylene with less than 4 carbon atoms.

**31.** A process for the manufacture of compounds according to any one of claims 3 to 25 and of pharmaceutically acceptable acid addition salts thereof, which process comprises

a) reacting a compound of the general formula

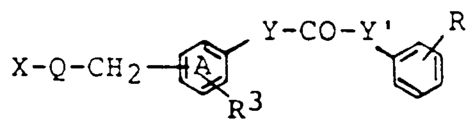

II

wherein X signifies a leaving group and A, $R^3$, Q, Y, Y' and R have the significance given in claim 3,

with an amine of the general formula $HNR^1R^2$, wherein $R^1$ and $R^2$ have the significance given in claim 3, or

b) oxidizing a compound of the general formula

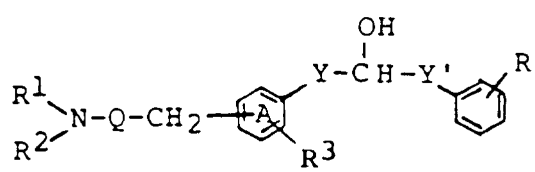

III

wherein A, $R^1$, $R^2$, $R^3$, Q, Y, Y' and R have the significance given in claim 3,

or

c) reacting a compound of the general formula

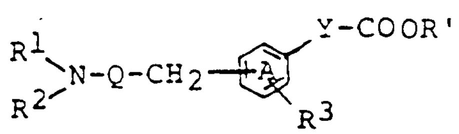

IV

wherein R' signifies lower alkyl and A, $R^1$, $R^2$, $R^3$, Q and Y have the significance given in claim 3,

with a compound of the general formula

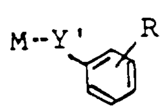

V

wherein M signifies -MgCl, -MgBr, -MgI or -Li and Y' and R have the significance given in claim 3,

or

d) treating a compound of the general formula

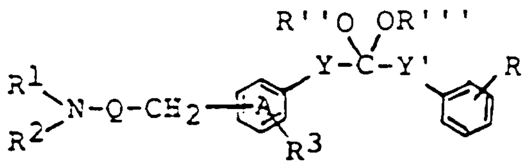

VI

wherein R'' and R''' each signify lower alkyl or together signify dimethylene or trimethylene and A, $R^1$, $R^2$, $R^3$, Q, Y, Y' and R have the significance given in claim 3,
with an aqueous acid, or
e) reacting a compound of the general formula

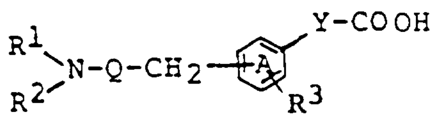

or

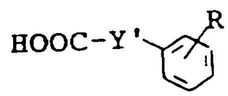

VIIa

VIIb

in the form of a reactive derivative in the presence of a Lewis acid with, respectively, a compound of the general formula

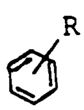

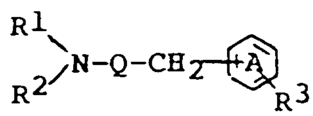

VIIIa

or

VIIIb

wherein A, $R^1$, $R^2$, $R^3$, Q, Y, Y' and R have the significance given in claim 3,
or
f) reacting a compound of the general formula

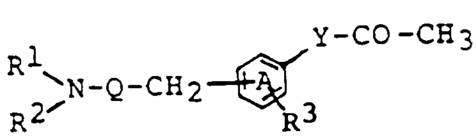

IX

wherein A, $R^1$, $R^2$, $R^3$, Q and Y have the significance given in claim 3,
in the presence of a base with a compound of the general formula

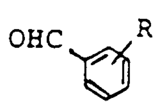

X

wherein R has the significance given in claim 3,
or

g) hydrogenating a compound of the general formula

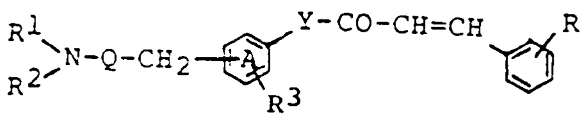

or

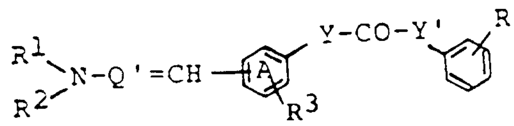

Ia

XI

wherein Q' signifies the group Q lessened by a hydrogen atom and A, $R^1$, $R^2$, $R^3$, Q, Y, Y' and R have the significance given in claim 3,
and
h) if desired, converting a compound of formula I obtained into a pharmaceutically acceptable acid addition salt.

**32.** A medicament containing a compound in accordance with any one of claims 1 to 25 and a therapeutically inert carrier.

**33.** An antifungally-active medicament, containing a compound in accordance with any one of claims 1 to 25 and a therapeutically inert carrier.

**34.** A medicament according to claim 32 or 33, which contains as an additional active substance a known antifungally-active substance which inhibits sterol biosynthesis.

**35.** The use of compounds according to any one of claims 1 to 25, optionally in combination with known antifungally-active substances which inhibit sterol biosynthesis, for the manufacture of antifungally-active medicaments.

**Claims for the following Contracting State : ES**

**1.** A process for the manufacture of compounds of the general formula

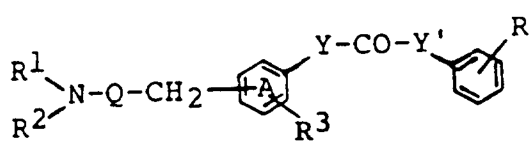

I

wherein $R^1$ and $R^2$ each signify hydrogen, lower alkyl or lower alkenyl or together signify straight-chain alkylene with 2 to 4 carbon atoms, $R^3$ signifies hydrogen, halogen or lower alkyl, Q signifies alkylene with 2 to 11 carbon atoms and at least 2 carbon atoms between the two free valencies or alkenylene with 4 to 11 carbon atoms and at least 4 carbon atoms between the two free valencies and Y and Y' each signify a direct bond or the group $-CH_2-$, $-CH_2CH_2-$, $-CH=CH-$ or $-C\equiv C-$, the group $R^1R^2N-Q-CH_2-$ is attached to the 3- or 4-position of the ring denoted by A and the symbol R signifies that the ring is unsubstituted or is substituted by halogen, trifluoromethyl, cyano, nitro, lower alkyl or lower alkoxy, with the proviso that Y and Y' do not simultaneously signify a direct bond when $R^1$ and $R^2$ each signify hydrogen or lower alkyl or together signify straight-chain alkylene with 2 to 4 carbon atoms, $R^3$ signifies hydrogen and Q signifies straight-chain alkylene with less than 4 carbon atoms,
and their pharmaceutically acceptable acid addition salts, characterized by

a) reacting a compound of the general formula

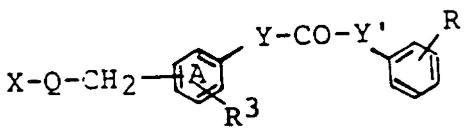

II

wherein X signifies a leaving group and A, $R^3$, Q, Y, Y' and R have the above significance, with an amine of the general formula $HNR^1R^2$, wherein $R^1$ and $R^2$ have the above significance, or

b) oxidizing a compound of the general formula

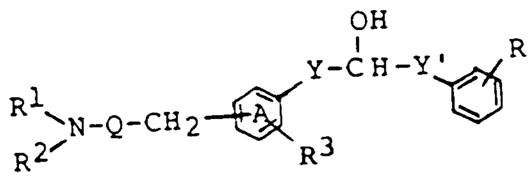

III

wherein A, $R^1$, $R^2$, $R^3$, Q, Y, Y' and R have the above significance,

or

c) reacting a compound of the general formula

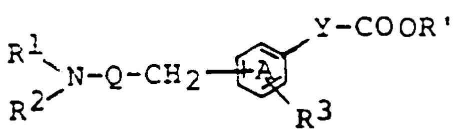

IV

wherein R' signifies lower alkyl and A, $R^1$, $R^2$, $R^3$, Q and Y have the above significance, with a compound of the general formula

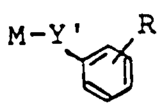

V

wherein M signifies -MgCl, -MgBr, -MgI or -Li and Y' and R have the above significance,

or

d) treating a compound of the general formula

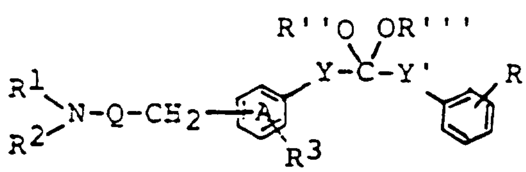

VI

wherein R'' and R''' each signify lower alkyl or together signify dimethylene or trimethylene and A, $R^1$, $R^2$, $R^3$, Q, Y, Y' and R have the above significance,

with an aqueous acid, or

e) reacting a compound of the general formula

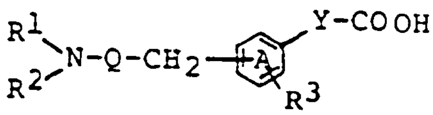 or 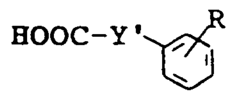

VIIa VIIb

in the form of a reactive derivative in the presence of a Lewis acid with, respectively, a compound of the general formula

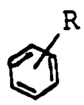

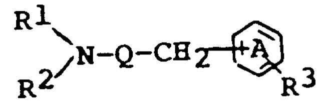

VIIIa or VIIIb

wherein A, $R^1$, $R^2$, $R^3$ Q, Y, Y' and R have the above significance.

or

f) reacting a compound of the general formula

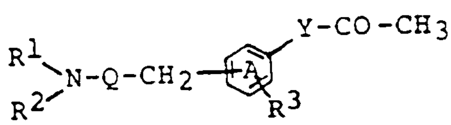

IX

wherein A, $R^1$, $R^2$, $R^3$, Q and Y have the above significance, in the presence of a base with a compound of the general formula

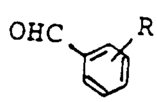

X

wherein R has the above significance, or

g) hydrogenating a compound of the general formula

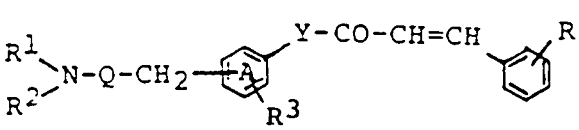 or 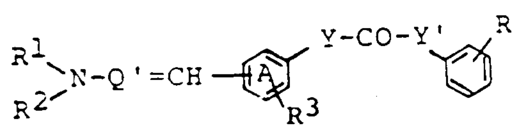

wherein Q' signifies the group Q lessened by a hydrogen atom and A, $R^1$, $R^2$, $R^3$, Q, Y, Y' and R have the above significance,

and

h) if desired, converting a compound of formula I obtained into a pharmaceutically acceptable acid addition salt.

**2.** A process according to claim 1, wherein $R^1$ and $R^2$ each signify hydrogen or lower alkyl or together signify straight-chain alkylene with 2 to 4 carbon atoms, $R^3$ signifies hydrogen, Q signifies alkylene with 2 to 11 carbon atoms and at least 2 carbon atoms between the two free valencies and Y and Y' each signify a direct bond or the group $-CH_2-$, $-CH_2CH_2-$, -CH=CH- or -C≡C-, the group $R^1R^2N-Q-CH_2-$ is attached to the 3- or 4-position of the ring denoted by A and the symbol R signifies that the ring is unsubstituted or is substituted by halogen, trifluoromethyl, nitro lower alkyl or lower alkoxy, with the proviso that Y and Y' do not simultaneously signify a direct bond when $R^3$ signifies hydrogen and Q signifies straight-chain alkylene with less than 4 carbon atoms.

**3.** A process according to claim 1, wherein Q signifies straight-chain alkylene with 4 to 7 carbon atoms.

**4.** A process according to claim 3, wherein $R^1$ and $R^2$ each signify $C_{1-4}$-alkyl or $C_{3-4}$-alkenyl or together signify $C_{3-4}$-alkylene.

**5.** A process according to claim 3 or 4, wherein $R^3$ signifies hydrogen.

**6.** A process according to any one of claims 3 to 5, wherein the group $R^1R^2N-Q-CH_2-$ is attached to the 4-position of the ring denoted by A.

**7.** A process according to any one of claims 3 to 6, wherein Y signifies a direct bond or the group $-CH_2-$.

**8.** A process according to any one of claims 3 to 7, wherein Y' signifies a direct bond or the group $-CH_2-$, $-CH_2CH_2-$ or -CH=CH-.

**9.** A process according to any one of claims 3 to 8, wherein the symbol R signifies that the ring is unsubstituted or is substituted by halogen trifluoromethyl, nitro or lower alkyl.

**10.** A process according to claim 1, characterized in that 4-[7-(dimethylamino)heptyl]benzophenone is manufactured.

**11.** A process according to claim 1, characterized in that 4'-[7-(dimethylamino)heptyl]-2-phenylacetophenone is manufactured.

**12.** A process according to claim 1, characterized in that 4'-fluoro-4-[7-(dimethylamino)heptyl]-benzophenone is manufactured.

**13.** A process according to claim 1, characterized in that 4-[7-(dimethylamino)heptyl]-4'-(trifluorometehyl]-benzophenone is manufactured.

**14.** A process according to claim 1, characterized in that 2,4-difluoro-4'-[7-(dimethylamino)heptyl]-benzophenone is manufactured.

**15.** A process according to claim 1, wherein Q signifies straight-chain alkylene with 2 or 3 carbon atoms.

**16.** A process according according to claim 15, wherein $R^1$ and $R^2$ each signify $C_{1-4}$-alkyl or $C_{3-4}$-alkenyl or together signify $C_{3-4}$-alkylene.

**17.** A process according to claim 15 or 16, wherein $R^3$ signifies hydrogen.

**18.** A process according to any one of claims 15 to 17, wherein the group $R^1R^2N-Q-CH_2-$ is attached to the 4-position of the ring denoted by A.

**19.** A process according to any one of claims 15 to 18, wherein Y signifies a direct bond and Y' signifies the group $-CH_2-$, $-CH_2CH_2-$ or -CH=CH-.

**20.** A process according to any one of claims 15 to 19, wherein the symbol R signifies that the ring is unsubstituted or is substituted by halogen, trifluoromethyl, nitro or lower alkyl.

**21.** A process according to claim 1, characterized in that 3-(4-chlorophenyl)-4'-[3-(dimethylamino)propyl]-propiophenone is manufactured.

**22.** A process according to claim 1, characterized in that 3-(2-methylphenyl)-4'-[3-(dimethylamino)propyl]-propiophenone is manufactured.

**23.** A process according to claim 1, characterized in that (E)-3-phenyl-4'-[3-(1-pyrrolidinyl)propyl]-acrylophenone is manufactured.

**24.** A process for the manufacture of medicaments, especially of antifungally-active medicaments, characterized by bringing a compound of fhe general formula

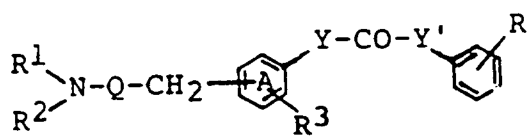 I

wherein $R^1$ and $R^2$ each signify hydrogen, lower alkyl or lower alkenyl or together signify straight-chain alkylene with 2 to 4 carbon atoms, $R^3$ signifies hydrogen, halogen or lower alkyl, Q signifies alkylene with 2 to 11 carbon atoms and at least 2 carbon atoms between the two free valencies or alkenylene with 4-11 carbon atoms and at least 4 carbon atoms between the two free valencies and Y and Y' each signify a direct bond or the group $-CH_2-$, $-CH_2CH_2-$,-CH=CH- or -C≡C-, the group $R^1R^2N-Q-CH_2-$ is attached to the 3- or 4-position of the ring denoted by A and the symbol R signifies that the ring is unsubstituted or is substituted by halogen, trifluoromethyl, cyano, nitro, lower alkyl or lower alkoxy,
or a pharmaceutically acceptable acid addition salt thereof into a galenical administration form together with a therapeutically inert carrier.

**25.** A process for the manufacture of antifungally-active medicaments, characterized by bringing a compound of formula I defined in claim 24 or a pharmaceutically acceptable acid addition salt thereof into a galenical administration form together with a known antifungally-active substance which inhibits sterol biosynthesis and a therapeutically inert carrier.

**26.** The use of compounds of formula I defined in claim 24 and their pharmaceutically acceptable acid addition salts, optionally in combination with known antifungally-active substances which inhibit sterol biosynthesis, for the manufacture of antifungally-active medicaments.

**Claims for the following Contracting State : GR**

**1.** A process for the manufacture of compounds of the general formula

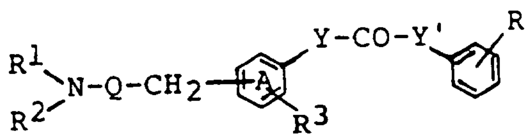 I

wherein $R^1$ and $R^2$ each signify hydrogen, lower alkyl or lower alkenyl or together signify straight-chain alkylene with 2 to 4 carbon atoms, $R^3$ signifies hydrogen, halogen or lower alkyl, Q signifies alkylene with 2 to 11 carbon atoms and at least 2 carbon atoms between the two free valencies or alkenylene with 4 to 11 carbon atoms and at least 4 carbon atoms between the two free valencies and Y and Y' each signify a direct bond or the group $-CH_2-$, $-CH_2CH_2-$, -CH=CH- or -C≡C-, the group

$R^1R^2N$-Q-$CH_2$- is attached to the 3- or 4-position of the ring denoted by A and the symbol R signifies that the ring is unsubstituted or is substituted by halogen trifluoromethyl, cyano, nitro, lower alkyl or lower alkoxy, with the proviso that Y and Y' do not simultaneously signify a direct bond when $R^1$ and $R^2$ each signify hydrogen or lower alkyl or together signify straight-chain alkylene with 2 to 4 carbon atoms, $R^3$ signifies hydrogen and Q signifies straight-chain alkylene with less than 4 carbon atoms, and their pharmaceutically acceptable acid addition salts, characterized by

a) reacting a compound of the general formula

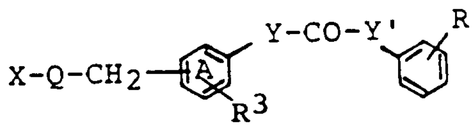

II

wherein X signifies a leaving group and A, $R^3$, Q, Y, Y' and R have the above significance, with an amine of the general formula $HNR^1R^2$, wherein $R^1$ and $R^2$ have the above significance, or

b) oxidizing a compound of the general formula

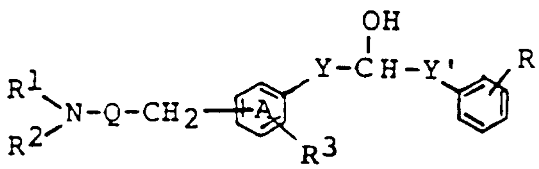

III

wherein A, $R^1$, $R^2$, $R^3$, Q, Y, Y' and R have the above significance, or

c) reacting a compound of the general formula

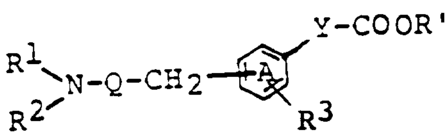

IV

wherein R' signifies lower alkyl and A, $R^1$, $R^2$, $R^3$, Q and Y have the above significance, with a compound of the general formula

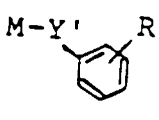

V

wherein M signifies -MgCl, -MgBr, -MgI or -Li and Y' and R have the above significance, or

d) treating a compound of the general formula

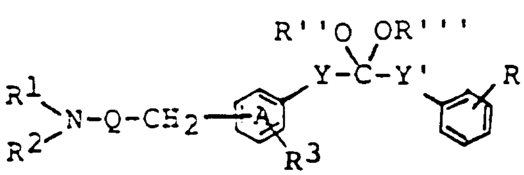

VI

wherein R'' and R''' each signify lower alkyl or together signify dimethylene or trimethylene and A, $R^1$, $R^2$, $R^3$, Q, Y, Y' and R have the above significance,

with an aqueous acid, or

e) reacting a compound of the general formula

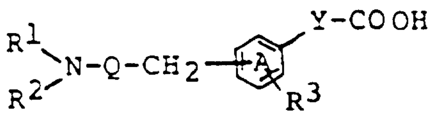 or 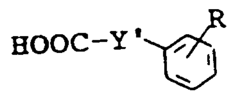

VIIa VIIb

in the form of a reactive derivative in the presence of a Lewis acid with, respectively, a compound of the general formula

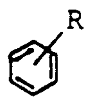 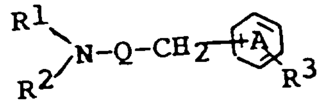

VIIIa or VIIIb

wherein A, $R^1$, $R^2$, $R^3$, Q, Y, Y' and R have the above significance,

or

f) reacting a compound of the general formula

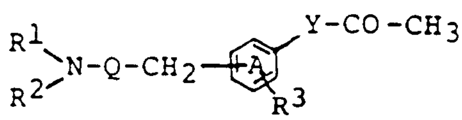

IX

wherein A, $R^1$, $R^2$, $R^3$, Q and Y have the above significance, in the presence of a base with a compound of the general formula

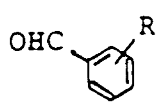

X

wherein R has the above significance,

or

g) hydrogenating a compound of the general formula

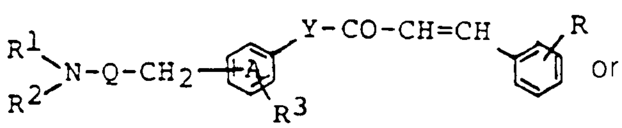 or 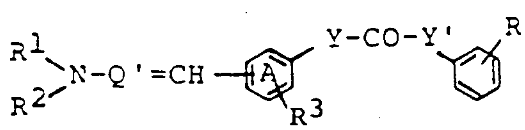

wherein Q' signifies the group Q lessened by a hydrogen atom and A, $R^1$, $R^2$, $R^3$, Q, Y, Y' and R have the above significance,

and

h) if desired, converting a compound of formula I obtained into a pharmaceutically acceptable acid addition salt.

**2.** A process according to claim 1, wherein $R^1$ and $R^2$ each signify hydrogen or lower alkyl or together signify straight-chain alkylene with 2 to 4 carbon atoms, $R^3$ signifies hydrogen, Q signifies alkylene with 2 to 11 carbon atoms and at least 2 carbon atoms between the two free valencies and Y and Y' each signify a direct bond or the group $-CH_2-$, $-CH_2CH_2-$, $-CH=CH-$ or $-C{\equiv}C-$, the group $R^1R^2N$-Q-$CH_2$- is attached to the 3- or 4-position of the ring denoted by A and the symbo R signifies that the ring is unsubstituted or is substituted by halogen, trifluoromethyl, nitro, lower alkyl or lower alkoxy, with the proviso that Y and Y' do not simultaneously signify a direct bond when $R^3$ signifies hydrogen and Q signifies straight-chain alkylene with less than 4 carbon atoms.

**3.** A process according to claim 1, wherein Q signifies straight-chain alkylene with 4 to 7 carbon atoms.

**4.** A process according to claim 3, wherein $R^1$ and $R^2$ each signify $C_{1-4}$-alkyl or $C_{3-4}$-alkenyl or together signify $C_{3-4}$-alkylene.

**5.** A process according to claim 3 or 4, wherein $R^3$ signifies hydrogen.

**6.** A process according to any one of claims 3 to 5, wherein the group $R^1R^2N$-Q-$CH_2$- is attached to the 4-position of the ring denoted by A.

**7.** A process according to any one of claims 3 to 6, wherein Y signifies a direct bond or the group $-CH_2-$,

**8.** A process according to any one of claims 3 to 7, wherein Y' signifies a direct bond or the group $-CH_2-$, $-CH_2CH_2-$ or $-CH=CH-$.

**9.** A process according to any one of claims 3 to 8, wherein the symbol R signifies that the ring is unsubstituted or is substituted by halogen, trifluoromethyl, nitro or lower alkyl.

**10.** A process according to claim 1, characterized in that 4-[7-(dimethylamino)heptyl]benzophenone is manufactured.

**11.** A process according to claim 1, characterized in that 4'-[7-(dimethylamino)heptyl]-2-phenylacetophenone is manufactured.

**12.** A process according to claim 1, characterized in that 4'-fluoro-4-[7-(dimethylamino)heptyl]-benzophenone is manufactured.

**13.** A process according to claim 1, characterized in that 4-[7-(dimethylamino)heptyl]-4'-(trifluorometehyl]-benzophenone is manufactured.

**14.** A process according to claim 1, characterized in that 2,4-difluoro-4'-[7-(dimethylamino)heptyl]-benzophenone is manufactured.

**15.** A process according to claim 1, wherein Q signifies straight-chain alkylene with 2 or 3 carbon atoms.

**16.** A process according according to claim 15, wherein $R^1$ and $R^2$ each signify $C_{1-4}$-alkyl or $C_{3-4}$-alkenyl or together signify $C_{3-4}$-alkylene.

**17.** A process according to claim 15 or 16, wherein $R^3$ signifies hydrogen.

**18.** A process according to any one of claims 15 to 17, wherein the group $R^1R^2N$-Q-$CH_2$- is attached to the 4-position of the ring denoted by A.

**19.** A process according to any one of claims 15 to 18, wherein Y signifies a direct bond and Y' signifies the group $-CH_2-$, $-CH_2CH_2-$ or $-CH=CH-$.

**20.** A process according to any one of claims 15 to 19, wherein the symbol R signifies that the ring is unsubstituted or is substituted by halogen, trifluoromethyl, nitro or lower alkyl.

**21.** A process according to claim 1, characterized in that 3-(4-chlorophenyl)-4'-[3-(dimethylamino)propyl]-propiophenone is manufactured.

**22.** A process according to claim 1, characterized in that 3-(2-methylphenyl)-4'-[3-(dimethylamino)propyl]-propiophenone is manufactured.

**23.** A process according to claim 1, characterized in that (E)-3-phenyl-4'-[3-(1-pyrrolidinyl)propyl]-acrylophenone is manufactured.

**24.** A process for the manufacture of medicaments, especially of antifungally-active medicaments, characterized by bringing a compound of fhe general formula

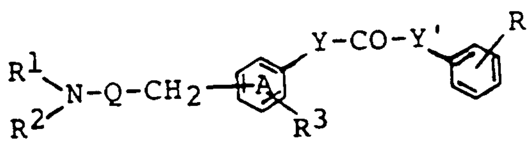

I

wherein $R^1$ and $R^2$ each signify hydrogen, lower alkyl or lower alkenyl or together signify straight--chain alkylene with 2 to 4 carbon atoms, $R^3$ signifies hydrogen, halogen or lower alkyl, Q signifies alkylene with 2 to 11 carbon atoms and at least 2 carbon atoms between the two free valencies or alkenylene with 4-11 carbon atoms and at least 4 carbon atoms between the two free valencies and Y and Y' each signify a direct bond or the group $-CH_2-$, $-CH_2CH_2-$,$-CH=CH-$ or $-C\equiv C-$, the group $R^1R^2N-Q-CH_2-$ is attached to the 3- or 4-position of the ring denoted by A and the symbol R signifies that the ring is unsubstituted or is substituted by halogen, trifluoromethyl, cyano, nitro, lower alkyl or lower alkoxy,
or a pharmaceutically acceptable acid addition salt thereof into a galenical administration form together with a therapeutically inert carrier.

**25.** A process for the manufacture of antifungally-active medicaments, characterized by bringing a compound of formula I defined in claim 24 or a pharmaceutically acceptable acid addition salt thereof into a galenical administration form together with a known antifungally-active substance which inhibits sterol biosynthesis and a therapeutically inert carrier.

**26.** The use of compounds of formula I defined in claim 24 and their pharmaceutically acceptable acid addition salts, optionally in combination with known antifungally-active substances which inhibit sterol biosynthesis, for the manufacture of antifungally-active medicaments.

**27.** Compounds of the general formula

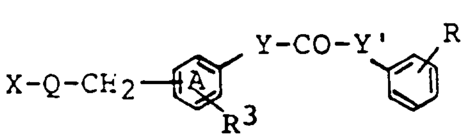

II

wherein X signifies a leaving group, $R^3$ signifies hydrogen, halogen or lower alkyl, Q signifies alkylene with 2 to 11 carbon atoms and at least 2 carbon atoms between the two free valencies or alkenylene with 4 to 11 carbon atoms and at least 4 carbon atoms between the two free valencies and Y and Y' each signify a direct bond or the group $-CH_2-$, $-CH_2CH_2-$, $-CH=CH-$ or $-C\equiv C-$, the group $X-Q-CH_2-$ is attached to the 3- or 4-position of the ring denoted by A and the symbol R signifies that the ring is unsubstituted or is substituted by halogen, trifluoromethyl, cyano, nitro, lower alkyl or lower alkoxy, with the proviso that Y and Y' do not simultaneously signify a direct bond when $R^3$ signifies hydrogen and Q signifies straight-chain alkylene with less than 4 carbon atoms.

**28.** Compounds of the general formula

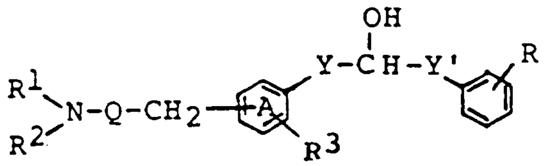

III

wherein $R^1$ and $R^2$ each signify hydrogen, lower alkyl or lower alkenyl or together signify straight--chain alkylene with 2 to 4 carbon atoms, $R^3$ signifies hydrogen, halogen or lower alkyl, Q signifies alkylene with 2 to 11 carbon atoms and at least 2 carbon atoms between the two free valencies or alkenylene with 4 to 11 carbon atoms and at least 4 carbon atoms between the two free valencies and Y and Y' each signify a direct bond or the group $-CH_2-$, $-CH_2CH_2-$, -CH=CH- or -C≡C-, the group $R^1R^2N-Q-CH_2-$ is attached to the 3- or 4-position of the ring denoted by A and the symbol R signifies that the ring is unsubstituted or is substituted by halogen, trifluoromethyl, cyano, nitro, lower alkyl or lower alkoxy with the proviso that Y and Y' do not simultaneously signify a direct bond when $R^1$ and $R^2$ each signify hydrogen or lower alkyl or together signify straight-chain alkylene with 2 to 4 carbon atoms, $R^3$ signifies hydrogen and Q signifies straight-chain alkylene with less than 4 carbon atoms.

**29.** Compounds of the general formula

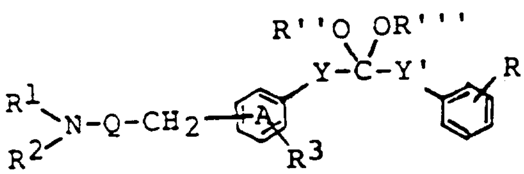

VI

wherein R'' and R''' each signify lower alkyl or together signify dimethylene or trimethylene, $R^1$ and $R^2$ each signify hydrogen, lower alkyl or lower alkenyl or together signify straight-chain alkylene with 2 to 4 carbon atoms, $R^3$ signifies hydrogen, halogen or lower alkyl, Q signifies alkylene with 2 to 11 carbon atoms and at least 2 carbon atoms between the two free valencies or alkenylene with 4 to 11 carbon atoms and at least 4 carbon atoms between the two free valencies and Y and Y' each signify a direct bond or the group $-CH_2-$, $-CH_2CH_2-$, -CH=CH- or -C≡C-, the group $R^1R^2N-Q-CH_2-$ is attached to the 3- or 4-position of the ring denoted by A and the symbol R signifies that the ring is unsubstituted or is substituted by halogen, trifluoromethyl, cyano, nitro, lower alkyl or lower alkoxy, with the proviso that Y and Y' do not simultaneously signify a direct bond when $R^1$ and $R^2$ each signify hydrogen or lower alkyl or together signify straight-chain alkylene with 2 to 4 carbon atoms, $R^3$ signifies hydrogen and Q signifies straight-chain alkylene with less than 4 carbon atoms.

**Revendications**

**Revendications pour les Etats contractants suivants : AT, BE, CH, DE, DK, FR, GB, IT, LI, LU, NL, SE**

**1.** Composés de la formule générale

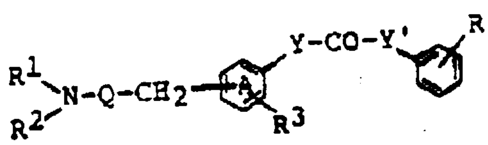

I

dans laquelle $R^1$ et $R^2$ signifient respectivement de l'hydrogène, de l'alkyle bas ou de l'alcényle bas ou ensemble de l'alkylène en chaîne linéaire avec 2 à 4 atomes de carbone, $R^3$ de l'hydrogène, de l'halogène ou de l'alkyle bas, Q de l'alkylène avec 2 à 11 atomes de carbone entre les deux valences libres ou de l'alcénylène avec 4 à 11 atomes de carbone et au moins 4 atomes de carbones entre les deux valences libres et Y et Y' respectivement une liaison directe ou le groupe $-CH_2-$, $-CH_2CH_2-$,

-CH = CH- ou -C≡C-, le groupe $R^1R^2N$-Q-$CH_2$- est lié en 3ème ou 4ème position du composé cyclique désigné par A, et le symbole R signifie que le composé cyclique n'est pas substitué ou est substitué par de l'halogène, du trifluorométhlyle, du cyano, du nitro, de l'alkyle bas ou de l'alkoxy bas, et leurs sels d'addition d'acide acceptables pour une utilisation en tant qu'agents thérapeutiques.

**2.** Composés selon la revendication 1, dans laquelle $R^1$ et $R^2$ signifient respectivement de l'hydrogène ou de l'alkyle bas ou ensemble de l'alkylène en chaîne linéaire avec 2 à 4 atomes de carbone, $R^3$ de l'bydrogène et Q de l'alkylène avec 2 à 11 atomes de carbone et au moins 2 atomes de carbone entre les deux valences libres et Y et Y' respectivement une liaison directe ou le groupe -$CH_2$-, -$CH_2CH_2$-, -CH = CH- ou -C≡C-, le groupe $R^1R^2N$-Q-$CH_2$ est lié en 3ème ou 4ème position du composé cyclique désigné par A, et le symbole R signifie que le composé cyclique n'est pas substitué ou est substitué par de l'halogène, du trifluorométhyle, du nitro, de l'alkyle bas ou de l'alkoxy bas, et leurs sels d'addition d'acide acceptables du point de vue pharmaceutique pour une utilisation en tant qu'agents thérapeutiques.

**3.** Composés de la formule générale

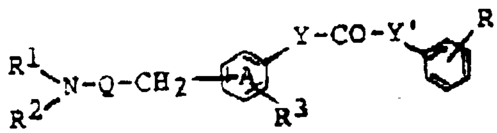

I

dans laquelle $R^1$ et $R^2$ siginifient respectivement de l'hydrogène, de l'alkyle bas ou de l'alcényle bas ou ensemble de l'alkylène en chaîne linéaire avec 2 à 4 atomes de carbone, $R^3$ de l'bydrogène, de l'halogène ou de l'alkyle bas, Q de l'alkylène avec 2 à 11 atomes de carbones et au moins 2 atomes de carbone entre les deux valences libres ou de l'alcénylène avec 4 à 11 atomes de carbone et au moins 4 atomes de carbone entre les deux valences libres et Y et Y', respectivement une liaison directe ou le groupe -$CH_2$-, -$CH_2CH_2$-, -CH = CH- ou -C≡C-, le groupe $R^1R^2N$-Q-$CH_2$ est lié en 3ème ou 4ème position du composé cyclique désigné par A, et le symbole R signifie que le composé cyclique n'est pas substitué ou est substitué par de l'halogène, du trifluorométhyle, du cyano, du nitro, de l'alkyl bas ou de l'alkoxy bas, Y et Y' ne signifiant pas en même temps une liaison directe, lorsque $R^1$ et $R^2$ signifient respectivement de l'hydrogène ou de l'alkyle bas ou ensemble de l'alkylène en chaîne linéaire avec 2 à 4 atomes de carbone, $R^3$ de l'hydrogène et Q de l'alkylène en chaîne linéaire avec moins de 4 atomes de carbone et leurs sels d'addition d'acide acceptables du point de vue pharmaceutique.

**4.** Composés selon la revendication 3, dans lesquels $R^1$ et $R^2$ signifient respectivement de l'hydrogène ou de l'alkyle bas ou ensemble de l'alkylène en chaîne linéaire avec 2 à 4 atomes de carbone, $R^3$ de l'hydrogène, Q de l'alkylène avec 2 à 11 atomes de carbone et au moins 2 atomes de carbone entre les deux valences libres et Y et Y' respectivement une liaison directe ou le groupe -$CH_2$-, -$CH_2CH_2$-, -CH = CH ou -C≡C, le groupe $R^1R^2N$-Q-$CH_2$ est lié en 3ème ou 4ème position du composé cyclique désigné par A, et le symbole R signifie que le composé cyclique n'est pas substitué ou est substitué par de l'halogène, du trifluorométhyl, du nitro, de l'alkyle bas ou de l'alkoxy bas, Y et Y' ne signifiant pas en même temps une liaison directe, lorsque $R^3$ signifie de l'hydrogène et Q de l'alkylène en chaîne linéaire avec moins de 4 atomes de carbone.

**5.** Composés selon la revendication 3, dans lesquels Q signifie de l'alkylène en chaîne linéaire avec 4 à 7 atomes de carbone.

**6.** Composés selon la revendication 5, dans lesquels $R^1$ et $R^2$ signifient respectivement de l'alkyle $C_{1-4}$ ou de l'alcényle $C_{3-4}$ ou ensemble de l'alkylène $C_{3-4}$.

**7.** Composés selon la revendication 5 ou 6, dans lesquels $R^3$ signifie de l'hydrogène.

**8.** Composés selon l'une quelconque des revendications 5 à 7, dans lesquels le groupe $R^1R^2N$-Q-$CH_2$ est lié en 4ème position du composé cyclique désigné par A.

**9.** Composés selon l'une quelconque des revendications 5 à 8, dans lesquels Y signifie une liaison directe ou le groupe $-CH_2-$.

**10.** Composés selon l'une quelconque des revendications 5 à 9, dans lesquels Y' signifie une liaison directe ou le groupe $-CH_2-$, $-CH_2CH_2-$ ou $-CH=CH-$.

**11.** Composés selon l'une des revendications 5 à 10, dans lesquels le symbole R signifie que le composé cyclique n'est pas substitué ou est substitué par de l'halogène, du trifluorométhyl, du nitro ou de l'alkyle bas.

**12.** 4-[7-(diméthylamino)heptyl]benzophénone.

**13.** 4'-[7-(diméthylamino) heptyl]-2-phénylacétophénone.

**14.** 4'-fluoro-4-[7-(diméthylamino)heptyl]benzophénone.

**15.** 4-[7-(diméthylamino) heptyl]-4'-(trifluorométhyl]-benzophénone.

**16.** 2,4-difluoro-4'-[7-(diméthylamino)heptyl]benzophénone.

**17.** Composés selon la revendication 3, dans lesquels Q signifie de l'alkylène en chaîne linéaire avec 2 ou 3 atomes de carbone.

**18.** Composés selon la revendication 17, dans lesquels $R^1$ et $R^2$ signifient respectivement de l'alkyle $C_{1-4}$ ou de l'alcényle $C_{3-4}$ ou ensemble de l'alkylène $C_{3-4}$.

**19.** Composés selon la revendication 17 ou 18, dans lesquels $R^3$ signifie de l'hydrogène.

**20.** Composés selon l'une des revendications 17 à 19 dans lesquels le groupe $R^1R^2N-Q-CH_2$ est lié en 4ème position du composé cyclique désigné par A.

**21.** Composés selon l'une des revendications 17 à 20, dans lesquels Y signifie une liaison directe et Y' le groupe $-CH_2-$, $-CH_2CH_2-$ ou $-CH=CH-$.

**22.** Composés selon l'une des revendications 17 à 21, dans lesquels le symbole R signifie que le composé cyclique n'est pas substitué ou est substitué par de l'halogène, du trifluorométhyle, du nitro ou de l'alkyle bas.

**23.** 3-(4-chlorophényl)-4'-[3-(diméthylamino) propyl]-propiophénone.

**24.** 3-(2-méthylphényl)-4'-[3-(diméthylamino) propyl]-propiophénone.

**25.** (E)-3-phényl-4'-[3-(1-pyrrolidinyl) propyl]acrylophénone.

**26.** Composés selon l'une des revendications 3 à 25 pour utilisation comme agents thérapeutiques.

**27.** Composés selon l'une des revendications 1 à 25 pour utilisation comme substances antifongiques actives.

**28.** Composés de la formule générale

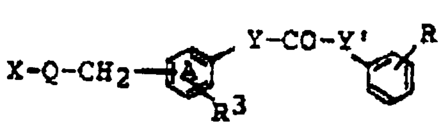

II

dans laquelle X signifie un groupe de départ, $R^3$ de l'hydrogène, de l'halogène ou de l'alkyle bas, Q de

l'alkylène avec 2 à 11 atomes de carbone et au moins 2 atomes de carbone encre les deux valences libres ou de l'alcénylène avec 4 à 11 atomes de carbone et au moins 4 atomes de carbone entre les deux valences libres et Y et Y' respectivement une liaison directe ou le groupe $-CH_2-$, $-CH_2CH_2-$, -CH = CH- ou -C≡C, le groupe $X-Q-CH_2$ est lié en 3ème ou 4ème position du composé cylique désigné par A, et le symbole R signifie que le composé cyclique n'est pas substitué ou est substitué par de l'halogène, du trifluorométhyle, du cyano, du nitro, de l'alkyle bas ou de l'alcoxy bas, Y et Y' ne signifiant pas simultanément une liaison directe, quand $R^3$ signifie de l'hydrogène et Q de l'alkylène en chaîne linéaire avec moins de 4 atomes de carbone.

**29.** Composés de la formule générale

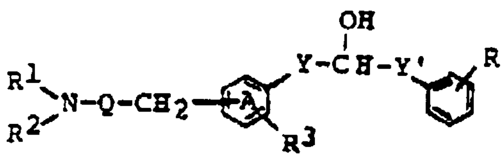

III

dans lesquels $R^1$ et $R^2$ signifient respectivement de l'hydrogène, de l'alkyle bas ou de l'alcényle bas ou ensemble de l'alkylène en chaîne linéaire avec 2 à 4 atomes de carbone, $R^3$ de l'hydrogène, de l'halogène ou de l'alkyle bas, Q de l'alkylène avec 2 à 11 atomes de carbone et au moins 2 atomes de carbone entre deux valences libres ou de l'alcénylène avec 4 à 11 atomes de carbone et au moins 4 atomes de carbone entre les deux valences libres et Y et Y' respectivement une liaison directe ou le groupe $-CH_2-$, $-CH_2CH_2-$, -CH = CH- ou -C≡C, le groupe $R^1R^2N-Q-CH_2-$ est lié en 3ème ou 4ème position du composé cyclique désigné par A, et le symbole R signifie que le composé cyclique n'est pas substitué ou est substitué par de l'halogène, du trifluorométhyle, du cyano, du nitro, de l'alkyl bas ou de l'alkoxy bas, Y et Y' ne signifiant pas simultanément une liaison directe, lorsque $R^1$ et $R^2$ signifient respectivement de l'hydrogène ou de l'alkyle bas ou ensemble de l'alkylène en chaîne linéaire avec 2 à 4 atomes de carbone, $R^3$ de l'hydrogène et Q de l'alkylène en chaîne linéaire avec moins de 4 atomes de carbone.

**30.** Composés selon la formule générale

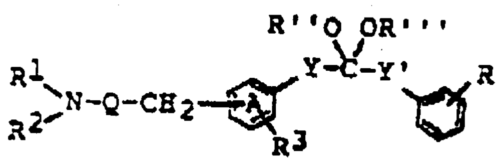

VI

dans laquelle R'' et R''' signifient respectivement de l'alkyle bas ou ensemble du diméthylène ou du triméthylène, $R^1$ et $R^2$ respectivement de l'hydrogène, de l'alkyle bas ou de l'alcényle bas ou ensemble de l'alkylène en chaîne linéaire avec 2 à 4 atomes de carbone, $R^3$ de l'hydrogène, de l'halogène ou de l'alkyle bas, Q de l'alkylène avec 2 à 11 atomes de carbone et au moins 2 atomes de carbone entre les deux valences libres ou de l'alcénylène avec 4 à 11 atomes de carbone et au moins 4 atomes de carbone entre les deux valences libres et Y et Y' respectivement une liaison directe ou le groupe $-CH_2-$, $-CH_2CH_2-$, -CH = CH- ou -C≡C-, le groupe $R^1R^2N-Q-CH_2-$ est lié en 3ème ou 4ème position du composé cyclique désigné par A, et le symbole R signifie que le composé cyclique n'est pas substitué ou est substitué par de l'halogène, du trifluorométhyle, du cyano, du nitro, de l'alkyle bas ou de l'alkoxy bas, Y et Y' ne signifiant pas simultanément une liaison directe, lorsque $R^1$ et $R^2$ signifient respectivement de l'hydrogène ou de l'alkyle bas ou ensemble de l'alkylène en chaîne linéaire avec 2 à 4 atomes de carbone, $R^3$ de l'hydrogène et Q de l'alkylène en chaîne linéaire avec moins de 4 atomes de carbone.

**31.** Procédé pour la fabrication de composés selon l'une quelconque des revendications 3 à 25 et de sels d'addition d'acide acceptables du point de vue pharmaceutique caractérisé en ce que

a) on décompose un composé de la formule générale

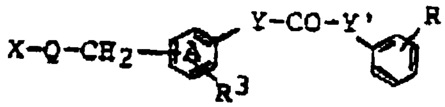

II

dans lequel X signifie un groupe de départ, et A, $R^3$, Q, Y, Y' et R ont la signification indiquée dans la revendication 3, avec une amine de la formule générale $HNR^1R^2$, dans laquelle $R^1$ et $R^2$ ont la signification indiquée dans la revendication 3, ou

b) on oxyde un composé de la formule générale

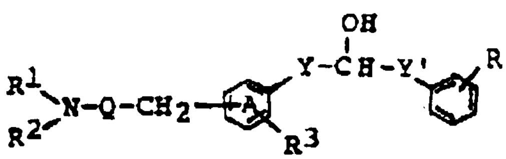

III

dans laquelle A, $R^1$, $R^2$, $R^3$, Q, Y, Y' et R ont la slgnification indiquée dans la revendication 3, ou

c) on décompose un composé de la formule générale

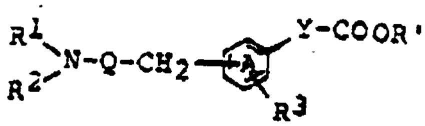

IV

dans laquelle R' signifie de l'alkyle bas, et A, $R^1$, $R^2$, $R^3$, Q et Y ont la signification indiquée dans la revendication 3, avec un composé de la formule générale

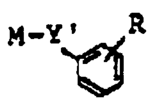

V

dans laquelle M signifie -MgCl, -MgBr, -MgJ ou -Li, et Y' et R ont la signification indiquée dans la revendication 3, ou

d) on traite un composé de la formule générale

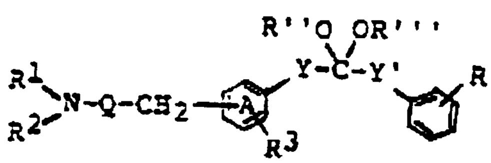

VI

dans laquelle R'' et R''' signifient respectivement de l'alkyle bas ou ensemble du diméthylène ou du triméthylène, et A, $R^1$, $R^2$, $R^3$, Q, Y, Y' et R ont la signification indiquée dans la revendication 3, avec un acide acqueux, ou

e) on décompose un composé de la formule générale

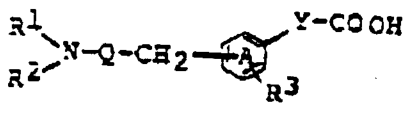

VIIa

ou

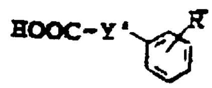

VIIb

sous forme d'un dérivé actif en présence d'un acide de Lewis avec un composé de la formule générale

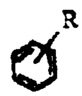

VIIIa

ou

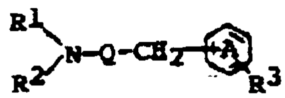

VIIIb

dans laquelle A, $R^1$, $R^2$, $R^3$, Q, Y, Y' et R ont la signification indiquée dans la revendication 3, ou
f) on décompose un composé de la formule générale

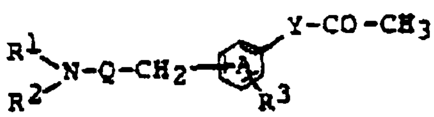

IX

dans laquelle A, $R^1$, $R^2$, $R^3$, Q et Y ont la signification indiquée dans la revendication 3, en présence d'une base avec un composé de la formule générale

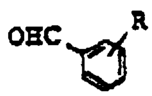

X

dans laquelle R a la signification indiquée dans la revendication 3.
g) on hydrogène un composé de la formule générale

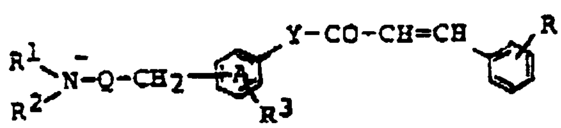

Ia

ou

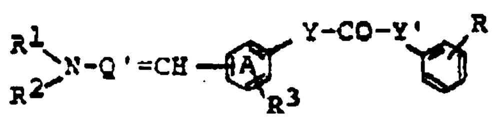

XI

dans laquelle Q' signifie le groupe Q moins un atome d'hydrogène, et A, $R^1$, $R^2$, $R^3$, Q, Y, Y' et R ont la signification indiquée dans la revendication 3, et
h) on transforme, si on le souhaite, un composé de la formule I en sel d'addition d'acide acceptable du point de vue pharmaceutique.

**32.** Médicaments, contenant un composé selon une des revendications 1 à 25 et un porteur inerte du point de vue thérapeutique.

**33.** Médicaments actifs antifongiques, contenant un composé selon une des revendications 1 à 25 et un porteur inerte du point de vue thérapeutique.

**34.** Médicaments selon la revendication 32 ou 33 caractérisé en ce qu'ils contiennent en tant qu'agent supplémentaire une substance active antifongique connue, qui bloque la biosynthèse du stérol.

**35.** Utilisation de composés selon une des revendications 1 à 25, le cas échéant en combinaison avec des substances actives antifongiques, lesquelles bloquent la biosynthèse du stérol, pour la fabrication d'agents actifs antifongiques.

**Revendications pour l'Etat contractant suivant : ES**

**1.** Procédé pour la fabrication de composés de la formule générale

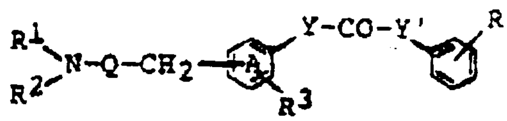

I

dans laquelle $R^1$ et $R^2$ signifient respectivement de l'hydrogène, de l'alkyle bas ou de l'alcényle bas ou ensemble de l'alkylène en chaîne linéaire avec 2 à 4 atomes de carbone, $R^3$ de l'hydrogène, de l'halogène ou de l'alkyle bas, Q de l'alkylène avec 2 à 11 atomes de carbone et au moins 2 atomes de carbone entre les deux valences libres ou de l'alcénylène avec 4 à 11 atomes de carbone et au moins 4 atomes de carbone entre les deux valences libres et Y et Y', respectivement une liaison directe ou le groupe $-CH_2-$, $-CH_2CH_2-$, $-CH=CH-$ ou $-C{\equiv}C-$, le groupe $R^1R^2N\text{-}Q\text{-}CH_2$ est lié en 3ème ou 4ème position du composé cyclique désigné par A, et le symbole R signifie que le composé cyclique n'est pas substitué ou est substitué par de l'halogène, du trifluorométhyle, du cyano, du nitro, de l'alkyle bas ou de l'alkoxy bas, Y et Y' ne signifiant pas en même temps une liaison directe, lorsque $R^1$ et $R^2$ signifient respectivement de l'hydrogène ou de l'alkyle bas ou ensemble de l'alkylène en chaîne linéaire avec 2 à 4 atomes de carbone, $R^3$ de l'hydrogène et Q de l'alkylène en chaîne linéaire avec moins de 4 atomes de carbone, et leurs sels d'addition d'acide acceptables du point de vue pharmaceutique, caractérisé en ce que

a) on décompose un composé de la formule générale

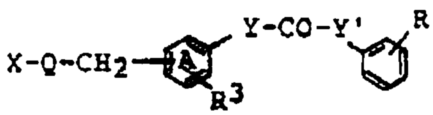

II

dans laquelle X signifie un groupe de départ, et A,$R^3$, Q, Y, Y' et R ont la signification susdite, avec une amine de la formule générale $HNR^1R^2$, dans laquelle $R^1$ et $R^2$ ont la signification susdite, ou

b) on oxyde un composé de la formule générale

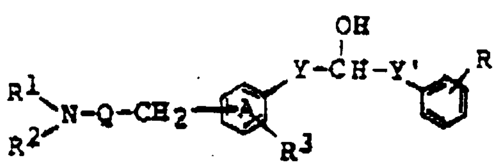

III

dans laquelle A, $R^1$, $R^2$, $R^3$, Q, Y, Y' et R ont la signification susdite, ou

c) on décompose un composé de la formule générale

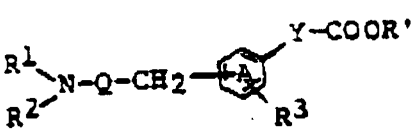

IV

dans laquelle R' signifie de l'alkyle bas, et A, $R^1$, $R^2$, $R^3$, Q et Y ont la signification susdite, avec un composé de la formule générale

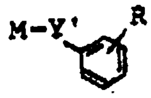

V

dans laquelle M signifie -MgCl, -MgBr, -MgJ ou -Li, et Y' et R ont la signification susdite, ou

d) on traite un composé de la formule générale

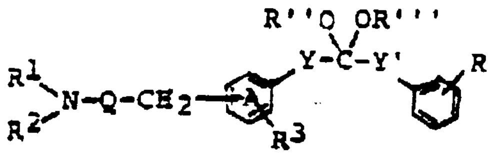

VI

dans laquelle R'' et R''' signifient respectivement de l'alkyle bas ou ensemble du diméthylène ou du triméthylène, et A, $R^1$, $R^2$, $R^3$, Q, Y, Y' et R ont la signification susdite, avec un acide aqueux, ou

e) on décompose un composé de la formule générale

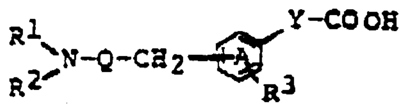

VIIa

ou

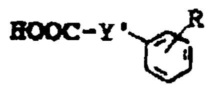

VIIb

sous forme d'un dérivé réactif en présence d'un acide de Lewis avec un composé de la formule générale

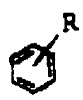

VIIIa

ou

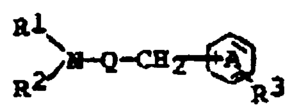

VIIIb

dans laquelle A, $R^1$, $R^2$, $R^3$, Q, Y, Y' et R ont la signification susdite, ou

f) on décompose un composé de la formule générale

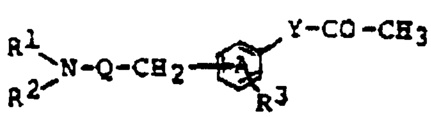

IX

dans laquelle A, $R^1$, $R^2$, $R^3$, Q et Y ont la signification susdite, en présence d'une base avec un composé de la formule générale

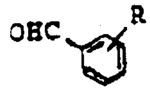

X

dans laquelle R a la signification susdite, ou

g) on hydrogène un composé de la formule générale

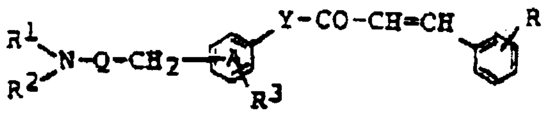

ou

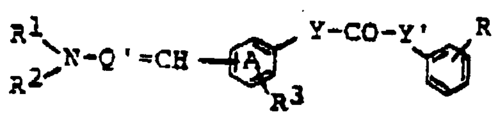

dans laquelle Q' signifie le groupe Q moins un atome d'hydrogène, et A, $R^1$, $R^2$, $R^3$, Q, Y, Y' et R ont la signification susdite, et

h) on transforme, si on le souhaite, un composé de la formule 1 en sel d'addition d'acide acceptable du point de vue pharmaceutique.

**2.** Procédé selon la revendication 1, dans lequel $R^1$ et $R^2$ signifient respectivement de l'hydrogène ou de l'alkyle bas ou ensemble de l'alkylène en chaîne linéaire avec 2 à 4 atomes de carbone, R de l'hydrogène, Q de l'alkylène avec 2 à 11 atomes de carbone et au moins 2 atomes de carbone entre les deux valences libres et Y et Y', respectivement une liaison directe ou le groupe $-CH_2-$, $-CH_2CH_2-$, $-CH=CH-$ ou $-C{\equiv}C-$, le groupe $R^1R^2N\text{-}Q\text{-}CH_2$ est lié en 3ème ou 4ème position du composé cyclique désigné par A, et le symbole R signifie que le composé cyclique n'est pas substitué ou est substitué par de l'halogène, du trifluorométhyle, du nitro, de l'alkyle bas ou de l'alkoxy bas, Y et Y' ne signifiant pas en même temps une liaison directe, lorsque $R^3$ signifie de l'hydrogène et Q de l'alkylène en chaîne linéaire avec moins de 4 atomes de carbone.

**3.** Procédé selon la revendication 1, dans lequel Q signifie de l'alkylène en chaîne linéaire avec 4 à 7 atomes d'hydrogène.

**4.** Procédé selon la revendication 3, dans lequel $R^1$ et $R^2$ signifient respectivement de l'alkyle $C_{1-4}$ ou de l'alcényle $C_{3-4}$ ou ensemble de l'alkylène $C_{3-4}$.

**5.** Procédé selon la revendication 3 ou 4, dans lequel $R^3$ signifie de l'hydrogène.

**6.** Procédé selon l'une quelconque des revendications 3 à 5, dans lequel le groupe $R^1R^2N\text{-}Q\text{-}CH_2\text{-}$ est lié en 4ème position du composé cyclique désigné par A.

**7.** Procédé selon l'une quelconque des revendications 3 à 6, dans lequel Y signifie une liaison directe ou le groupe $-CH_2-$.

**8.** Procédé selon l'une quelconque des revendications 3 à 7, dans lequel Y' signifie une liaison directe ou le groupe $-CH_2-$, $-CH_2CH_2-$ ou $-CH=CH-$.

**9.** Procédé selon l'une quelconque des revendications 3 à 8, dans lequel le symbole R signifie que le composé cyclique n'est pas substitué ou est substitué par de l'halogène, du trifluorométhyle, du nitro ou de l'alkyle bas.

**10.** Procédé selon la revendication 1, caractérisé en ce que l'on fabrique du 4-[7(diméthylamino)heptyl]-benzophénone.

**11.** Procédé selon la revendication 1, caractérisé en ce que l'on fabrique du 4'-[7(diméthylamino)heptyl]-2-phénylacétophénone.

**12.** Procédé selon la revendication 1, caractérisé en ce que l'on fabrique du 4'-fluoro-4-[7-(diméthylamino) heptyl]benzophénone.

**13.** Procédé selon la revendication 1, caractérisé en ce que l'on fabrique du 4-[7-(diméthylamino)heptyl]-4'-(trifluorométhyl] - benzophénone.

**14.** Procédé selon la revendication 1, caractérisé en ce que l'on fabrique du 2,4-difluoro-4'-7[-(diméthylamino)heptyl]benzophénone.

**15.** Procédé selon la revendication 1, dans lequel Q signifie de l'alkylène en chaîne linéaire avec 2 ou 3 atomes de carbone.

**16.** Procédé selon la revendication 15, dans lequel $R^1$ et $R^2$ signifient respectivement de l'alkyle $C_{1-4}$ ou de l'alcényle $C_{3-4}$ ou ensemble de l'alkylène $C_{3-4}$.

**17.** Procédé selon la revendication 15 ou 16, dans lequel $R^3$ signifie de l'hydrogène.

**18.** Procédé selon l'une quelconque des revendications 15 à 17, dans lequel le groupe $R^1R^2N\text{-}Q\text{-}CH_2$ est lié en 4ème position du composé cyclique désigné par A.

**19.** Procédé selon l'une quelconque des revendications 15 à 18, dans lequel Y signifie une liaison directe et Y' le groupe $-CH_2-$, $-CH_2CH_2-$ ou $-CH=CH-$.

**20.** Procédé selon l'une quelconque des revendications 15 à 19, dans lequel le symbole R signifie que le composé cyclique n'est pas substitué ou qu'il est substitué par de l'halogène, du trifluorométhyle, du nitro ou de l'alkyle bas.

**21.** Procédé selon la revendication 1, caractérisé en ce que l'on fabrique du 3-(4-chlorophényl)-4'-[3-(diméthylamino)propyl]-propiophénone.

**22.** Procédé selon la revendication 1, caractérisé en ce que l'on fabrique du 3-(2-méthylphényl)-4'-[3-(diméthylamino)propyl] -propiophénone.

**23.** Procédé selon la revendication 1, caractérisé en ce que l'on fabrique du (E)-3-phéhyl-4'-[3-(1-pyrrolidinyl)propyl]acrylophénone.

**24.** Procédé pour la fabrication de médicaments, notamment de médicaments actifs antifongiques, caractérisé en ce que l'on amène un composé de la formule générale

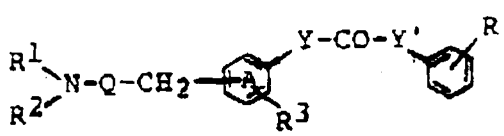

I

dans laquelle $R^1$ et $R^2$ signifient respectivement de l'hydrogène, de l'alkyle bas ou de l'alcényle bas ou ensemble de l'alkylène en chaîne linéaire avec 2 à 4 atomes de carbone, $R^3$ de l'hydrogène, de l'halogène ou de l'alkyle bas, Q de l'alkylène avec 2 à 11 atomes de carbone et au moins 2 atomes de carbone entre les deux valences libres ou de l'alcénylène avec 4 à 11 atomes de carbone et au moins 4 atomes de carbone entre les deux valences libres et Y et Y' respectivement une liaison directe ou le groupe $-CH_2-$, $-CH_2CH_2-$, $-CH=CH-$ ou $-C\equiv C-$, le groupe $R^1R^2N\text{-}Q\text{-}CH_2$ est lié en 3ème ou la 4ème position du composé cyclique caractérisé par A, et le symbole R signifie que le composé cyclique n'est pas substitué ou est substitué par de l'halogène, du trifluorométhyle, du cyano, du nitro, de l'alkyle bas ou de l'alkoxy bas, ou un sel d'addition d'acide de ce composé acceptable du point de vue pharmaceutique, sous une forme galénique, avec un porteur inerte du point de vue thérapeutique.

**25.** Procédé pour la fabrication de médicaments actifs antifongiques, caractérisé en ce que l'on amène un composé de la formule I définie dans la revendication 24 ou un sel d'addition d'acide de ce composé, acceptable du point de vue pharmaceutique, avec une substance active antifongique connue, qui bloque la biosynthèse du stérol, et un porteur inerte du point de vue thérapeutique, sous une forme galénique.

**26.** Utilisation de composés de la formule I définie dans la revendication 24 et leurs sels d'addition d'acide acceptables du point de vue pharmaceutique, le cas échéant, en combinaison avec des substances

actives antifongiques connues, qui bloquent la biosynthèse du stérol, pour la fabrication de médicaments actifs antifongiques.

**Revendications pour l'Etat contractant suivant : GR**

1. Procédé pour la fabrication de composés de la formule générale

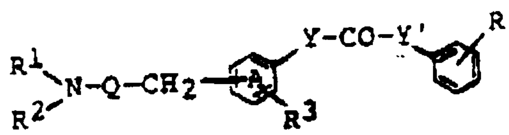

I

dans laquelle $R^1$ et $R^2$ signifient respectivement de l'hydrogène, de l'alkyle bas ou de l'alcényle bas ou ensemble de l'alkylène en chaîne linéaire avec 2 à 4 atomes de carbone, $R^3$ de l'hydrogène, de l'halogène ou de l'alkyle bas, Q de l'alkylène avec 2 à 11 atomes de carbone et au moins 2 atomes de carbone entre les deux valences libres ou de l'alcénylène avec 4 à 11 atomes de carbone et au moins 4 atomes de carbone entre les deux valences libres et Y et Y', respectivement une liaison directe ou le groupe $-CH_2-$, $-CH_2CH_2-$, -CH=CH- ou -C≡C-, le groupe $R^1R^2N-Q-CH_2$ est lié en 3ème ou 4ème position du composé cyclique désigné par A, et le symbole R signifie que le composé cyclique n'est pas substitué ou est substitué par de l'halogène, du trifluorométhyle, du cyano, du nitro, de l'alkyle bas ou de l'alkoxy bas, Y et Y' ne signifiant pas en même temps une liaison directe, lorsque $R^1$ et $R^2$ signifient respectivement de l'hydrogène ou de l'alkyle bas ou ensemble de l'alkylène en chaîne linéaire avec 2 à 4 atomes de carbone, $R^3$ de l'hydrogène et Q de l'alkylène en chaîne linéaire avec moins de 4 atomes de carbone, et leurs sels d'addition d'acide acceptables du point de vue pharmaceutique, caractérisé en ce que

a) on décompose un composé de la formule générale

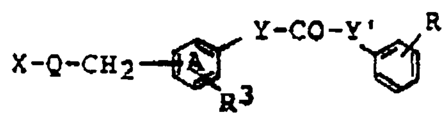

II

dans laquelle X signifie un groupe de départ, et A,$R^3$, Q, Y, Y' et R ont la signification susdite, avec une amine de la formule générale $HNR^1R^2$, dans laquelle $R^1$ et $R^2$ ont la signification susdite, ou

b) on oxyde un composé de la formule générale

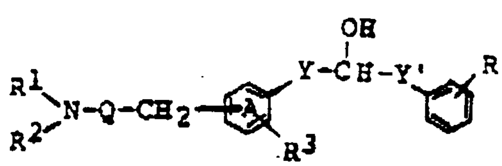

III

dans laquelle A, $R^1$, $R^2$, $R^3$, Q, Y, Y' et R ont la signification susdite, ou

c) on décompose un composé de la formule générale

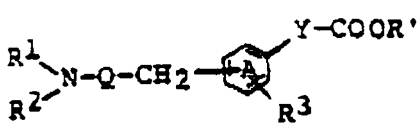

IV

dans laquelle R' signifie de l'alkyle bas, et A, $R^1$, $R^2$, $R^3$, Q et Y ont la signification susdite, avec un composé de la formule générale

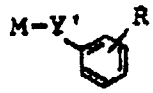

V

dans laquelle M signifie -MgCl, -MgBr, -MgJ ou -Li, et Y' et R ont la signification susdite, ou
d) on traite un composé de la formule générale

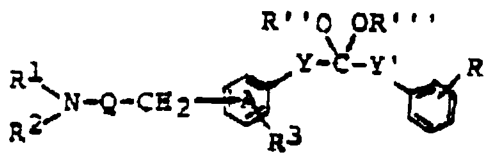

VI

dans laquelle R'' et R''' signifient respectivement de l'alkyle bas ou ensemble du diméthylène ou du triméthylène, et A, $R^1$, $R^2$, $R^3$, Q, Y, Y' et R ont la signification susdite, avec un acide aqueux, ou
e) on décompose un composé de la formule générale

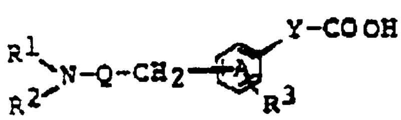

VIIa

ou

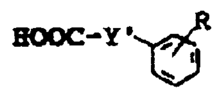

VIIb

sous forme d'un dérivé réactif en présence d'un acide de Lewis avec un composé de la formule générale

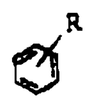

VIIIa

ou

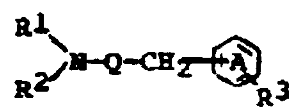

VIIIb

dans laquelle A, $R^1$, $R^2$, $R^3$, Q, Y, Y' et R ont la signification susdite, ou
f) on décompose un composé de la formule générale

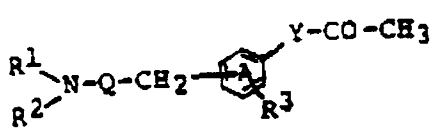

IX

dans laquelle A, $R^1$, $R^2$, $R^3$, Q et Y ont la signification susdite, en présence d'une base avec un composé de la formule générale

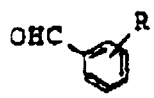

X

dans laquelle R a la signification susdite, ou

g) on hydrogène un composé de la formule générale

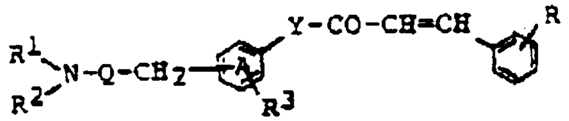

ou

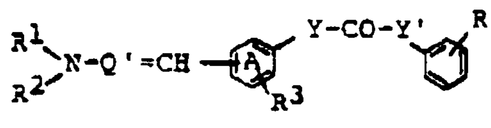

dans laquelle Q' signifie le groupe Q moins un atome d'hydrogène, et A, $R^1$, $R^2$, $R^3$, Q, Y, Y' et R ont la signification susdite, et

h) on transforme, si on le souhaite, un composé de la formule 1 en sel d'addition d'acide acceptable du point de vue pharmaceutique.

**2.** Procédé selon la revendication 1, dans lequel $R^1$ et $R^2$ signifient respectivement de l'hydrogène ou de l'alkyle bas ou ensemble de l'alkylène en chaîne linéaire avec 2 à 4 atomes de carbone, R de l'hydrogène, Q de l'alkylène avec 2 à 11 atomes de carbone et au moins 2 atomes de carbone entre les deux valences libres et Y et Y', respectivement une liaison directe ou le groupe $-CH_2-$, $-CH_2CH_2-$, -CH = CH- ou -C≡C-, le groupe $R^1R^2N$-Q-$CH_2$ est lié en 3ème ou 4ème position du composé cyclique désigné par A, et le symbole R signifie que le composé cyclique n'est pas substitué ou est substitué par de l'halogène, du trifluorométhyle, du nitro, de l'alkyle bas ou de l'alkoxy bas, Y et Y' ne signifiant pas en même temps une liaison directe, lorsque $R^3$ signifie de l'hydrogène et Q de l'alkylène en chaîne linéaire avec moins de 4 atomes de carbone.

**3.** Procédé selon la revendication 1, dans lequel Q signifie de l'alkylène en chaîne linéaire avec 4 à 7 atomes d'hydrogène.

**4.** Procédé selon la revendication 3, dans lequel $R^1$ et $R^2$ signifient respectivement de l'alkyle $C_{1-4}$ ou de l'alcényle $C_{3-4}$ ou ensemble de l'alkylène $C_{3-4}$.

**5.** Procédé selon la revendication 3 ou 4, dans lequel $R^3$ signifie de l'hydrogène.

**6.** Procédé selon l'une quelconque des revendications 3 à 5, dans lequel le groupe $R^1R^2N$-Q-$CH_2$ est lié en 4ème position du composé cyclique désigné par A.

**7.** Procédé selon l'une quelconque des revendications 3 à 6, dans lequel Y signifie une liaison directe ou le groupe $-CH_2-$.

**8.** Procédé selon l'une quelconque des revendications 3 à 7, dans lequel Y' signifie une liaison directe ou le groupe $-CH_2-$, $-CH_2CH_2-$ ou -CH = CH-.

**9.** Procédé selon l'une quelconque des revendications 3 à 8, dans lequel le symbole R signifie que le composé cyclique n'est pas substitué ou est substitué par de l'halogène, du trifluorométhyle, du nitro ou de l'alkyle bas.

**10.** Procédé selon la revendication 1, caractérisé en ce que l'on fabrique du 4-[7(diméthylamino)heptyl]-benzophénone.

**11.** Procédé selon la revendication 1, caractérisé en ce que l'on fabrique du 4'-[7(diméthylamino)heptyl]-2-phénylacétophénone.

**12.** Procédé selon la revendication 1, caractérisé en ce que l'on fabrique du 4'-fluoro-4-[7-(diméthylamino) heptyl]benzophénone.

**13.** Procédé selon la revendication 1, caractérisé en ce que l'on fabrique du 4-[7-(diméthylamino)heptyl]-4'-(trifluorométhyl] - benzophénone.

**14.** Procédé selon la revendication 1, caractérisé en ce que l'on fabrique du 2,4-difluoro-4'-7[-(diméthylamino)heptyl]benzophénone.

**15.** Procédé selon la revendication 1, dans lequel Q signifie de l'alkylène en chaîne linéaire avec 2 ou 3 atomes de carbone.

**16.** Procédé selon la revendication 15, dans lequel $R^1$ et $R^2$ signifient respectivement de l'alkyle $C_{1-4}$ ou de l'alcényle $C_{3-4}$ ou ensemble de l'alkylène $C_{3-4}$.

**17.** Procédé selon la revendication 15 ou 16, dans lequel $R^3$ signifie de l'hydrogène.

**18.** Procédé selon l'une quelconque des revendications 15 à 17, dans lequel le groupe $R^1R^2N\text{-}Q\text{-}CH_2$ est lié en 4ème position du composé cyclique désigné par A.

**19.** Procédé selon l'une quelconque des revendications 15 à 18, dans lequel Y signifie une liaison directe et Y' le groupe $-CH_2-$, $-CH_2CH_2-$ ou $-CH=CH-$.

**20.** Procédé selon l'une quelconque des revendications 15 à 19, dans lequel le symbole R signifie que le composé cyclique n'est pas substitué ou qu'il est substitué par de l'halogène, du trifluorométhyle, du nitro ou de l'alkyle bas.

**21.** Procédé selon la revendication 1, caractérisé en ce que l'on fabrique du 3-(4-chlorophényl)-4'-[3-(diméthylamino)propyl]-propiophénone.

**22.** Procédé selon la revendication 1, caractérisé en ce que l'on fabrique du 3-(2-méthylphényl)-4'-[3-(diméthylamino)propyl] -propiophénone.

**23.** Procédé selon la revendication 1, caractérisé en ce que l'on fabrique du (E)-3-phényl-4'-[3-(1-pyrrolidinyl)propyl]acrylophéhone.

**24.** Procédé pour la fabrication de médicaments, notamment de médicaments actifs antifongiques, caractérisé en ce que l'on amène un composé de la formule générale

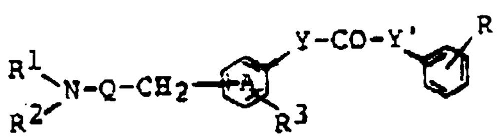

I

dans laquelle $R^1$ et $R^2$ signifient respectivement de l'hydrogène, de l'alkyle bas ou de l'alcényle bas ou ensemble de l'alkylène en chaîne linéaire avec 2 à 4 atomes de carbone, $R^3$ de l'hydrogène, de l'halogène ou de l'alkyle bas, Q de l'alkylène avec 2 à 11 atomes de carbone et au moins 2 atomes de carbone entre les deux valences libres ou de l'alcénylène avec 4 à 11 atomes de carbone et au moins 4 atomes de carbone entre les deux valences libres et Y et Y' respectivement une liaison directe ou le groupe $-CH_2-$, $-CH_2CH_2-$, $-CH=CH-$ ou $-C\equiv C-$, le groupe $R^1R^2N\text{-}Q\text{-}CH_2$ est lié en 3ème ou la 4ème position du composé cyclique caractérisé par A, et le symbole R signifie que le composé cyclique n'est pas substitué ou est substitué par de l'halogène, du trifluorométhyle, du cyano, du nitro, de l'alkyle bas ou de l'alkoxy bas, ou un sel d'addition d'acide de ce composé acceptable du point de vue pharmaceutique, sous une forme galénique, avec un porteur inerte du point de vue thérapeutique.

**25.** Procédé pour la fabrication de médicaments actifs antifongiques, caractérisé en ce que l'on amène un composé de la formule I définie dans la revendication 24 ou un sel d'addition d'acide de ce composé, acceptable du point de vue pharmaceutique, avec une substance active antifongique connue, qui bloque la biosyrithèse du stérol, et un porteur inerte du point de vue thérapeutique, sous une forme galénique.

**26.** Utilisation de composés de la formule I définie dans la revendication 24 et leurs sels d'addition d'acide acceptables du point de vue pharmaceutique, le cas échéant, en combinaison avec des substances actives antifongiques connues, qui bloquent la biosynthèse du stérol, pour la fabrication de médicaments actifs antifongiques.

**27.** Composés de la formule générale

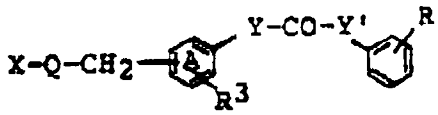

II

dans laquelle X signifie un groupe de départ, $R^3$ de l'hydrogène, de l'halogène ou de l'alkyle bas, Q de l'alkylène avec 2 à 11 atomes de carbone et au moins 2 atomes de carbone entre les deux valences libres et Y et Y', respectivement une liaison directe ou le groupe $-CH_2-$, $CH_2CH_2-$, -CH = CH- ou -C≡C, le groupe $X-Q-CH_2$ est lié en 3ème ou 4ème position du composé cyclique désigné par A, et le symbole R signifie que le composé cyclique n'est pas substitué ou qu'il est substitué par de l'halogène, du trifluorométhyle, du cyano, du nitro, de l'alkyle bas ou de l'alkoxy bas, Y et Y' ne signifiant pas en même temps une liaison directe, lorsque $R^3$ signifie de l'hydrogène et Q de l'alkylène en chaîne linéaire avec moins de 4 atomes de carbone.

**28.** Composés de la formule générale

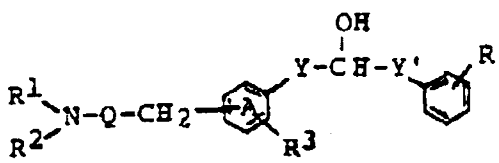

III

dans laquelle $R^1$ et $R^2$ signifient respectivement de l'hydrogène, de l'alkyle bas ou de l'alcényle bas ou ensemble de l'alkylène en chaîne linéaire avec 2 à 4 atomes de carbone, $R^3$ de l'hydrogène, de l'halogène ou de l'alkyle bas, Q de l'alkylène avec 2 à 11 atomes de carbone et au moins 2 atomes de carbone entre les deux valences libres ou de l'alcénylène avec 4 à 11 atomes de carbone et au moins 4 atomes de carbone entre les deux valences libres et Y et Y' respectivement une liaison directe ou le groupe $-CH_2-$, $-CH_2CH2-$, -CH = CH- ou -C≡C-, le groupe $R^1R^2N-Q-CH_2$ est lié en 3ème ou 4ème position du composé cyclique désigné par A, et le symbole R signifie que le composé cyclique n'est pas substitué ou qu'il est substitué par de l'halogène, du trifluorométhyle, du cyano, du nitro, de l'alkyle bas ou de l'alkoxy bas, Y et Y' ne signifiant pas en même temps une liaison directe, lorsque $R^1$ et $R^2$ signifient respectivement de l'hydrogène ou de l'alkyle bas ou ensemble de l'alkylène en chaîne linéaire avec 2 à 4 atomes de carbone, $R^3$ de l'hydrogène et Q de l'alkylène en chaîne linéaire avec moins de 4 atomes de carbone.

**29.** Composés de la formule générale

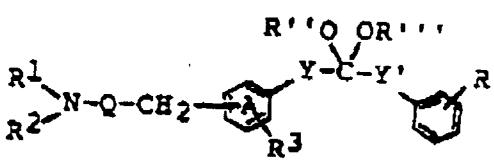

VI

dans laquelle R'' et R''' signifient respectivement de l'alkyle bas ou ensemble du diméthylène ou du triméthylène, $R^1$ et $R^2$ respectivement de l'hydrogène, de l'alkyle bas ou de l'alcényle bas ou ensemble de l'alkylène en chaîne linéaire avec 2 à 4 atomes de carbone, $R^3$ de l'hydrogène, de l'halogène ou de l'alkyle bas, Q de l'alkylène avec 2 à 11 atomes de carbone et au moins 2 atomes de carbone entre les deux valences libres ou de l'alcénylène avec 4 à 11 atomes de carbone et au moins 4 atomes de carbone entre les deux valences libres et Y et Y', respectivement une liaison directe ou le groupe $-CH_2-$, $-CH_2CH_2-$, -CH = CH- ou -C≡C-, le groupe $R^1R^2N-Q-CH_2$ est lié en 3ème ou 4ème position du composé cyclique désigné par A, et le symbole R signifie que le composé cyclique n'est pas substitué ou est substitué par de l'halogène, du trifluorométhyle, du cyano, du nitro, de l'alkyle bas ou de l'alkoxy bas, Y et Y' ne signifiant pas en même temps une liaison directe, lorsque $R^1$ et $R_2$ signifient

respectivement de l'hydrogène ou de l'alkyle bas ou ensemble de l'alkylène en chaîne linéaire avec 2 à 4 atomes de carbone, $R^3$ de l'hydrogène et Q de l'alkylène en chaîne linéaire avec moins de 4 atomes de carbone.